# EUROPEAN PATENT SPECIFICATION

(11) **EP 3 763 211 B1**
(45) Date of publication and mention of the grant of the patent: **21.10.2026**
(21) Application number: 20181788.9
(22) Date of filing: 24.02.2017
(51) Int. Cl.: A01N 43/80, C07D 261/04, A01P 7/04, A01P 7/02, A01P 3/00, A61K 9/00, A61K 47/00, A61K 31/422

(54) **ANTIPARASITIC ISOXAZOLINE COMPOUNDS, LONG-ACTING INJECTABLE FORMULATIONS COMPRISING THEM, METHODS AND USES THEREOF**
ANTIPARASITÄRE ISOXAZOLINVERBINDUNGEN, LANGWIRKENDE INJIZIERBARE FORMULIERUNGEN DAMIT, VERFAHREN UND VERWENDUNGEN DAVON
COMPOSÉS D'ISOXAZOLINE ANTIPARASITAIRES, FORMULATIONS INJECTABLES À LONGUE DURÉE D'ACTION LES COMPRENANT, PROCÉDÉS ET UTILISATIONS ASSOCIÉS

(30) Priority: 24.02.2016 US 201662299333 P; 25.08.2016 US 201662379348 P
(43) Date of publication of application: 13.01.2021
(62) Document number(s) of the earlier application(s) in accordance with Art. 76 EPC: 17709300.2 / 3 419 424
(73) Proprietor: Boehringer Ingelheim Vetmedica GmbH, 55216 Ingelheim am Rhein (DE)
(72) Inventor: LE HIR DE FALLOIS, Lois, Patrick, Atlanta, GA 30305 (US); MENG, Charles, Q., Grayson, GA 30017 (US); CADY, Susan, Mancini, Yardley, PA 19067 (US); CHEIFETZ, Peter, East Windsor, NJ 08520 (US); GALESKA, Izabela, Newtown, PA 18940 (US)
(74) Representative: D Young & Co LLP

(56) References cited:
- EP-A1- 1 731 512
- EP-A1- 2 172 448
- WO-A1-2007/079162
- WO-A1-2011/075591
- WO-A1-2011/104089
- WO-A1-2011/149749
- WO-A1-2011/157748
- WO-A1-2012/017359
- WO-A1-2012/107533
- WO-A1-2012/120135
- WO-A1-2012/120399
- WO-A1-2013/039948
- WO-A1-2013/078070
- WO-A1-2013/119442
- WO-A1-2014/039475
- WO-A1-2014/189837
- WO-A1-2015/048371
- WO-A1-2015/066277
- WO-A1-2016/138339
- WO-A1-2016/164487
- WO-A2-2008/154528
- WO-A2-2009/002809
- WO-A2-2009/024541
- WO-A2-2010/070068
- WO-A2-2011/157733
- JP-A- 2016 145 160
- SUSANNE KILP ET AL: "Pharmacokinetics of fluralaner in dogs following a single oral or intravenous administration", PARASITES & VECTORS, BIOMED CENTRAL LTD, LONDON UK, vol. 7, no. 1, 85, 7 March 2014 (2014-03-07), pages 1 - 5, XP021179157, ISSN: 1756-3305, DOI: 10.1186/1756-3305-7-85
- DATABASE WPI Week 200729, Derwent World Patents Index; AN 2007-294920, XP002769912

## Description

### FIELD OF THE INVENTION

The present invention provides long-acting injectable compositions comprising at least one isoxazoline active agent, a neutral oiland, optionally, a co-solvent. The invention also provides for the use of these compounds and compositions against pests and parasites (including ectoparasites (e.g., fleas or ticks) and/or endoparasites), and methods for controlling pests and preventing or treating parasitic infections and infestations in animals.

### CROSS REFERENCE TO RELATED APPLICATIONS

This application claims the benefit of priority to U.S. Provisional Application Nos. 62/299,333, filed February 24, 2016, and 62/379,348, filed August 25, 2016.

### BACKGROUND OF THE INVENTION

Animals such as mammals and birds are often susceptible to parasite infestations/infections. These parasites may be ectoparasites, such as fleas, ticks and parasitic flies, and endoparasites such as nematodes and other worms. Domesticated animals, such as cats and dogs, are often infested with one or more of the following ectoparasites:
- fleas (e.g. *Ctenocephalides* spp., such as *Ctenocephalides felis* and the like);
- ticks (e.g. *Rhipicephalus* spp., *Ixodes* spp., *Dermacentor* spp., *Amblyomma* spp., and the like);
- mites (e.g. *Demodex* spp., *Sarcoptes* spp., *Otodectes* spp., and the like);
- lice (e.g. *Trichodectes* spp., *Cheyletiella* spp., *Linognathus* spp. and the like);
- mosquitoes (*Aedes* spp., *Culex* spp., *Anopheles* spp. and the like); and
- flies (*Haematobia* spp., *Musca* spp., *Stomoxys* spp., *Dermatobia* spp., *Cochliomyia* spp. and the like).

Fleas are a particular problem because not only do they adversely affect the health of the animal or human, but they also cause a great deal of psychological stress. Moreover, fleas may also transmit pathogenic agents to animals and humans, such as tapeworm (*Dipylidium caninum*)*.*

Similarly, ticks are also harmful to the physical and psychological health of the animal or human. However, the most serious problem associated with ticks is that they are vectors of pathogenic agents in both humans and animals. Major diseases which may be transmitted by ticks include borreliosis (Lyme disease caused by *Borrelia burgdorferi*)*,* babesiosis (or piroplasmosis caused by *Babesia* spp.) and rickettsioses (e.g. Rocky Mountain spotted fever). Ticks also release toxins which cause inflammation or paralysis in the host. Occasionally, these toxins are fatal to the host.

Likewise, farm animals are also susceptible to parasite infestations. For example, cattle and other bovines are affected by a large number of parasites. A parasite which is prevalent among cattle in some regions are ticks of the genus *Rhipicephalus,* especially those of the species *microplus* (cattle tick), *decoloratus* and *annulatus.* Ticks such as *Rhipicephalus microplus* (formerly *Boophilus microplus*) are difficult to control because they lay eggs in the pasture where farm animals graze. This species of ticks is considered a one-host tick and spends immature and adult stages on one animal before the female engorges and falls off the host to lay eggs in the environment. The life cycle of the tick is approximately three to four weeks. In addition to cattle, *Rhipicephalus microplus* may infest buffalo, horses, donkeys, goats, sheep, deer, pigs, and dogs. A heavy tick burden on animals can decrease production and damage hides as well as transmit diseases such as babesiosis ("cattle fever") and anaplasmosis.

Animals and humans also suffer from endoparasitic infections including, for example, helminthiasis which is caused by parasitic worms categorized as cestodes (tapeworm), nematodes (roundworm) and trematodes (flatworm or flukes). These parasites adversely affect the nutrition of the animal and cause severe economic losses in pigs, sheep, horses, and cattle as well as affecting companion animals and poultry. Other parasites which occur in the gastrointestinal tract of animals and humans include those from the genus *Ancylostoma, Necator, Ascaris, Strongyloides, Trichinella, Capillaria, Toxocara, Toxascaris, Trichuris, Enterobius* and parasites which are found in the blood or other tissues and organs such as filarial worms and the extra intestinal stages of *Strongyloides, Toxocara* and *Trichinella.*

Recently, isoxazole and isoxazoline-containing compounds have been demonstrated to be effective against parasites that harm animals. For example, US 7,964,204 (to DuPont) discloses isoxazoline compounds according to formula (I) below, which are active against ectoparasites and/or endoparasites. 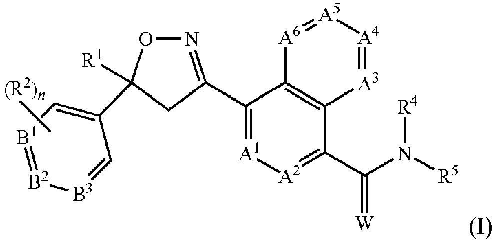 In addition, published patent application nos. US 2010/0254960 A1, WO 2007/070606 A2, WO 2007/123855 A2, WO 2010/003923 A1, US7951828 & US7662972, US 2010/0137372 A1, US 2010/0179194 A2, US 2011/0086886 A2, US 2011/0059988 A1, US 2010/0179195 A1 and WO 2007/075459 A2 and U.S. Patent Nos. 7,951,828 and 7,662,972 describe various other parasiticidal isoxazoline compounds. Other published patent applications that describe various other parasiticidal isoxazoline compounds and compositions comprising the same include WO 2007/079162 A1, WO 2008/154528 A1, WO 2009/002809 A2, WO 2011/149749 A1, WO 2014/039475 A1, US 8,466,115, WO 2012/120399, WO 2014/039484, WO 2014/189837, (Zoetis), WO 2012 120135 A1 (Novartis), WO 2016/164487, WO 2016/138339, JP 2016-145160, WO 2011/157733, WO 2011/075591, WO 2015/066277, WO 2010/070068, WO 2011/104089, WO 2012/120399, WO 2009/024541, WO 2011/149749, WO 2015/048371, WO 2012/017359, WO 2011/157748, WO 2012/107533, WO 2013/078070, WO 2008/154528, EP 2172448, JP 2007-016017 and EP 1731512. WO 2012/089623 describes topical localized isoxazoline compositions comprising glycofurol. WO 2013/039948 A1 provides for topical veterinary compositions comprising at least one isoxazoline active agent and WO 2013/119442 A1 provides for oral veterinary compositions such as a soft chew which comprising at least one isoxazoline active agent. Kilp et al. (2014), "Pharmacokinetics of fluralaner in dogs following a single oral or intravenous administration", Parasites Vectors, 7, 85 provides for oral and intravenous veterinary compositions comprising an isoxazoline active agent.

In additional to topical and oral dosage forms, it is sometimes possible to formulate active agents as long-acting compositions, depending upon, for example, the physiochemical properties of the individual active agent; these properties include, for example, solubility, bioavailability, etc. For example, US 6,733,767 and US 8,362,086 provide for long acting injectable compositions comprising a bioactive substance, such as, for example, an avermectin or a milbemycin and a biological acceptable polymer.

Notwithstanding the highly active isoxazoline active agents and compositions comprising isoxazoline active agents alone or in combination with other active agents described in the documents above, there is a need for more effective isoxazoline compounds and veterinary compositions and methods with improved efficacy, bioavailability, and spectrum of coverage to protect animals against endoparasites and/or ectoparasites. More specifically, there is a need to develop a long-acting injectable composition comprising an isoxazoline compound, which has good bioavailability and exhibits a reduced irritation at the injection site while still being effective against parasites (e.g., fleas and ticks) for a long duration (i.e., from three (3) to six (6) months.

### SUMMARY OF THE INVENTION

The present invention is defined in the appended claims.

In a first aspect, the present invention provides for novel and inventive long-acting injectable compositions for the treatment or prevention of parasite infections or infestations in an animal comprising an antiparasitic effective amount of at least one isoxazoline compound of formula (Ic), a neutral oil that is selected from triglycerides of fractionated plant fatty acids with chain lengths of C₈ to C₁₀, optionally, a co-solvent, optionally, an antioxidant, and optionally a pharmaceutically acceptable additive, excipient or mixtures thereof, wherein no other pharmaceutically acceptable polymers are present.

In accordance with the first aspect of the present invention, it has been discovered that the inventive long-acting compositions generally show desirable bioavailability and duration of efficacy, while causing minimal irritation at the injection site. The compositions also provide desirable safety profiles toward the warm-blooded and bird animal recipients. In addition, it has been discovered that a single administration of such compositions generally provides potent activity against one or more parasites (e.g., ectoparasites), while also tending to provide fast onset of activity, long duration of activity, and/or desirable safety profiles.

The invention encompasses uses or veterinary uses of the compositions described herein for the treatment or prevention of parasitic infections and infestations in or on animals (either wild or domesticated), including livestock and companion animals such as cats, dogs, horses, chickens, sheep, goats, pigs, turkeys and cattle, with the aim of ridding these hosts of parasites commonly encountered by such animals.

The invention also provides long-acting injectable compositions as defined herein for use in treating or preventing parasites in an animal in need thereof, wherein the parasites are treated or prevented for about 3 to 6 months. Surprisingly, it has been found that the inventive isoxazoline-containing compositions described herein exhibit superior broad spectrum efficacy against harmful parasites (e.g. ectoparasites such as fleas and ticks) more rapidly, and over a long duration compared to other injectable compositions containing isoxazoline active agents known in the art while exhibiting minimal irritation at the injection site.

The invention provides long-acting injectable compositions comprising an isoxazoline compound of formula (Ic): 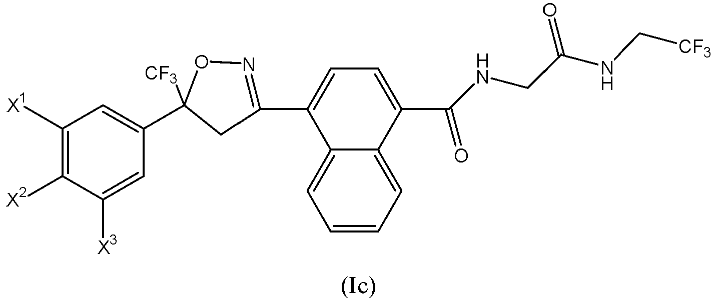 or a pharmaceutically acceptable salt thereof; wherein X¹, X² and X³ are each independently H, halogen, C₁-C₃alkyl or C₁-C₃haloalkyl. Long acting compositions comprising a compound of formula (Ic) wherein X¹ is chloro, X² is fluoro and X³ is CF₃ have been shown to have surprisingly long-lasting and excellent efficacy against *Rhipicephalus microplus* with a quick onset and a very long duration of time.

In another embodiment, the long-acting injectable compositions and methods comprise 4-[5-[3-chloro-5-(trifluoromethyl)phenyl]-4,5-dihydro-5-(trifluoromethyl)-3-isoxazolyl]-N-[2-oxo-2-[(2,2,2-trifluoroethyl)amino]ethyl]-1-naphthalenecarboxamide as the active agent.

In other embodiments, the long-acting injectable compositions may further comprise one or more additional active agents that are systemically active. Systemically-acting active agents may include, but are not limited to, isoxazoline active agents of different structure, a systemically-acting neonicotinoid active agent, a systemically-acting 1-N-arylpyrazole active agent, macrocyclic lactones such as avermectin and milbemycin compounds, a cyclic depsipeptide such as emodepside or PF1022A or analogs thereof, benzimidazoles, imidazothiazoles, a tetrahydropyrimidine active agent, an organophosphate active agent, levamisole, a paraherquamide active agent and/or a marcfortine active agent, praziquantel, closantel, clorsulon, morantel, pyrantel, a spinosyn or spinosoid active agent, an amino acetonitrile active agent, an aryloazol-2-yl cyanoethyl active agent, a systemically-acting insect growth regulator. In one embodiment, the long-acting injectable compositions comprise at least one macrocyclic lactone active agent, including, but not limited to, avermectins or milbemycins. In some embodiments, the avermectin or milbemycin active agent is eprinomectin, ivermectin, selamectin, milbemectin, milbemycin D, milbemycin oxime, or moxidectin.

In other embodiments, the compositions and methods comprise at least one of thiabendazole, oxibendazole, mebendazole, fenbendazole, oxfendazole, albendazole, triclabendazole, febantel, levamisole, pyrantel, morantel, praziquantel, closantel, clorsulon, an amino acetonitrile active agent, or an aryloazol-2-yl cyanoethylamino active agent.

Also described herein are novel and inventive pesticidal and parasiticidal isoxazoline compounds of formula (Id) shown: 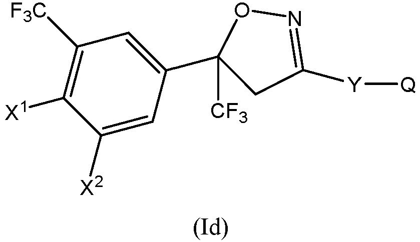
wherein X¹ is bromo, chloro, iodo or fluoro; and X² is chloro, fluoro or CF₃;
Y is a group Y-1, Y-2, Y-3, Y-4 where Z is N or CH, Y-5 or Y-6 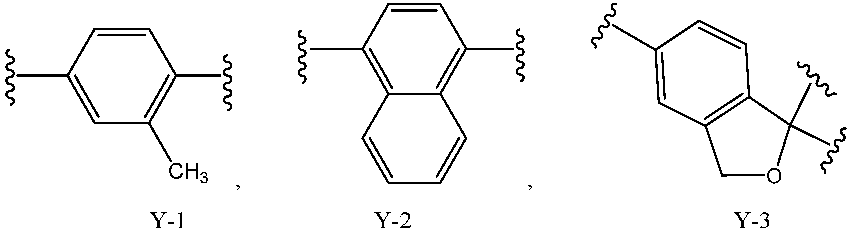 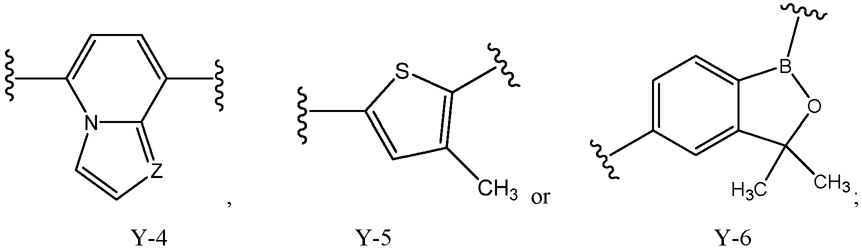 and Q is OH, -C(O)NHCH₂C(O)NHCH₂CF₃, -C(O)CH₂S(O)₂CH₃, -C(O)NHCH₂CH₂SCH₃ or (-CH₂-)(-CH₂-)N(CO)CH₂S(O)₂CH₃.

The compounds of formula (Id) are highly active against arthropod pests and parasites and useful for protecting animals, including livestock and companion animals such as cats, dogs, horses, chickens, sheep, goats, pigs, turkeys and cattle, from parasites that infest or infect such animals.

These and other embodiments are disclosed or are obvious from and encompassed by, the following Detailed Description.

### DETAILED DESCRIPTION

### Long-acting Injectable Compositions

In a first aspect, the present invention provides for novel and inventive long-acting injectable compositions for the treatment or prevention of parasitic infections or infestations in an animal comprising an antiparasitic effective amount of at least one isoxazoline compound of formula (Ic), a neutral oil that is selected from triglycerides of fractionated plant fatty acids with chain lengths of C₈ to C₁₀, optionally, a co-solvent, optionally, an antioxidant, and, optionally, a pharmaceutically acceptable additive and/or excipient, wherein no other pharmaceutically acceptable polymers, as defined herein, are present.

In another embodiment, the present invention provides for long-acting injectable compositions as defined above for use in the treatment and/or prophylaxis of parasitic infections and infestations in or on animals in need thereof, wherein the parasites are treated or prevented for about 3 to 6 months.

In one embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ia): 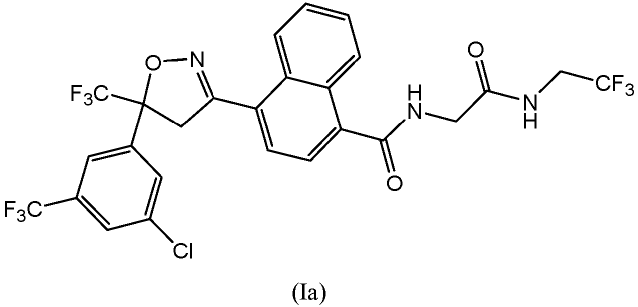 or a pharmaceutically acceptable salt thereof
b) a neutral oil that is selected from triglycerides of fractionated plant fatty acids with chain lengths of C₈ to C₁₀;
c) optionally, at least one co-solvent;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of at least one isoxazoline compound of formula (Ia) as described above, or a pharmaceutically acceptable salt thereof;
b) a neutral oil that is selected from triglycerides of fractionated plant fatty acids with chain lengths of C₈ to C₁₀;
c) optionally, at least one co-solvent, wherein said co-solvent is a polar solvent miscible with water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of at least one isoxazoline compound of formula (Ia) as described above, or a pharmaceutically acceptable salt thereof;
b) a neutral oil that is selected from triglycerides of fractionated plant fatty acids with chain lengths of C₈ to C₁₀;
c) optionally, at least one co-solvent wherein said co-solvent is not miscible with water or only partially soluble in water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of at least one isoxazoline compound of formula (Ia) as described above, or a pharmaceutically acceptable salt thereof;
b) a neutral oil that is selected from triglycerides of fractionated plant fatty acids with chain lengths of C₈ to C₁₀;
c) optionally, at least one co-solvent wherein said co-solvent is ethanol, isopropanol, benzyl alcohol, or a combination thereof;
d) optionally, an antioxidant; and
e) optionally, at least one pharmaceutically acceptable additive, excipient or mixtures thereof;
wherein no other pharmaceutically acceptable polymers are present.

The present invention provides for a long-acting injectable composition for the treatment and/or prevention (prophylaxis) of parasitic infections and infestations in or on animals comprising:
a) an antiparasitic effective amount of an isoxazoline compound of formula (Ic): 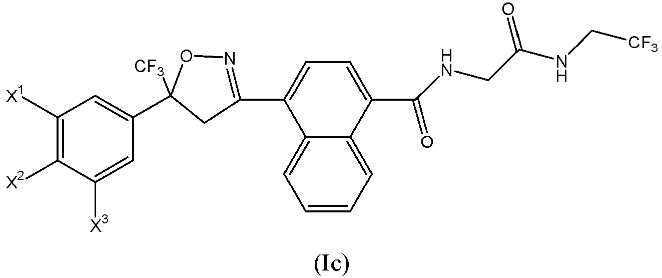
   or a pharmaceutically acceptable salt thereof
   wherein
   X¹, X² and X³ are each independently H, halogen, C₁-C₃alkyl or C₁-C₃haloalkyl;
b) a neutral oil that is selected from triglycerides of fractionated plant fatty acids with chain lengths of C₈ to C₁₀;
c) optionally, at least one co-solvent;
d) optionally, an antioxidant; and
e) optionally, at least one pharmaceutically acceptable additive, excipient or mixtures thereof;
wherein no other pharmaceutically acceptable polymers are present.

In one embodiment, the present invention provides for a long-acting injectable composition for the treatment and/or prevention (prophylaxis) of parasitic infections and infestations in or on animals comprising:
a) an antiparasitic effective amount of at least one isoxazoline compound of formula (Ic) as described above, or a pharmaceutically acceptable salt thereof;
b) a neutral oil that is selected from triglycerides of fractionated plant fatty acids with chain lengths of C₈ to C₁₀;
c) optionally, at least one co-solvent, wherein said co-solvent is a polar solvent miscible with water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of at least one isoxazoline compound of formula (Ic) as described above, or a pharmaceutically acceptable salt thereof;
b) a neutral oil that is selected from triglycerides of fractionated plant fatty acids with chain lengths of C₈ to C₁₀;
c) optionally, at least one co-solvent wherein said co-solvent is not miscible with water or only partially soluble in water;
d) optionally, an antioxidant; and
e) optionally, at least one pharmaceutically acceptable additive, excipient or mixtures thereof;
wherein no other pharmaceutically acceptable polymers are present.

In one embodiment, the long-acting injectable compositions of the invention comprise a compound of formula (Ic) wherein X¹ and X³ are independently halogen and X² is hydrogen.

In another embodiment, the long-acting injectable compositions of the invention comprise a compound of formula (Ic), wherein X¹, X² and X³ are each independently halogen.

In another embodiment of the invention, the long-acting injectable compositions comprise a compound of formula (Ic), wherein X¹ and X³ are each independently halogen and X² is C₁-C₃haloalkyl.

In still another embodiment, the invention provides a long-acting injectable composition comprising a compound of formula (Ic), wherein X¹ and X² are independently halogen and X³ is C₁-C₃haloalkyl.

In another embodiment, the invention provides a long-acting injectable composition comprising a compound of formula (Ic), wherein X¹ and X² are independently halogen and X³ is CF₃.

In another embodiment, the invention provides a long-acting injectable composition comprising a compound of formula (Ic), wherein X¹ and X³ are chloro and X² is hydrogen.

In yet another embodiment, the invention provides a long-acting injectable composition comprising a compound of formula (Ic), wherein X¹ is chloro, X² is fluoro and X³ is CF₃.

In another embodiment, the invention provides a long-acting injectable composition comprising a compound of formula (Ic), wherein X¹ and X³ are chloro and X² is fluoro.

In another embodiment, the long-acting injectable compositions of present invention comprise an antiparasitic effective amount of 4-[5-[3-chloro-5-(trifluoromethyl)phenyl]-4,5-dihydro-5-(trifluoromethyl)-3-isoxazolyl]-N-[2-oxo-2-[(2,2,2-trifluoroethyl)amino]ethyl]-1-naphthalanecarboxamide (Compound of formula Ia).

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic): 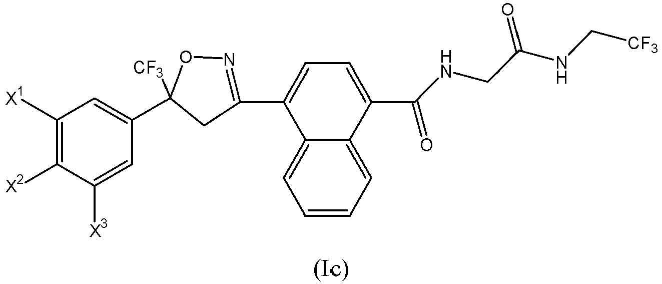
   or a pharmaceutically acceptable salt thereof
   wherein
   X¹ and X³ are each independently halogen or C₁-C₃haloalkyl; and
   X² is halogen or hydrogen;
b) a neutral oil that is selected from triglycerides of fractionated plant fatty acids with chain lengths of C₈ to C₁₀;
c) optionally, at least one co-solvent;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) shown above, or a pharmaceutically acceptable salt thereof,
   wherein
   X¹ and X³ are each independently halogen or C₁-C₃haloalkyl; and
   X² is halogen or hydrogen;
b) a neutral oil that is selected from triglycerides of fractionated plant fatty acids with chain lengths of C₈ to C₁₀;
c) optionally, at least one co-solvent, wherein said co-solvent is a polar solvent miscible with water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of at least one isoxazoline compound of formula (Ic) as described above, or a pharmaceutically acceptable salt thereof;
   wherein
   X¹ and X³ are each independently halogen or C₁-C₃haloalkyl; and
   X² is halogen or hydrogen;
b) a neutral oil that is selected from triglycerides of fractionated plant fatty acids with chain lengths of C₈ to C₁₀;
c) optionally, at least one co-solvent wherein said co-solvent is not miscible with water or only partially soluble in water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as shown above,
   or a pharmaceutically acceptable salt thereof,
   wherein
   X¹ and X² are each independently chloro or fluoro; and
   X³ is chloro or CF₃;
b) a neutral oil that is selected from triglycerides of fractionated plant fatty acids with chain lengths of C₈ to C₁₀;
c) optionally, at least one co-solvent;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as shown above, or a pharmaceutically acceptable salt thereof,
   wherein
   X¹ and X² are each independently chloro or fluoro; and
   X³ is chloro or CF₃;
b) a neutral oil that is selected from triglycerides of fractionated plant fatty acids with chain lengths of C₈ to C₁₀;
c) optionally, at least one co-solvent, wherein said co-solvent is a polar solvent that is miscible with water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of at least one isoxazoline compound of formula (Ic) as described above, or a pharmaceutically acceptable salt thereof;
   wherein
   X¹ and X² are each independently chloro or fluoro; and
   X³ is chloro or CF₃;
b) a neutral oil that is selected from triglycerides of fractionated plant fatty acids with chain lengths of C₈ to C₁₀;
c) optionally, at least one co-solvent wherein said co-solvent is not miscible with water or only partially soluble in water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of at least one isoxazoline compound of formula (Ic) as described above, or a pharmaceutically acceptable salt thereof;
   wherein
   X¹ and X² are each independently chloro or fluoro; and
   X³ is chloro or CF₃;
b) a neutral oil that is selected from triglycerides of fractionated plant fatty acids with chain lengths of C₈ to C₁₀;
c) optionally, at least one co-solvent wherein said co-solvent is ethanol, isopropanol, benzyl alcohol, or a combination thereof;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as shown above, or a pharmaceutically acceptable salt thereof
   wherein
   X¹ and X³ are each chloro; and
   X² is fluoro or hydrogen;
b) a neutral oil that is selected from triglycerides of fractionated plant fatty acids with chain lengths of Cs to C₁₀;
c) optionally, at least one co-solvent;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as shown above, or a pharmaceutically acceptable salt thereof,
   wherein
   X¹ and X³ are each chloro; and
   X² is fluoro or hydrogen;
b) a neutral oil that is selected from triglycerides of fractionated plant fatty acids with chain lengths of C₈ to C₁₀;
c) optionally, at least one co-solvent, wherein said co-solvent is a polar solvent that is miscible with water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of at least one isoxazoline compound of formula (Ic) as described above, or a pharmaceutically acceptable salt thereof;
   wherein
   X¹ and X³ are each chloro; and
   X² is fluoro or hydrogen;
b) a neutral oil that is selected from triglycerides of fractionated plant fatty acids with chain lengths of C₈ to C₁₀;
c) optionally, at least one co-solvent wherein said co-solvent is not miscible with water or only partially soluble in water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as shown above,
   or a pharmaceutically acceptable salt thereof,
   wherein
   X¹ is chloro;
   X² is fluoro; and
   X³ is CF₃;
b) a neutral oil that is selected from triglycerides of fractionated plant fatty acids with chain lengths of C₈ to C₁₀;
c) optionally, at least one co-solvent;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as described above, or a pharmaceutically acceptable salt thereof,
   wherein
   X¹ is chloro;
   X² is fluoro; and
   X³ is CF₃;
b) a neutral oil that is selected from triglycerides of fractionated plant fatty acids with chain lengths of C₈ to C₁₀;
c) optionally, at least one co-solvent, wherein said co-solvent is a polar solvent that is miscible with water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of at least one isoxazoline compound of formula (Ic) as described above, or a pharmaceutically acceptable salt thereof;
   wherein
   X¹ is chloro;
   X² is fluoro; and
   X³ is CF₃;
b) a neutral oil that is selected from triglycerides of fractionated plant fatty acids with chain lengths of C₈ to C₁₀;
c) optionally, at least one co-solvent wherein said co-solvent is not miscible with water or only partially soluble in water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of at least one isoxazoline compound of formula (Ic) as described above, or a pharmaceutically acceptable salt thereof;
   wherein
   X¹ is chloro;
   X² is fluoro; and
   X³ is CF₃;
b) a neutral oil that is selected from triglycerides of fractionated plant fatty acids with chain lengths of C₈ to C₁₀;
c) optionally, a co-solvent, wherein the co-solvent is a C₁-C₆alcohol, a glycol ether (e.g., including, but limited to, diethyleneglycol monoethyl ether, butyl diglycol, dipropylene glycol n-butyl ether, ethyleneglycol monoethyl ether, ethyleneglycol monomethyl ether, dipropylene glycol monomethyl ether, propylene glycol monomethyl ether, propylene glycol monoethyl ether, and the like), glycerol, propylene glycol, cyclic carbonates (e.g., propylene carbonate), 2-pyrrolidone, N-methylpyrrolidone, dimethyl isosorbide (DMI), dimethylacetamide, dimethylsulfoxide or glycerol formal, or a combination thereof;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of at least one isoxazoline compound of formula (Ic) as described above, or a pharmaceutically acceptable salt thereof;
   wherein
   X¹ is chloro;
   X² is fluoro; and
   X³ is CF₃;
b) a neutral oil that is selected from triglycerides of fractionated plant fatty acids with chain lengths of C₈ to C₁₀;
c) optionally, at least one co-solvent wherein said co-solvent is a C₁-C₆alcohol, benzyl alcohol, or a combination thereof;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of at least one isoxazoline compound of formula (Ic) as described above, or a pharmaceutically acceptable salt thereof;
   wherein
   X¹ is chloro;
   X² is fluoro; and
   X³ is CF₃;
b) a neutral oil that is selected from triglycerides of fractionated plant fatty acids with chain lengths of C₈ to C₁₀;
c) optionally, at least one co-solvent wherein said co-solvent is ethanol, isopropanol, benzyl alcohol, or a combination thereof;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of at least one isoxazoline compound of formula (Ic) as described above, or a pharmaceutically acceptable salt thereof;
   wherein
   X¹ is chloro;
   X² is fluoro; and
   X³ is CF₃;
b) a neutral oil that is selected from triglycerides of fractionated plant fatty acids with chain lengths of C₈ to C₁₀;
c) optionally, at least one co-solvent wherein said co-solvent is ethanol, isopropanol, benzyl alcohol, or a combination thereof;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as shown above, or a pharmaceutically acceptable salt thereof,
   wherein
   X¹ and X³ are chloro; and
   X² is fluoro;
b) a neutral oil that is selected from triglycerides of fractionated plant fatty acids with chain lengths of C₈ to C₁₀;
c) optionally, at least one co-solvent;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as described above, or a pharmaceutically acceptable salt thereof;
   wherein
   X¹ and X³ are chloro; and
   X² is fluoro;
b) a neutral oil that is selected from triglycerides of fractionated plant fatty acids with chain lengths of C₈ to C₁₀;
c) optionally, at least one co-solvent, wherein said co-solvent is a polar solvent that is miscible with water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as described above, or a pharmaceutically acceptable salt thereof
   wherein
   X¹ and X³ are chloro; and
   X² is fluoro;
b) a neutral oil that is selected from triglycerides of fractionated plant fatty acids with chain lengths of C₈ to C₁₀;
c) optionally, at least one co-solvent wherein said co-solvent is not miscible with water or only partially soluble in water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as described above, or a pharmaceutically acceptable salt thereof
   wherein
   X¹ and X³ are chloro; and
   X² is fluoro;
b) a neutral oil that is selected from triglycerides of fractionated plant fatty acids with chain lengths of C₈ to C₁₀;
c) optionally, a co-solvent, wherein the co-solvent is a C₁-C₆alcohol, a glycol ether (e.g., including, but limited to, diethyleneglycol monoethyl ether, butyl diglycol, dipropylene glycol n-butyl ether, ethyleneglycol monoethyl ether, ethyleneglycol monomethyl ether, dipropylene glycol monomethyl ether, propylene glycol monomethyl ether, propylene glycol monoethyl ether, and the like), glycerol, propylene glycol, cyclic carbonates (e.g., propylene carbonate), 2-pyrrolidone, N-methylpyrrolidone, dimethyl isosorbide (DMI), dimethylacetamide, dimethylsulfoxide or glycerol formal, or a combination thereof;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as described above, or a pharmaceutically acceptable salt thereof
   wherein
   X¹ and X³ are chloro; and
   X² is fluoro;
b) a neutral oil that is selected from triglycerides of fractionated plant fatty acids with chain lengths of C₈ to C₁₀;
c) optionally, at least one co-solvent wherein said co-solvent is ethanol, isopropanol, benzyl alcohol, or a combination thereof;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as shown above,
   or a pharmaceutically acceptable salt thereof,
   wherein
   X¹, X² and X³ are each chloro;
b) a neutral oil that is selected from triglycerides of fractionated plant fatty acids with chain lengths of C₈ to C₁₀;
c) optionally, at least one co-solvent;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as described above, or a pharmaceutically acceptable salt thereof
   wherein
   X¹, X² and X³ are each chloro;
b) a neutral oil that is selected from triglycerides of fractionated plant fatty acids with chain lengths of C₈ to C₁₀;
c) optionally, at least one co-solvent, wherein said co-solvent is a polar solvent that is miscible with water;
d) optionally, an antioxidant; and
e) optionally, at least one pharmaceutically acceptable additive, excipient or mixtures thereof;
wherein no other pharmaceutically acceptable polymers are present.

**In** another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as described above, or a pharmaceutically acceptable salt thereof
   wherein
   X¹, X² and X³ are each chloro;
b) a neutral oil that is selected from triglycerides of fractionated plant fatty acids with chain lengths of C₈ to C₁₀;
c) optionally, at least one co-solvent wherein said co-solvent is not miscible with water or only partially soluble in water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as described above, or a pharmaceutically acceptable salt thereof
   wherein
   X¹, X² and X³ are each chloro;
b) a neutral oil that is selected from triglycerides of fractionated plant fatty acids with chain lengths of C₈ to C₁₀;
c) optionally, a co-solvent, wherein the co-solvent is a C₁-C₆alcohol, a glycol ether (e.g., including, but limited to, diethyleneglycol monoethyl ether, butyl diglycol, dipropylene glycol n-butyl ether, ethyleneglycol monoethyl ether, ethyleneglycol monomethyl ether, dipropylene glycol monomethyl ether, propylene glycol monomethyl ether, propylene glycol monoethyl ether, and the like), glycerol, propylene glycol, cyclic carbonates (e.g., propylene carbonate), 2-pyrrolidone, N-methylpyrrolidone, dimethyl isosorbide (DMI), dimethylacetamide, dimethylsulfoxide or glycerol formal, or a combination thereof;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as described above, or a pharmaceutically acceptable salt thereof
   wherein
   X¹, X² and X³ are each chloro;
b) a neutral oil that is selected from triglycerides of fractionated plant fatty acids with chain lengths of C₈ to C₁₀
c) optionally, a co-solvent, wherein the co-solvent is a C₁-C₆alcohol, benzyl alcohol, or a combination thereof;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as shown above,
   or a pharmaceutically acceptable salt thereof,
   wherein
   X¹, X² and X³ are each independently chloro or fluoro;
b) a neutral oil that is selected from triglycerides of fractionated plant fatty acids with chain lengths of C₈ to C₁₀;
c) optionally, at least one co-solvent;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as described above, or a pharmaceutically acceptable salt thereof
   wherein
   X¹, X² and X³ are each independently chloro or fluoro;
b) a neutral oil that is selected from triglycerides of fractionated plant fatty acids with chain lengths of C₈ to C₁₀;
c) optionally, at least one co-solvent, wherein said co-solvent is a polar solvent that is miscible with water;
d) optionally, an antioxidant; and
e) optionally, at least one pharmaceutically acceptable additive, excipient or mixtures thereof;
wherein no other pharmaceutically acceptable polymers are present.

In another embodiment, the present invention provides for a long-acting injectable composition for the treatment and/or prevention (prophylaxis) of parasitic infections and infestations in or on animals comprising:
a) an antiparasitic effective amount of an isoxazoline compound of formula (Ic) as described above, or a pharmaceutically acceptable salt thereof
   wherein
   X¹, X² and X³ are each independently chloro or fluoro;
b) a neutral oil that is selected from triglycerides of fractionated plant fatty acids with chain lengths of C₈ to C₁₀;
c) optionally, at least one co-solvent wherein said co-solvent is not miscible with water or only partially soluble in water;
d) optionally, an antioxidant; and
e) optionally, at least one pharmaceutically acceptable additive, excipient or mixtures thereof;
wherein no other pharmaceutically acceptable polymers are present.

The compounds of formula (I) can exist as stereoisomers, and each individual stereoisomer present are encompassed by the structural formulas depicted herein. The various stereoisomers include enantiomers, diastereomers and atop isomers. For avoidance of doubt, when an isoxazoline compound (e.g. any of the isoxazoline active agents as described herein) includes two or more stereoisomers (e.g. an (*S*)- and (*R*)-enantiomers), the formulae depicted herein that does not explicitly include stereochemical configurations encompasses each of the possible stereoisomers. One of skill in the art will understand that one stereoisomer of an active isoxazoline compound may be more active and/or may exhibit beneficial properties relative to the other enantiomer. In addition, the skilled person in the art knows how to separate, enrich, and/or selectively prepare a stereoisomer of the isoxazoline compounds described herein. The isoxazoline compounds described herein contain a chiral quaternary carbon atom in the five-membered isoxazoline ring (shown by the asterisk (*) in the structures below); therefore, the compounds will contain at least two possible stereoisomers. As an example for the compounds of formula (Ia), the two possible stereoisomers resulting from the quaternary carbon are shown as formulae (*S*)-Ia and (*R*)-Ia:

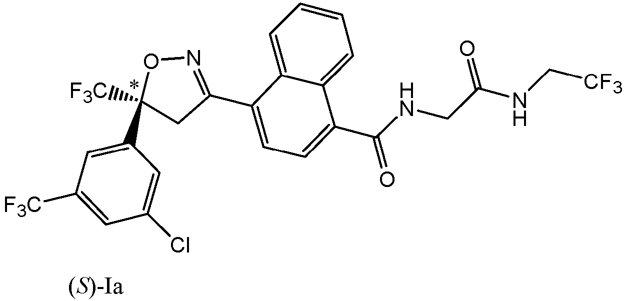

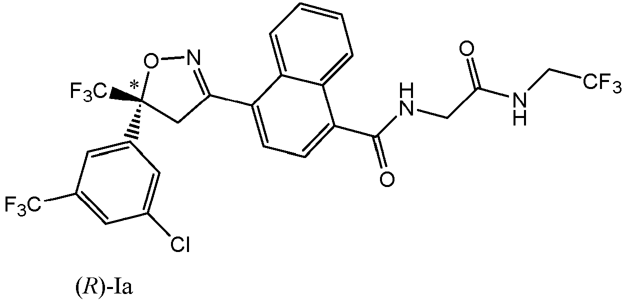

The compound of formula (*S*)-Ia above has the (*S*) configuration at the chiral carbon atom and the compound of formula (*R*)-Ia has the (*R*) configuration. Molecular depictions drawn herein follow standard conventions for depicting stereochemistry. To indicate stereo configuration, bonds rising from the plane of the drawing and towards the viewer are denoted by solid wedges wherein the broad end of the wedge is attached to the atom rising from the plane of the drawing towards the viewer. Bonds going below the plane of the drawing and away from the viewer are denoted by dashed wedges wherein the narrow end of the wedge is attached to the atom further away from the viewer. Constant width lines indicate bonds with a direction opposite or neutral relative to bonds shown with solid or dashed wedges; constant width lines also depict bonds in molecules or parts of molecules in which no particular stereo configuration is intended to be specified.

In one embodiment of the invention, the more biologically active enantiomer is believed to be formula (*S*)-Ia. Similarly, the more biologically active enantiomers of isoxazoline compounds of formula (Ic) are believed to have the (*S*) configuration at the chiral carbon of the isoxazoline ring. In certain embodiments, an isoxazoline compound of the invention, or compositions comprising the compound, are enriched in an enantiomer that displays significant in vitro and in vivo activity (the eutomer) with a favorable toxicity profile relative to a compound or a composition enriched with the other corresponding enantiomer that displays significantly less in vitro and in vivo activity (the distomer).

When enantiomerically enriched, one enantiomer is present in greater amounts than the other, and the extent of enrichment may be defined by an expression of enantiomeric excess ("ee"), which is defined as (2x-1)·100 %, where x is the mole fraction of the dominant enantiomer in the mixture (e.g., an ee of 20 % corresponds to a 60:40 ratio of enantiomers). In some embodiments, the compositions of the invention comprise compounds that have at least a 50 % enantiomeric excess. In other embodiments, the compositions of the invention comprise compounds that have at least a 75 % enantiomeric excess, at least a 90 % enantiomeric excess, or at least a 94 % enantiomeric excess of the more active isomer. Of particular note are enantiomerically pure embodiments of the more active isomer (the eutomer).

Compounds of this invention may also exist as one or more conformational isomers due to restricted rotation about the amide bond bonded to the aryl or heteroaryl ring (e.g. the amide bonded to the naphthyl group in formula (I)). This invention comprises mixtures of conformational isomers. In addition, this invention includes compounds that are enriched in one conformer relative to others.

It will be appreciated that in addition to the compounds of formula (Ia), isoxazoline compounds of formula (Ic) will also have at least two possible enantiomers as a result of the quaternary carbon atom on the isoxazoline ring. In addition, certain compounds may include other chiral centers in one or more substituents.

This invention comprises racemic mixtures, for example, equal amounts of the enantiomers of formulae (I). The invention also includes compounds of formula (Ia), or formula (Ic) that are enriched in one enantiomer compared to the racemic mixture. Also included are the essentially pure enantiomers of the compounds of formula (Ia), or formula (Ic).

Hence, in one embodiment, the long-acting injectable compositions of the present invention comprise an antiparasitic effective amount of at least one isoxazoline of formula (Ia), or formula (Ic), which is substantially enriched in one enantiomer, or a pharmaceutically acceptable salt thereof. The term "substantially enriched" means that the compound is enriched in a weight: weight ratio of at least about 1.5 or higher in favor of the desired enantiomer. In another embodiment, the long-acting compositions of the invention comprise at least one isoxazoline compound of formula (Ia), or formula (Ic) that are enriched in one enantiomer in a weight:weight ratio of at least 2:1, at least 5:1 or at least 10:1. In another embodiment, the compositions comprise at least one compound of formula (Ia), or formula (Ic), which is enriched in one enantiomer in a weight: weight ratio of at least 15:1 or at least 20:1, or a pharmaceutically acceptable salt thereof. In an embodiment, the isoxazoline compounds of formula (Ia), or formula (Ic) present in the compositions of the invention are essentially pure enantiomers.

In another embodiment of the invention, the compositions comprise a compound of formula (Ia), or formula (Ic) that is enriched in the (*S*)-enantiomer in a weight:weight ratio is at least approximately 1.5:1 or 2:1. In yet another embodiment, the compositions of the invention comprise a compound of formula (Ia), or formula (Ic) that is enriched in the (S)-enantiomer in a weight: weight ratio of at least about 5:1 or greater. In still another embodiment, the compositions of the invention comprise a compound of formula (Ia), or formula (Ic) that is enriched in the (S)-enantiomer in a weight:weight ratio of at least approximately 10:1, 20:1, or greater. In still another embodiment, the compositions of the invention comprise a compound of formula (Ia), or formula (Ic) that is essentially the pure (S)-enantiomer.

In one embodiment, the compositions of the invention comprise a compound of (Ia), or (Ic) that is substantially enriched in an enantiomer. In another embodiment, the long-acting injectable compositions of the invention comprise a compound of (Ia), or (Ic) that is substantially enriched in the (*S*)-enantiomer. In another embodiment, the long-acting injectable compositions of the invention comprise a compound of (Ia), or (Ic) that is substantially enriched in the (*R*)-enantiomer.

In another embodiment of the invention, the compositions comprise a compound of (Ia), or (Ic) that is enriched in the (*S*)-enantiomer in a weight:weight ratio is at least approximately 1.5:1 or 2:1 or greater. In yet another embodiment, the compositions of the invention comprise a compound of (Ia), or (Ic) that is enriched in the (*S*)-enantiomer in a weight:weight ratio of at least about 5:1 or greater. In still another embodiment, the compositions of the invention comprise a compound of (Ia), or (Ic) that is enriched in the (*S*)-enantiomer in a weight:weight ratio of at least approximately 10:1, 20:1, or greater. In still another embodiment, the compositions of the invention comprise a compound of (Ia), or (Ic) that is essentially the pure (*S*)-enantiomer.

In another embodiment of the invention, the compositions comprise a compound of (Ia), or (Ic) that is enriched in the (*R*)-enantiomer in a weight:weight ratio is at least approximately 2:1 or greater. In yet another embodiment, the compositions of the invention comprise a compound of (Ia), or (Ic) that is enriched in the (*R*)-enantiomer in a weight:weight ratio of at least about 5:1 or greater. In still another embodiment, the compositions of the invention comprise a compound of (Ia), or (Ic) that is enriched in the (*R*)-enantiomer in a weight: weight ratio of at least about 10:1, 20:1, or greater. In still another embodiment, the compositions of the invention comprise a compound of (Ia), or (Ic) that is essentially the pure (*R*)-enantiomer.

In one embodiment, the compositions of the invention may comprise about 0.5 to about 50% (w/v) of an isoxazoline active agent of any of formulae (Ia), or (Ic) or a pharmaceutically acceptable salt thereof, either as a racemic mixture or enriched in an enantiomer as described above. In another embodiment, the compositions of the invention may comprise about 1 to about 40% (w/v) of an isoxazoline active agent of any of formulae (Ia), or (Ic) or a pharmaceutically acceptable salt thereof. In yet another embodiment, the compositions of the invention may comprise about 1 to about 30% (w/v), about 1 to about 20% (w/v) or about 1 to about 15% (w/v) of an isoxazoline active agent of any of formulae (Ia), or (Ic) or a pharmaceutically acceptable salt thereof. In another embodiment, the compositions of the invention may comprise about 0.5 to about 10% (w/v) or about 0.5% to about 5% (w/v) of an isoxazoline active agent of any of formulae (Ia), or (Ic) or a pharmaceutically acceptable salt thereof.

In another embodiment, the compositions of the invention may comprise about 5 to about 40% (w/v) or about 5 to about 30% (w/v) of an isoxazoline active agent, or a pharmaceutically acceptable salt thereof. In another embodiment, the compositions may comprise about 10% to about 40% (w/v) of an isoxazoline active agent, or a pharmaceutically acceptable salt thereof. In yet another embodiment, the compositions of the invention may comprise about 15% to about 40% (w/v), about 15% to about 35% (w/v) or about 15% to about 30% (w/v) of an isoxazoline compound, or a pharmaceutically acceptable salt thereof.

In certain embodiments the present invention provides for long-acting injectable compositions for the treatment and/or prevention of parasitic infections and infestations in or on animals comprising:
a) about 0.5 to 30% (w/v) of an isoxazoline active agent as defined herein, or a pharmaceutically acceptable salt thereof;
b) a neutral oil that is selected from triglycerides of fractionated plant fatty acids with chain lengths of C₈ to C₁₀;
c) optionally, about 1% to 40% (w/v) of co-solvent;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In another embodiment the present invention provides for long-acting injectable compositions for the treatment and/or prevention of parasitic infections and infestations in or on animals comprising:
a) about 0.5 to 20% (w/v) of an isoxazoline active agent as defined herein, or a pharmaceutically acceptable salt thereof;
b) a neutral oil that is selected from triglycerides of fractionated plant fatty acids with chain lengths of C₈ to C₁₀;
c) optionally, about 1% to 40% (w/v) of co-solvent;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In certain embodiments the present invention provides for long-acting injectable compositions for the treatment and/or prevention of parasitic infections and infestations in or on animals comprising:
a) about 5 to 30% (w/v) of an isoxazoline active agent as defined herein, or a pharmaceutically acceptable salt thereof;
b) a neutral oil that is selected from triglycerides of fractionated plant fatty acids with chain lengths of C₈ to C₁₀;
c) optionally, about 1% to 40% (w/v) of co-solvent;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In certain embodiments the present invention provides for long-acting injectable compositions for the treatment and/or prevention of parasitic infections and infestations in or on animals comprising:
a) about 0.5 to 30% (w/v) of an isoxazoline active agent as defined herein, or a pharmaceutically acceptable salt thereof;
b) a neutral oil that is selected from triglycerides of fractionated plant fatty acids with chain lengths of C₈ to C₁₀;
c) optionally, about 1% to 40% (w/v) of co-solvent, wherein said co-solvent is a polar solvent that is miscible with water;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In another embodiment the present invention provides for long-acting injectable compositions for the treatment and/or prevention of parasitic infections and infestations in or on animals comprising:
a) about 0.5 to 20% (w/v) of an isoxazoline active agent as defined herein, or a pharmaceutically acceptable salt thereof;
b) a neutral oil that is selected from triglycerides of fractionated plant fatty acids with chain lengths of C₈ to C₁₀;
c) optionally, about 1% to 40% (w/v) of co-solvent, wherein said co-solvent is a polar solvent that is miscible with water;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In certain embodiments the present invention provides for long-acting injectable compositions for the treatment and/or prevention of parasitic infections and infestations in or on animals comprising:
a) about 5 to 30% (w/v) of an isoxazoline active agent as defined herein, or a pharmaceutically acceptable salt thereof;
b) a neutral oil that is selected from triglycerides of fractionated plant fatty acids with chain lengths of C₈ to C₁₀;
c) optionally, about 1% to 40% (w/v) of co-solvent, wherein said co-solvent is a polar solvent that is miscible with water;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In certain embodiments the present invention provides for long-acting injectable compositions for the treatment and/or prophylaxis of parasitic infections and infestations in or on animals comprising:
a) about 0.5 to 30% (w/v) of an isoxazoline active agent of the formula (Ia), (e.g., a compound of formulae I), such as, a compound of the formula: 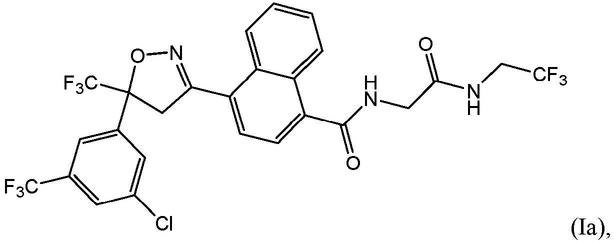 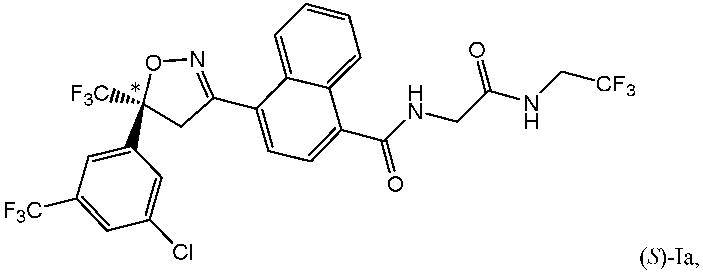 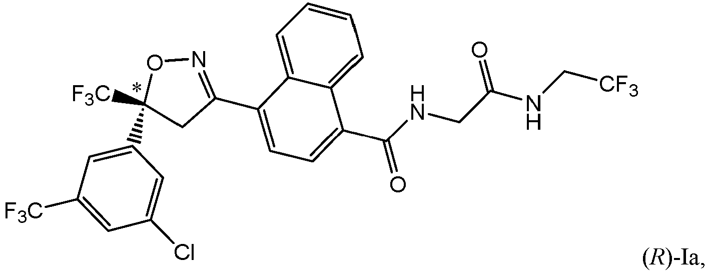 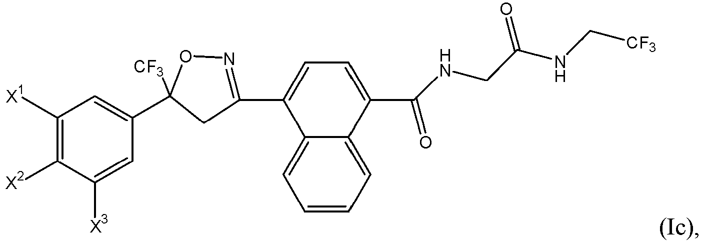 wherein X¹, X² and X³ are independently chloro, fluoro or CF₃; 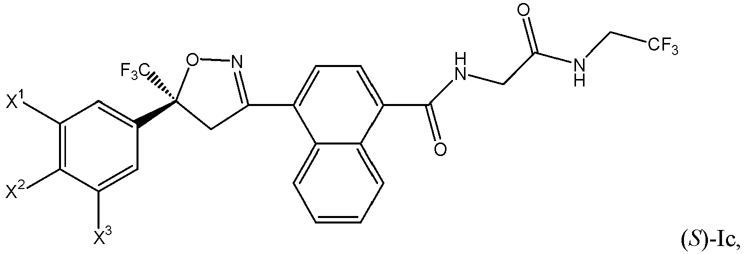 wherein X¹, X² and X³ are independently chloro, fluoro or CF₃; 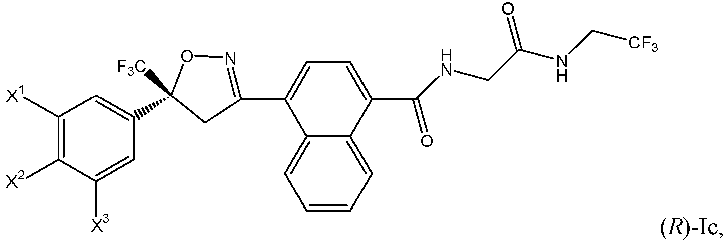 wherein X¹, X² and X³ are independently chloro, fluoro or CF₃;
   or a pharmaceutically acceptable salt thereof,
b) a neutral oil that is selected from triglycerides of fractionated plant fatty acids with chain lengths of C₈ to C₁₀;
c) optionally, about 1% to 40% (w/v) of a co-solvent, wherein the co-solvent is a C₁-C₆alcohol, a glycol ether (e.g., including, but limited to, diethyleneglycol monoethyl ether, butyl diglycol, dipropylene glycol n-butyl ether, ethyleneglycol monoethyl ether, ethyleneglycol monomethyl ether, dipropylene glycol monomethyl ether, propylene glycol monomethyl ether, propylene glycol monoethyl ether, and the like), glycerol, propylene glycol, cyclic carbonates (e.g., propylene carbonate), 2-pyrrolidone, N-methylpyrrolidone, dimethyl isosorbide (DMI), dimethylacetamide, dimethylsulfoxide or glycerol formal, or a combination thereof;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In certain embodiments the present invention provides for long-acting injectable compositions for the treatment and/or prophylaxis of parasitic infections and infestations in or on animals comprising:
a) about 0.5 to 20% (w/v) of an isoxazoline active agent of the formula (Ia), (S)-Ia, (R)-Ia, (Ic), wherein X¹, X² and X³ are independently chloro, fluoro or CF₃; (S)-Ic, wherein X¹, X² and X³ are independently chloro, fluoro or CF₃; (R)-Ic wherein X¹, X² and X³ are independently chloro, fluoro or CF₃ as shown above;
   or a pharmaceutically acceptable salt thereof,
b) a neutral oil that is selected from triglycerides of fractionated plant fatty acids with chain lengths of C₈ to C₁₀;
c) optionally, about 1% to 40% (w/v) of co-solvent, wherein the co-solvent is a C₁-C₆alcohol, a glycol ether (e.g., including, but limited to, diethyleneglycol monoethyl ether, butyl diglycol, dipropylene glycol n-butyl ether, ethyleneglycol monoethyl ether, ethyleneglycol monomethyl ether, dipropylene glycol monomethyl ether, propylene glycol monomethyl ether, propylene glycol monoethyl ether, and the like), glycerol, propylene glycol, cyclic carbonates (e.g., propylene carbonate), 2-pyrrolidone, N-methylpyrrolidone, dimethyl isosorbide (DMI), dimethylacetamide, dimethylsulfoxide or glycerol formal, or a combination thereof;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In other embodiments the present invention provides for long-acting injectable compositions for the treatment and/or prophylaxis of parasitic infections and infestations in or on animals comprising:
a) about 1 to 20% (w/v) of an isoxazoline active agent of the formula (Ia), (*S*)-Ia, (*R*)-Ia, (Ic), wherein X¹, X² and X³ are independently chloro, fluoro or CF₃; (*S*)-Ic, wherein X¹, X² and X³ are independently chloro, fluoro or CF₃; (R)-Ic wherein X¹, X² and X³ are independently chloro, fluoro or CF₃ as shown above;
   or a pharmaceutically acceptable salt thereof,
b) a neutral oil that is selected from triglycerides of fractionated plant fatty acids with chain lengths of C₈ to C₁₀;
c) optionally, about 1% to 40% (w/v) of co-solvent, wherein the co-solvent is a C₁-C₆alcohol, a glycol ether (e.g., including, but limited to, diethyleneglycol monoethyl ether, butyl diglycol, dipropylene glycol n-butyl ether, ethyleneglycol monoethyl ether, ethyleneglycol monomethyl ether, dipropylene glycol monomethyl ether, propylene glycol monomethyl ether, propylene glycol monoethyl ether, and the like), glycerol, propylene glycol, cyclic carbonates (e.g., propylene carbonate), 2-pyrrolidone, N-methylpyrrolidone, dimethyl isosorbide (DMI), dimethylacetamide, dimethylsulfoxide or glycerol formal, or a combination thereof;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In other embodiments the present invention provides for long-acting injectable compositions for the treatment and/or prophylaxis of parasitic infections and infestations in or on animals comprising:
a) about 1 to 15% (w/v) of an isoxazoline active agent of the formula (Ia), (*S*)-Ia, (*R*)-Ia, (Ic), wherein X¹, X² and X³ are independently chloro, fluoro or CF₃; (S)-Ic, wherein X¹, X² and X³ are independently chloro, fluoro or CF₃; (*R*)-Ic wherein X¹, X² and X³ are independently chloro, fluoro or CF₃ as shown above;
   or a pharmaceutically acceptable salt thereof,
b) a neutral oil that is selected from triglycerides of fractionated plant fatty acids with chain lengths of C₈ to C₁₀;
c) optionally, about 1% to 40% (w/v) of co-solvent, wherein the co-solvent is a C₁-C₆alcohol, a glycol ether (e.g., including, but limited to, diethyleneglycol monoethyl ether, butyl diglycol, dipropylene glycol n-butyl ether, ethyleneglycol monoethyl ether, ethyleneglycol monomethyl ether, dipropylene glycol monomethyl ether, propylene glycol monomethyl ether, propylene glycol monoethyl ether, and the like), glycerol, propylene glycol, cyclic carbonates (e.g., propylene carbonate), 2-pyrrolidone, N-methylpyrrolidone, dimethyl isosorbide (DMI), dimethylacetamide, dimethylsulfoxide or glycerol formal, or a combination thereof;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In certain embodiments the present invention provides for long-acting injectable compositions for the treatment and/or prophylaxis of parasitic infections and infestations in or on animals comprising:
a) about 0.5 to 30% (w/v) of an isoxazoline active agent of the formula (Ia), (S)-Ia, (Ic), wherein X¹, X² and X³ are independently chloro, fluoro or CF₃; (S)-Ic, wherein X¹, X² and X³ are independently chloro, fluoro or CF₃ as shown above, or a pharmaceutically acceptable salt thereof;
b) a neutral oil that is selected from triglycerides of fractionated plant fatty acids with chain lengths of C₈ to C₁₀;
c) optionally, about 1% to 40% (w/v) of a co-solvent, wherein the co-solvent is a C₁-C₆alcohol, benzyl alcohol, or a combination thereof;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In certain embodiments the present invention provides for long-acting injectable compositions for the treatment and/or prophylaxis of parasitic infections and infestations in or on animals comprising:
a) about 0.5 to 20% (w/v) of an isoxazoline active agent of the formula (Ia), (*S*)-Ia, (Ic), wherein X¹, X² and X³ are independently chloro, fluoro or CF₃; (S)-Ic, wherein X¹, X² and X³ are independently chloro, fluoro or CF₃ as shown above, or a pharmaceutically acceptable salt thereof;
b) a neutral oil that is selected from triglycerides of fractionated plant fatty acids with chain lengths of C₈ to C₁₀;
c) optionally, about 1% to 40% (w/v) of co-solvent, wherein the co-solvent is a C₁-C₆alcohol, benzyl alcohol, or a combination thereof;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In other embodiments the present invention provides for long-acting injectable compositions for the treatment and/or prophylaxis of parasitic infections and infestations in or on animals comprising:
a) about 1 to 20% (w/v) of an isoxazoline active agent of the formula (Ia), (*S*)-Ia, (Ic), wherein X¹, X² and X³ are independently chloro, fluoro or CF₃; (S)-Ic, wherein X¹, X² and X³ are independently chloro, fluoro or CF₃ as shown above, or a pharmaceutically acceptable salt thereof;
b) a neutral oil that is selected from triglycerides of fractionated plant fatty acids with chain lengths of C₈ to C₁₀;
c) optionally, about 1% to 40% (w/v) of co-solvent, wherein the co-solvent is a C₁-C₆alcohol, benzyl alcohol, or a combination thereof;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In other embodiments the present invention provides for long-acting injectable compositions for the treatment and/or prophylaxis of parasitic infections and infestations in or on animals comprising:
a) about 1 to 15% (w/v) of an isoxazoline active agent of the formula (Ia), (*S*)-Ia, (Ic), wherein X¹, X² and X³ are independently chloro, fluoro or CF₃; (*S*)-Ic, wherein X¹, X² and X³ are independently chloro, fluoro or CF₃; as shown above, or a pharmaceutically acceptable salt thereof;
b) a neutral oil that is selected from triglycerides of fractionated plant fatty acids with chain lengths of C₈ to C₁₀;
c) optionally, about 1% to 40% (w/v) of co-solvent, wherein the co-solvent is a C₁-C₆alcohol, benzyl alcohol, or a combination thereof;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In certain embodiments the present invention provides for long-acting injectable compositions for the treatment and/or prophylaxis of parasitic infections and infestations in or on animals comprising:
a) about 0.5 to 30% (w/v) of an isoxazoline active agent of the formula (Ia), (*S*)-Ia, (Ic), wherein X¹ is chloro, X² is fluoro and X³ is CF₃; or (*S*)-Ic, wherein X¹ is chloro, X² is fluoro and X³ is CF₃, as shown above, or a pharmaceutically acceptable salt thereof;
b) a neutral oil that is selected from triglycerides of fractionated plant fatty acids with chain lengths of C₈ to C₁₀;
c) optionally, about 1% to 40% (w/v) of a co-solvent, wherein said co-solvent is ethanol, isopropanol, benzyl alcohol, or a combination thereof;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In certain embodiments the present invention provides for long-acting injectable compositions for the treatment and/or prophylaxis of parasitic infections and infestations in or on animals comprising:
a) about 0.5 to 30% (w/v) of an isoxazoline active agent of the formula (Ia), (S)-Ia, (Ic), wherein X¹ is chloro, X² is fluoro and X³ is CF₃; or (S)-Ic, wherein X¹ is chloro, X² is fluoro and X³ is CF₃, as shown above, or a pharmaceutically acceptable salt thereof;
b) a neutral oil that is selected from triglycerides of fractionated plant fatty acids with chain lengths of C₈ to C₁₀;
c) optionally, about 1% to 20% (w/v) of a co-solvent, wherein the co-solvent is ethanol, isopropanol or benzyl alcohol, or a combination thereof;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In certain embodiments the present invention provides for long-acting injectable compositions for the treatment and/or prophylaxis of parasitic infections and infestations in or on animals comprising:
a) about 0.5 to 20% (w/v) of an isoxazoline active agent of the formula (Ia), (S)-Ia, (Ic), wherein X¹ is chloro, X² is fluoro and X³ is CF₃; or (*S*)-Ic, wherein X¹ is chloro, X² is fluoro and X³ is CF₃, as shown above, or a pharmaceutically acceptable salt thereof;
b) a neutral oil that is selected from triglycerides of fractionated plant fatty acids with chain lengths of C₈ to C₁₀;
c) optionally, about 1% to 20% (w/v) of co-solvent, wherein the co-solvent is ethanol, isopropanol or benzyl alcohol, or a combination thereof;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In other embodiments the present invention provides for long-acting injectable compositions for the treatment and/or prophylaxis of parasitic infections and infestations in or on animals comprising:
a) about 1 to 20% (w/v) of an isoxazoline active agent of the formula (Ia), (*S*)-Ia, (Ic), wherein X¹ is chloro, X² is fluoro and X³ is CF₃; or (*S*)-Ic, wherein X¹ is chloro, X² is fluoro and X³ is CF₃, as shown above, or a pharmaceutically acceptable salt thereof;
b) a neutral oil that is selected from triglycerides of fractionated plant fatty acids with chain lengths of C₈ to C₁₀;
c) optionally, about 1% to 20% (w/v) of co-solvent, wherein the co-solvent is ethanol, isopropanol or benzyl alcohol, or a combination thereof;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In other embodiments the present invention provides for long-acting injectable compositions for the treatment and/or prophylaxis of parasitic infections and infestations in or on animals comprising:
a) about 1 to 15% (w/v) of an isoxazoline active agent of the formula (Ia), (S)-Ia, (Ic), wherein X¹ is chloro, X² is fluoro and X³ is CF₃; or (S)-Ic, wherein X¹ is chloro, X² is fluoro and X³ is CF₃, as shown above, or a pharmaceutically acceptable salt thereof;
b) a neutral oil that is selected from triglycerides of fractionated plant fatty acids with chain lengths of C₈ to C₁₀;
c) optionally, about 1% to 20% (w/v) of co-solvent, wherein the co-solvent is ethanol, isopropanol or benzyl alcohol, or a combination thereof;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In other embodiments the present invention provides for long-acting injectable compositions for the treatment and/or prophylaxis of parasitic infections and infestations in or on animals comprising:
a) about 1 to 15% (w/v) of an isoxazoline active agent of the formula (Ia), (*S*)-Ia, (Ic), wherein X¹ is chloro, X² is fluoro and X³ is CF₃; or (S)-Ic, wherein X¹ is chloro, X² is fluoro and X³ is CF₃, as shown above, or a pharmaceutically acceptable salt thereof;
b) a neutral oil that is selected from triglycerides of fractionated plant fatty acids with chain lengths of C₈ to C₁₀;
c) optionally, about 1% to 20% (w/v) at least one co-solvent, wherein said co-solvent is ethanol, isopropanol, benzyl alcohol, or a combination thereof;
d) optionally, about 0.01% to about 2.0% (w/v) of an antioxidant; and
e) optionally about 0.01% to about 5.0% (w/v) of at least one pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In certain embodiments the present invention provides for long-acting injectable compositions for the treatment and/or prophylaxis of parasitic infections and infestations in or on animals comprising:
a) about 0.5 to 30% (w/v) of an isoxazoline active agent as defined herein, such as, a compound of the formula (Ia), (*S*)-Ia, (*R*)-Ia, (Ic), wherein X¹, X² and X³ are independently chloro, fluoro or CF₃; (*S*)-Ic, wherein X¹, X² and X³ are independently chloro, fluoro or CF₃; (*R*)-Ic wherein X¹, X² and X³ are independently chloro, fluoro or CF₃ as shown above;
b) a neutral oil that is selected from triglycerides of fractionated plant fatty acids with chain lengths of C₈ to C₁₀;
c) optionally, about 5% to 40% (w/v) of co-solvent selected from the group consisting of ethanol and isopropanol;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and neutral oil is present in the overall composition in a proportion representing the complement to 100% of the composition.

In other embodiments the present invention provides for long-acting injectable compositions for the treatment and/or prophylaxis of parasitic infections and infestations in or on animals comprising:
a) about 1 to 30% (w/v) of an isoxazoline active agent as defined herein, such as, a compound of the formula (Ia), (*S*)-Ia, (*R*)-Ia, (Ic), wherein X¹, X² and X³ are independently chloro, fluoro or CF₃; (*S*)-Ic, wherein X¹, X² and X³ are independently chloro, fluoro or CF₃; (*R*)-Ic wherein X¹, X² and X³ are independently chloro, fluoro or CF₃ as shown above;
b) a neutral oil that is selected from triglycerides of fractionated plant fatty acids with chain lengths of C₈ to C₁₀;
c) optionally, about 5% to 40% (w/v) of co-solvent selected from the group consisting of ethanol and isopropanol;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and the neutral oil is present in the overall composition in a proportion representing the complement to 100% of the composition.

In yet other embodiments the present invention provides for long-acting injectable compositions for the treatment and/or prophylaxis of parasitic infections and infestations in or on animals comprising:
a) about 1 to 20% (w/v) of an isoxazoline active agent as defined herein, such as, a compound of the formula (Ia), (*S*)-Ia, (*R*)-Ia, (Ic), wherein X¹, X² and X³ are independently chloro, fluoro or CF₃; (S)-Ic, wherein X¹, X² and X³ are independently chloro, fluoro or CF₃; (*R*)-Ic wherein X¹, X² and X³ are independently chloro, fluoro or CF₃ as shown above; or a pharmaceutically acceptable salt thereof;
b) a neutral oil that is selected from triglycerides of fractionated plant fatty acids with chain lengths of C₈ to C₁₀;
c) optionally, about 5% to 40% (w/v) of co-solvent selected from the group consisting of ethanol and isopropanol;
d) optionally, about 0.01% to about 2.0% (w/v) of an antioxidant; and
e) optionally about 0.01% to about 5.0% (w/v) of a pharmaceutically acceptable additive, excipient or mixtures thereof;
wherein the only pharmaceutically acceptable polymer present in said long-acting injectable composition is a liquid PEG and wherein the liquid PEG and the neutral oil is present in the overall composition in a proportion representing the complement to 100% of the composition.

Another embodiment of the present invention is a long-acting injectable composition for the treatment and/or prevention of parasitic infections and infestations in or on animals consisting essentially of:
a) an antiparasitic effective amount of at least one isoxazoline active agent as defined herein, and optionally at least one additional active agent as identified in this application;
b) a neutral oil that is selected from triglycerides of fractionated plant fatty acids with chain lengths of C₈ to C₁₀;
c) optionally, at least one co-solvent wherein said co-solvent is a polar solvent miscible with water;
d) optionally, an antioxidant; and
e) optionally, at least one pharmaceutically acceptable additive, excipient or mixtures thereof.

Another embodiment of the present invention is a long-acting injectable composition for the treatment and/or prevention of parasitic infections and infestations in or on animals consisting of:
a) an antiparasitic effective amount of at least one isoxazoline active agent as defined herein, and optionally at least one additional active agent as identified in this application;
b) a neutral oil that is selected from triglycerides of fractionated plant fatty acids with chain lengths of C₈ to C₁₀;
c) at least one co-solvent wherein said co-solvent is not miscible with water;
d) optionally, an antioxidant; and
e) optionally, at least one pharmaceutically acceptable additive, excipient or mixtures thereof.

In this disclosure and in the claims, terms such as "comprises," "comprising," "containing" and "having" and the like can have the meaning "includes," "including," and the like; "consisting essentially of' or "consists essentially" is open-ended, allowing for the presence of more than that which is recited so long as basic or novel characteristics of that which is recited is not changed by the presence of more than that which is recited, but excludes prior art embodiments. The term "consisting of' excludes any element, step or ingredient not specified in the claims.

### Definitions

Terms used herein will have their customary meaning in the art unless specified otherwise. The organic moieties mentioned in the definitions of the variables of formula (Ic) are - like the term halogen - collective terms for individual listings of the individual group members. The prefix Cₙ-Cₘ indicates in each case the possible number of carbon atoms in the group.

The term "animal" is used herein to include all mammals, birds and fish and also include all vertebrate animals. Animals include, but are not limited to, cats, dogs, cattle, chickens, cows, deer, goats, horses, llamas, pigs, sheep and yaks. It also includes an individual animal in all stages of development, including embryonic and fetal stages. In some embodiments, the animal will be a non-human animal.

The term "essentially pure" is used herein to indicate that a compound or an enantiomer is at least about 90% pure, at least about 95%, at least about 98% pure, or higher.

The term "alkyl" refers to saturated straight, branched, primary, secondary or tertiary hydrocarbons, including those having 1 to 20 atoms. In some embodiments, alkyl groups will include C₁-C₁₂, C₁-C₁₀, C₁-C₈, C₁-C₆ or C₁-C₄ alkyl groups. Examples of C₁-C₁₀ alkyl include, but are not limited to, methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, heptyl, octyl, 2-ethylhexyl, nonyl and decyl and their isomers. C₁-C₄-alkyl means for example methyl, ethyl, propyl, 1-methylethyl, butyl, 1-methylpropyl, 2-methylpropyl or 1,1-dimethylethyl.

Cyclic alkyl groups or "cycloalkyl" include those with 3 to 10 carbon atoms having single or multiple condensed rings. In some embodiments, cycloalkyl groups include C₄-C₇ or C₃-C₄ cyclic alkyl groups. Non-limiting examples of cycloalkyl groups include adamantyl, cyclopropyl, cyclobutyl, cyclopentyl, cyclohexyl, cycloheptyl, cyclooctyl and the like.

The alkyl groups described herein can be unsubstituted or substituted with one or more moieties selected from the group consisting of alkyl, halo, haloalkyl, hydroxyl, carboxyl, acyl, acyloxy, amino, alkyl- or dialkylamino, amido, arylamino, alkoxy, aryloxy, nitro, cyano, azido, thiol, imino, sulfonic acid, sulfate, sulfonyl, sulfanyl, sulfinyl, sulfamoyl, ester, phosphonyl, phosphinyl, phosphoryl, phosphine, thioester, thioether, acid halide, anhydride, oxime, hydrazine, carbamate, phosphoric acid, phosphate, phosphonate, or any other viable functional group that does not inhibit the biological activity of the compounds of the invention, either unprotected, or protected as necessary, as known to those skilled in the art, for example, as taught in Greene, et al., Protective Groups in Organic Synthesis, John Wiley and Sons, Third Edition, 1999.

Terms including the term "alkyl" such as "alkylcycloalkyl," "cycloalkylalkyl," "alkylamino," or "dialkylamino" will be understood to comprise an alkyl group as defined above linked to the other functional group, where the group is linked to the compound through the last group listed, as understood by those of skill in the art.

The term "alkenyl" refers to both straight and branched carbon chains which have at least one carbon-carbon double bond. In some embodiments, alkenyl groups may include C₂-C₂₀ alkenyl groups. In other embodiments, alkenyl includes C₂-C₁₂, C₂-C₁₀, C₂-C₈, C₂-C₆ or C₂-C₄ alkenyl groups. In one embodiment of alkenyl, the number of double bonds is 1-3, in another embodiment of alkenyl, the number of double bonds is one or two. Other ranges of carbon-carbon double bonds and carbon numbers are also contemplated depending on the location of the alkenyl moiety on the molecule. "C₂-C₁₀-alkenyl" groups may include more than one double bond in the chain. Examples include, but are not limited to, ethenyl, 1-propenyl, 2-propenyl, 1-methyl-ethenyl, 1-butenyl, 2-butenyl, 3-butenyl, 1-methyl-1-propenyl, 2-methyl-1-propenyl, 1-methyl-2-propenyl, 2-methyl-2-propenyl; 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

"Alkynyl" refers to both straight and branched carbon chains which have at least one carbon-carbon triple bond. In one embodiment of alkynyl, the number of triple bonds is 1-3; in another embodiment of alkynyl, the number of triple bonds is one or two. In some embodiments, alkynyl groups include from C₂-C₂₀ alkynyl groups. In other embodiments, alkynyl groups may include C₂-C₁₂, C₂-C₁₀, C₂-C₈, C₂-C₆ or C₂-C₄ alkynyl groups. Other ranges of carbon-carbon triple bonds and carbon numbers are also contemplated depending on the location of the alkenyl moiety on the molecule. For example, the term "C₂-C₁₀-alkynyl" as used herein refers to a straight-chain or branched unsaturated hydrocarbon group having 2 to 10 carbon atoms and containing at least one triple bond, such as ethynyl, prop-1-yn-1-yl, prop-2-yn-1-yl, n-but-1-yn-1-yl, n-but-1-yn-3-yl, n-but-1-yn-4-yl, n-but-2-yn-1-yl, n-pent-1-yn-1-yl, n-pent-1-yn-3-yl, n-pent-1-yn-4-yl, n-pent-1-yn-5-yl, n-pent-2-yn-1-yl, n-pent-2-yn-4-yl, n-pent-2-yn-5-yl, 3-methylbut-1-yn-3-yl, 3-methylbut-1-yn-4-yl, n-hex-1-yn-1-yl, n-hex-1-yn-3-yl, n-hex-1-yn-4-yl, n-hex-1-yn-5-yl, n-hex-1-yn-6-yl, n-hex-2-yn-1-yl, n-hex-2-yn-4-yl, n-hex-2-yn-5-yl, n-hex-2-yn-6-yl, n-hex-3-yn-1-yl, n-hex-3-yn-2-yl, 3-methylpent-1-yn-1-yl, 3-methylpent-1-yn-3-yl, 3-methylpent-1-yn-4-yl, 3-methylpent-1-yn-5-yl, 4-methylpent-1-yn-1-yl, 4-methylpent-2-yn-4-yl or 4-methylpent-2-yn-5-yl and the like.

The term "haloalkyl" refers to an alkyl group, as defined herein, which is substituted by one or more halogen atoms. For example C₁-C₄-haloalkyl includes, but is not limited to, chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl and the like.

The term "haloalkenyl" refers to an alkenyl group, as defined herein, which is substituted by one or more halogen atoms.

The term "haloalkynyl" refers to an alkynyl group, as defined herein, which is substituted by one or more halogen atoms.

"Alkoxy" refers to alkyl-O-, wherein alkyl is as defined above. Similarly, the terms "alkenyloxy," "alkynyloxy," "haloalkoxy," "haloalkenyloxy," "haloalkynyloxy," "cycloalkoxy," "cycloalkenyloxy," "halocycloalkoxy," and "halocycloalkenyloxy" refer to the groups alkenyl-O-, alkynyl-O-, haloalkyl-O-, haloalkenyl-O-, haloalkynyl-O-, cycloalkyl-O-, cycloalkenyl-O-, halocycloalkyl-O-, and halocycloalkenyl-O-, respectively, wherein alkenyl, alkynyl, haloalkyl, haloalkenyl, haloalkynyl, cycloalkyl, cycloalkenyl, halocycloalkyl, and halocycloalkenyl are as defined above. Examples of C₁-C₆-alkoxy include, but are not limited to, methoxy, ethoxy, C₂H₅-CH₂O-, (CH₃)₂CHO-, n-butoxy, C₂H₅-CH(CH₃)O-, (CH₃)₂CH-CH₂O-, (CH₃)₃CO-, n-pentoxy, 1-methylbutoxy, 2-methylbutoxy, 3-methylbutoxy, 1,1-dimethylpropoxy, 1,2-dimethylpropoxy, 2,2-dimethyl-propoxy, 1-ethylpropoxy, n-hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy, 1-ethyl-2-methylpropoxy and the like.

The term "alkylthio" refers to alkyl-S-, wherein alkyl is as defined above. Similarly, the terms "haloalkylthio," "cycloalkylthio," and the like, refer to haloalkyl-S- and cycloalkyl-S-where haloalkyl and cycloalkyl are as defined above.

The term "alkylsulfinyl" refers to alkyl-S(O)-, wherein alkyl is as defined above. Similarly, the term "haloalkylsulfinyl" refers to haloalkyl-S(O)- where haloalkyl is as defined above.

The term "alkylsulfonyl" refers to alkyl-S(O)₂-, wherein alkyl is as defined above. Similarly, the term "haloalkylsulfonyl" refers to haloalkyl-S(O)₂- where haloalkyl is as defined above.

The term alkylamino and dialkylamino refer to alkyl-NH- and (alkyl)₂N- where alkyl is as defined above. Similarly, the terms "haloalkylamino" refers to haloalkyl-NH- where haloalkyl is as defined above.

The terms "alkylcarbonyl," "alkoxycarbonyl," "alkylaminocarbonyl," and "dialkylaminocarbonyl" refer to alkyl-C(O)-, alkoxy-C(O)-, alkylamino-C(O)- and dialkylamino-C(O)- where alkyl, alkoxy, alkylamino and dialkylamino are as defined above. Similarly, the terms "haloalkylcarbonyl," "haloalkoxycarbonyl," "haloalkylaminocarbonyl," and "dihaloalkylaminocarbonyl" refer to the groups haloalkyl-C(O)-, haloalkoxy-C(O)-, haloalkylamino-C(O)- and dihaloalkylamino-C(O)- where haloalkyl, haloalkoxy, haloalkylamino and dihaloalkylamino are as defined above.

"Aryl" refers to a monovalent aromatic carbocyclic group of from 6 to 14 carbon atoms having a single ring or multiple condensed rings. In some embodiments, aryl groups include C₆-C₁₀ aryl groups. Aryl groups include, but are not limited to, phenyl, biphenyl, naphthyl, tetrahydronaphtyl, phenylcyclopropyl and indanyl. Aryl groups may be unsubstituted or substituted by one or more moieties selected from halogen, cyano, nitro, hydroxy, mercapto, amino, alkyl, alkenyl, alkynyl, cycloalkyl, cycloalkenyl, haloalkyl, haloalkenyl, haloalkynyl, halocycloalkyl, halocycloalkenyl, alkoxy, alkenyloxy, alkynyloxy, haloalkoxy, haloalkenyloxy, haloalkynyloxy, cycloalkoxy, cycloalkenyloxy, halocycloalkoxy, halocycloalkenyloxy, alkylthio, haloalkylthio, cycloalkylthio, halocycloalkylthio, alkylsulfinyl, alkenylsulfinyl, alkynyl-sulfinyl, haloalkylsulfinyl, haloalkenylsulfinyl, haloalkynylsulfinyl, alkylsulfonyl, alkenylsulfonyl, alkynylsulfonyl, haloalkyl-sulfonyl, haloalkenylsulfonyl, haloalkynylsulfonyl, alkylamino, alkenylamino, alkynylamino, di(alkyl)amino, di(alkenyl)-amino, di(alkynyl)amino, or trialkylsilyl.

The terms "aralkyl" or "arylalkyl" refers to an aryl group that is bonded to the parent compound through a diradical alkylene bridge, (-CH₂-)ₙ, where n is 1-12 and where "aryl" is as defined above.

"Heteroaryl" refers to a monovalent aromatic group of from 1 to 15 carbon atoms, preferably from 1 to 10 carbon atoms, having one or more oxygen, nitrogen, and sulfur heteroatoms within the ring, preferably 1 to 4 heteroatoms, or 1 to 3 heteroatoms. The nitrogen and sulfur heteroatoms may optionally be oxidized. Such heteroaryl groups can have a single ring (e.g., pyridyl or furyl) or multiple condensed rings provided that the point of attachment is through a heteroaryl ring atom. Preferred heteroaryls include pyridyl, pyridazinyl, pyrimidinyl, pyrazinyl, triazinyl, pyrrolyl, indolyl, quinolinyl, isoquinolinyl, quinazolinyl, quinoxalinnyl, furanyl, thiophenyl, furyl, pyrrolyl, imidazolyl, oxazolyl, isoxazolyl, isothiazolyl, pyrazolyl benzofuranyl, and benzothiophenyl. Heteroaryl rings may be unsubstituted or substituted by one or more moieties as described for aryl above.

"Heterocyclyl," "heterocyclic" or "heterocyclo" refer to fully saturated or unsaturated, cyclic groups, for example, 3 to 7 membered monocyclic or 4 to 7 membered monocyclic; 7 to 11 membered bicyclic, or 10 to 15 membered tricyclic ring systems, which have one or more oxygen, sulfur or nitrogen heteroatoms in ring, preferably 1 to 4 or 1 to 3 heteroatoms. The nitrogen and sulfur heteroatoms may optionally be oxidized and the nitrogen heteroatoms may optionally be quaternized. The heterocyclic group may be attached at any heteroatom or carbon atom of the ring or ring system and may be unsubstituted or substituted by one or more moieties as described for aryl groups above.

Exemplary monocyclic heterocyclic groups include, but are not limited to, pyrrolidinyl, pyrrolyl, pyrazolyl, oxetanyl, pyrazolinyl, imidazolyl, imidazolinyl, imidazolidinyl, oxazolyl, oxazolidinyl, isoxazolinyl, isoxazolyl, thiazolyl, thiadiazolyl, thiazolidinyl, isothiazolyl, isothiazolidinyl, furyl, tetrahydrofuryl, thienyl, oxadiazolyl, piperidinyl, piperazinyl, 2-oxopiperazinyl, 2-oxopiperidinyl, 2-oxopyrrolodinyl, 2-oxoazepinyl, azepinyl, 4-piperidonyl, pyridinyl, pyrazinyl, pyrimidinyl, pyridazinyl, tetrahydropyranyl, morpholinyl, thiamorpholinyl, thiamorpholinyl sulfoxide, thiamorpholinyl sulfone, 1,3-dioxolane and tetrahydro-1,1-dioxothienyl, triazolyl, triazinyl, and the like.

Exemplary bicyclic heterocyclic groups include, but are not limited to, indolyl, benzothiazolyl, benzoxazolyl, benzodioxolyl, benzothienyl, quinuclidinyl, quinolinyl, tetra-hydroisoquinolinyl, isoquinolinyl, benzimidazolyl, benzopyranyl, indolizinyl, benzofuryl, chromonyl, coumarinyl, benzopyranyl, cinnolinyl, quinoxalinyl, indazolyl, pyrrolopyridyl, furopyridinyl (such as furo[2,3-c]pyridinyl, furo[3,2-b]pyridinyl]or furo[2,3-b]pyridinyl), dihydroisoindolyl, dihydroquinazolinyl (such as 3,4-dihydro-4-oxo-quinazolinyl), tetrahydroquinolinyl and the like.

Exemplary tricyclic heterocyclic groups include carbazolyl, benzidolyl, phenanthrolinyl, acridinyl, phenanthridinyl, xanthenyl, and the like.

Halogen means the atoms fluorine, chlorine, bromine and iodine. The designation of "halo" (e.g. as illustrated in the term haloalkyl) refers to all degrees of substitutions from a single substitution to a perhalo substitution (e.g. as illustrated with methyl as chloromethyl (-CH₂Cl), dichloromethyl (-CHCl₂), trichloromethyl (-CCl₃)).

By the term "enriched" is meant when the weight:weight ratio is at least approximately 1.05 or higher in favor of the enantiomer that displays significant in vitro and in vivo activity (the eutomer).

### Stereoisomers and polymorphic forms

As noted above, it will be appreciated by those of skill in the art that certain compounds within the compositions of the invention may exist and be isolated as optically active and racemic forms. Compounds having one or more chiral centers, including at a sulfur atom, may be present as single enantiomers or diastereomers or as mixtures of enantiomers and/or diastereomers. For example, it is well known in the art that sulfoxide compounds may be optically active and may exist as single enantiomers or racemic mixtures. In addition, compounds within the compositions of the invention may include one or more chiral centers, which results in a theoretical number of optically active isomers. Where compounds within the compositions of the invention include n chiral centers, the compounds may comprise up to 2ⁿ optical isomers. The present invention encompasses compositions comprising the specific enantiomers or diastereomers of each compound as well as mixtures of different enantiomers and/or diastereomers of the compounds of the invention that possess the useful properties described herein. In addition, the invention encompasses compositions comprising one or more conformational isomers (e.g. rotamers) as well as mixtures of conformational isomers. Conformational isomers of the isoxazoline compounds may be produced by a restriction of rotation about the amide bond bonded to the aryl or heteroaryl ring (e.g. the amide bonded to the naphthyl group in formula (Ic)). The optically active forms can be prepared by, for example, resolution of the racemic forms by selective crystallization techniques, by synthesis from optically active precursors, by chiral synthesis, by chromatographic separation using a chiral stationary phase or by enzymatic resolution.

In addition, the compounds within the compositions of the invention may exist as hydrates or solvates, in which a certain stoichiometric amount of water or a solvent is associated with the molecule in the crystalline form. The compositions of the invention may include hydrates and solvates of the active agents. In some embodiments, the compositions of the invention may include up to 15% (w/w), up to 20% (w/w), or up to 30% (w/w) of a particular solid form.

### Salts

Also contemplated within the scope of the invention are acid or base salts, where applicable, of the compounds of the invention provided for herein.

The term "acid salt" contemplates salts of the compounds with all pharmaceutically acceptable inorganic or organic acids. Inorganic acids include mineral acids such as hydrohalic acids such as hydrobromic acid and hydrochloric acid, sulfuric acid, phosphoric acids and nitric acid. Organic acids include all pharmaceutically acceptable aliphatic, alicyclic and aromatic carboxylic acids, dicarboxylic acids, tricarboxylic acids and fatty acids. In one embodiment of the acids, the acids are straight chain or branched, saturated or unsaturated C₁-C₂₀ aliphatic carboxylic acids, which are optionally substituted by halogen or by hydroxyl groups, or C₆-C₁₂ aromatic carboxylic acids. Examples of such acids are carbonic acid, formic acid, acetic acid, propionic acid, isopropionic acid, valeric acid, α-hydroxy acids such as glycolic acid and lactic acid, chloroacetic acid, benzoic acid, methane sulfonic acid, and salicylic acid. Examples of dicarboxylic acids include oxalic acid, malic acid, succinic acid, tartaric acid, fumaric acid, and maleic acid. An example of a tricarboxylic acid is citric acid. Fatty acids include all pharmaceutically acceptable saturated or unsaturated aliphatic or aromatic carboxylic acids having 4 to 24 carbon atoms. Examples include butyric acid, isobutyric acid, sec-butyric acid, lauric acid, palmitic acid, stearic acid, oleic acid, linoleic acid, linolenic acid, and phenylsteric acid. Other acids include gluconic acid, glycoheptonic acid and lactobionic acid.

The term "base salt" contemplates salts of the compounds with all pharmaceutically acceptable inorganic or organic bases, including hydroxides, carbonates or bicarbonates of alkali metal or alkaline earth metals. Salts formed with such bases include, for example, the alkali metal and alkaline earth metal salts, including, but not limited to, as the lithium, sodium, potassium, magnesium or calcium salts. Salts formed with organic bases include the common hydrocarbon and heterocyclic amine salts, which include, for example, ammonium salts (NH4⁺), alkyl- and dialkylammonium salts, and salts of cyclic amines such as the morpholine and piperidine salts.

In another embodiment, the long-acting injectable compositions of present invention comprise an effective amount of at least one isoxazoline as defined herein, or a pharmaceutically acceptable salt thereof in combination at least one other active agent. In one embodiment, the long-acting injectable compositions comprise an effective amount of at least one isoxazoline compound of formula (I), or a pharmaceutically acceptable salt thereof, in combination with at least one other active agent that is systemically-active.

Additional veterinary/pharmaceutical active ingredients may be used with the compositions of the invention. In some embodiments, the additional active agents may include, but are not limited to, acaricides, anthelmintics, anti-parasitics and insecticides. Anti-parasitic agents can include both ectoparasiticidal and/or endoparasiticidal agents.

Veterinary pharmaceutical agents that may be included in the compositions of the invention are well-known in the art (see e.g. Plumb' Veterinary Drug Handbook, 5th Edition, ed. Donald C. Plumb, Blackwell Publishing, (2005) or The Merck Veterinary Manual, 9th Edition, (January 2005)) and include but are not limited to acarbose, acepromazine maleate, acetaminophen, acetazolamide, acetazolamide sodium, acetic acid, acetohydroxamic acid, acetylcysteine, acitretin, acyclovir, albendazole, albuterol sulfate, alfentanil, allopurinol, alprazolam, altrenogest, amantadine, amikacin sulfate, aminocaproic acid, aminopentamide hydrogen sulfate, aminophylline/theophylline, amiodarone, amitriptyline, amlodipine besylate, ammonium chloride, ammonium molybdate, amoxicillin, clavulanate potassium, amphotericin B desoxycholate, amphotericin B lipid-based, ampicillin, amprolium, antacids (oral), antivenin, apomorphione, apramycin sulfate, ascorbic acid, asparaginase, aspiring, atenolol, atipamezole, atracurium besylate, atropine sulfate, aurnofin, aurothioglucose, azaperone, azathioprine, azithromycin, baclofen, barbituates, benazepril, betamethasone, bethanechol chloride, bisacodyl, bismuth subsalicylate, bleomycin sulfate, boldenone undecylenate, bromides, bromocriptine mesylate, budenoside, buprenorphine, buspirone, busulfan, butorphanol tartrate, cabergoline, calcitonin salmon, calcitrol, calcium salts, captopril, carbenicillin indanyl sodium, carbimazole, carboplatin, carnitine, carprofen, carvedilol, cefadroxil, cefazolin sodium, cefixime, clorsulon, cefoperazone sodium, cefotaxime sodium, cefotetan disodium, cefoxitin sodium, cefpodoxime proxetil, ceftazidime, ceftiofur sodium, ceftiofur, ceftiaxone sodium, cephalexin, cephalosporins, cephapirin, charcoal (activated), chlorambucil, chloramphenicol, chlordiazepoxide, chlordiazepoxide +/- clidinium bromide, chlorothiazide, chlorpheniramine maleate, chlorpromazine, chlorpropamide, chlortetracycline, chorionic gonadotropin (HCG), chromium, cimetidine, ciprofloxacin, cisapride, cisplatin, citrate salts, clarithromycin, clemastine fumarate, clenbuterol, clindamycin, clofazimine, clomipramine, claonazepam, clonidine, cloprostenol sodium, clorazepate dipotassium, clorsulon, cloxacillin, codeine phosphate, colchicine, corticotropin (ACTH), cosyntropin, cyclophosphamide, cyclosporine, cyproheptadine, cytarabine, dacarbazine, dactinomycin/actinomycin D, dalteparin sodium, danazol, dantrolene sodium, dapsone, decoquinate, deferoxamine mesylate, deracoxib, deslorelin acetate, desmopressin acetate, desoxycorticosterone pivalate, detomidine, dexamethasone, dexpanthenol, dexraazoxane, dextran, diazepam, diazoxide (oral), dichlorphenamide, diclofenac sodium, dicloxacillin, diethylcarbamazine citrate, diethylstilbestrol (DES), difloxacin, digoxin, dihydrotachysterol (DHT), diltiazem, dimenhydrinate, dimercaprol/BAL, dimethyl sulfoxide, dinoprost tromethamine, diphenylhydramine, disopyramide phosphate, dobutamine, docusate/DSS, dolasetron mesylate, domperidone, dopamine, doramectin, doxapram, doxepin, doxorubicin, doxycycline, edetate calcium disodium.calcium EDTA, edrophonium chloride, enalapril/enalaprilat, enoxaparin sodium, enrofloxacin, ephedrine sulfate, epinephrine, epoetin/erythropoietin, eprinomectin, epsiprantel, erythromycin, esmolol, estradiol cypionate, ethacrynic acid/ethacrynate sodium, ethanol (alcohol), etidronate sodium, etodolac, etomidate, euthanasia agents w/pentobarbital, famotidine, fatty acids (essential/omega), felbamate, fentanyl, ferrous sulfate, filgrastim, finasteride, fipronil, florfenicol, fluconazole, flucytosine, fludrocortisone acetate, flumazenil, flumethasone, flunixin meglumine, fluorouracil (5-FU), fluoxetine, fluticasone propionate, fluvoxamine maleate, fomepizole (4-MP), furazolidone, furosemide, gabapentin, gemcitabine, gentamicin sulfate, glimepiride, glipizide, glucagon, glucocorticoid agents, glucosamine/chondroitin sulfate, glutamine, glyburide, glycerin (oral), glycopyrrolate, gonadorelin, grisseofulvin, guaifenesin, halothane, hemoglobin glutamer-200 (OXYGLOBIN^{®}), heparin, hetastarch, hyaluronate sodium, hydrazaline, hydrochlorothiazide, hydrocodone bitartrate, hydrocortisone, hydromorphone, hydroxyurea, hydroxyzine, ifosfamide, imidacloprid, imidocarb dipropinate, impenem-cilastatin sodium, imipramine, inamrinone lactate, insulin, interferon alfa-2a (human recombinant), iodide (sodium/potassium), ipecac (syrup), ipodate sodium, iron dextran, isoflurane, isoproterenol, isotretinoin, isoxsuprine, itraconazole, ivermectin, kaolin/pectin, ketamine, ketoconazole, ketoprofen, ketorolac tromethamine, lactulose, leuprolide, levamisole, levetiracetam, levothyroxine sodium, lidocaine, lincomycin, liothyronine sodium, lisinopril, lomustine (CCNU), lufenuron, lysine, magnesium, mannitol, marbofloxacin, mechlorethamine, meclizine, meclofenamic acid, medetomidine, medium chain triglycerides, medroxyprogesterone acetate, megestrol acetate, melarsomine, melatonin, meloxican, melphalan, meperidine, mercaptopurine, meropenem, metformin, methadone, methazolamide, methenamine mandelate/hippurate, methimazole, methionine, methocarbamol, methohexital sodium, methotrexate, methoxyflurane, methylene blue, methylphenidate, methylprednisolone, metoclopramide, metoprolol, metronidaxole, mexiletine, mibolerlone, midazolam milbemycin oxime, mineral oil, minocycline, misoprostol, mitotane, mitoxantrone, morphine sulfate, moxidectin, naloxone, mandrolone decanoate, naproxen, narcotic (opiate) agonist analgesics, neomycin sulfate, neostigmine, niacinamide, nitazoxanide, nitenpyram, nitrofurantoin, nitroglycerin, nitroprusside sodium, nizatidine, novobiocin sodium, nystatin, octreotide acetate, olsalazine sodium, omeprozole, ondansetron, opiate antidiarrheals, orbifloxacin, oxacillin sodium, oxazepam, oxibutynin chloride, oxymorphone, oxytretracycline, oxytocin, pamidronate disodium, pancreplipase, pancuronium bromide, paromomycin sulfate, parozetine, pencillamine, general information penicillins, penicillin G, penicillin V potassium, pentazocine, pentobarbital sodium, pentosan polysulfate sodium, pentoxifylline, pergolide mesylate, phenobarbital, phenoxybenzamine, pheylbutazone, phenylephrine, phenypropanolamine, phenytoin sodium, pheromones, parenteral phosphate, phytonadione/vitamin K-1, pimobendan, piperazine, pirlimycin, piroxicam, polysulfated glycosaminoglycan, ponazuril, potassium chloride, pralidoxime chloride, prazosin, prednisolone/prednisone, primidone, procainamide, procarbazine, prochlorperazine, propantheline bromide, propionibacterium acnes injection, propofol, propranolol, protamine sulfate, pseudoephedrine, psyllium hydrophilic mucilloid, pyridostigmine bromide, pyrilamine maleate, pyrimethamine, quinacrine, quinidine, ranitidine, rifampin, s-adenosyl-methionine (SAMe), saline/hyperosmotic laxative, selamectin, selegiline /l-deprenyl, sertraline, sevelamer, sevoflurane, silymarin/milk thistle, sodium bicarbonate, sodium polystyrene sulfonate, sodium stibogluconate, sodium sulfate, sodum thiosulfate, somatotropin, sotalol, spectinomycin, spironolactone, stanozolol, streptokinase, streptozocin, succimer, succinylcholine chloride, sucralfate, sufentanil citrate, sulfachlorpyridazine sodium, sulfadiazine/trimethroprim, sulfamethoxazole/trimethoprim, sulfadimentoxine, sulfadimethoxine/ormetoprim, sulfasalazine, taurine, tepoxaline, terbinafline, terbutaline sulfate, testosterone, tetracycline, thiacetarsamide sodium, thiamine, thioguanine, thiopental sodium, thiotepa, thyrotropin, tiamulin, ticarcilin disodium, tiletamine /zolazepam, tilmocsin, tiopronin, tobramycin sulfate, tocainide, tolazoline, telfenamic acid, topiramate, tramadol, trimcinolone acetonide, trientine, trilostane, trimepraxine tartrate w/prednisolone, tripelennamine, tylosin, urdosiol, valproic acid, vanadium, vancomycin, vasopressin, vecuronium bromide, verapamil, vinblastine sulfate, vincristine sulfate, vitamin E/selenium, warfarin sodium, xylazine, yohimbine, zafirlukast, zidovudine (AZT), zinc acetate/zinc sulfate, zonisamide and mixtures thereof.

In one embodiment of the invention, arylpyrazole compounds such as phenylpyrazoles, known in the art may be combined with the isoxazoline compounds in the long-acting injectable compositions of the invention. Examples of such arylpyrazole compounds include but are not limited to those described in U.S. Patent Nos. 6,001,384; 6,010,710; 6,083,519; 6,096,329; 6,174,540; 6,685,954 and 6,998,131 (each assigned to Merial, Ltd., Duluth, GA).

In another embodiment of the invention, one or more macrocyclic lactones or lactams, which act as an acaricide, anthelmintic agent and/or insecticide, can be added to the compositions of the invention.

The macrocyclic lactones include, but are not limited to, avermectins such as abamectin, dimadectin, doramectin, emamectin, eprinomectin, ivermectin, latidectin, lepimectin, selamectin and ML-1,694,554, and milbemycins such as milbemectin, milbemycin D, milbemycin oxime, moxidectin and nemadectin. Also included are the 5-oxo and 5-oxime derivatives of said avermectins and milbemycins. Examples of combinations of arylpyrazole compounds with macrocyclic lactones include but are not limited to those described in U.S. Patent Nos. 6,426,333; 6,482,425; 6,962,713 and 6,998,131 (each assigned to Merial, Ltd., Duluth, GA).

The macrocyclic lactone compounds are known in the art and can easily be obtained commercially or through synthesis techniques known in the art. Reference is made to the widely available technical and commercial literature. For avermectins, ivermectin and abamectin, reference may be made, for example, to the work "Ivermectin and Abamectin", 1989, by M.H. Fischer and H. Mrozik, William C. Campbell, published by Springer Verlag., or Albers-Schönberg et al. (1981), "Avermectins Structure Determination", J. Am. Chem. Soc., 103, 4216-4221. For doramectin, "Veterinary Parasitology", vol. 49, No. 1, July 1993, 5-15 may be consulted. For milbemycins, reference may be made, *inter alia,* to Davies H.G. et al., 1986, "Avermectins and Milbemycins", Nat. Prod. Rep., 3, 87-121, Mrozik H. et al., 1983, Synthesis of Milbemycins from Avermectins, Tetrahedron Lett., 24, 5333-5336, U.S. Patent No. 4,134,973 and EP 0 677 054.

Macrocyclic lactones are either natural products or are semi-synthetic derivatives thereof. The structure of the avermectins and milbemycins are closely related, e.g., by sharing a complex 16-membered macrocyclic lactone ring. The natural product avermectins are disclosed in U.S. Patent No. 4,310,519 and the 22,23-dihydro avermectin compounds are disclosed in U.S. Patent No. 4,199,569. Mention is also made of U.S. Patent Nos. 4,468,390, 5,824,653, EP 0 007 812 A1, U.K. Patent Specification 1 390 336, EP 0 002 916, and New Zealand Patent No. 237 086, *inter alia.* Naturally occurring milbemycins are described in U.S. Patent No. 3,950,360 as well as in the various references cited in "The Merck Index" 12th ed., S. Budavari, Ed., Merck & Co., Inc. Whitehouse Station, New Jersey (1996). Latidectin is described in the "International Nonproprietary Names for Pharmaceutical Substances (INN)", WHO Drug Information, vol. 17, no. 4, pp. 263- 286, (2003). Semisynthetic derivatives of these classes of compounds are well known in the art and are described, for example, in U.S. Patent Nos. 5,077,308, 4,859,657, 4,963,582, 4,855,317, 4,871,719, 4,874,749, 4,427,663, 4,310,519, 4,199,569, 5,055,596, 4,973,711, 4,978,677, 4,920,148 and EP 0 667 054.

In another embodiment of the invention, the invention comprises a long-acting injectable composition comprising an isoxazoline compound in combination with systemically-acting compounds from a class of acaricides or insecticides known as insect growth regulators (IGRs). Compounds belonging to this group are well known to the practitioner and represent a wide range of different chemical classes. These compounds all act by interfering with the development or growth of the insect pests. Insect growth regulators are described, for example, in U.S. Patent Nos. 3,748,356, 3,818,047, 4,225,598, 4,798,837, 4,751,225, EP 0 179 022 or UK. 2 140 010 as well as U.S. Patent Nos. 6,096,329 and 6,685,954.

In one embodiment the IGR is a compound that mimics juvenile hormone. Examples of juvenile hormone mimics include azadirachtin, diofenolan, fenoxycarb, hydroprene, kinoprene, methoprene, pyriproxyfen, tetrahydroazadirachtin and 4-chloro-2(2-chloro-2-methyl-propyl)-5-(6-iodo-3-pyridylmethoxy)pyridazine-3(2H)-one.

In an embodiment, the long-acting injectable compositions of present invention comprise an effective amount of at least one isoxazoline of formula (Ic), or a pharmaceutically acceptable salt thereof, in combination with methoprene or pyriproxyfen.

In another embodiment, the IGR compound is a chitin synthesis inhibitor. Chitin synthesis inhibitors include chlorofluazuron, cyromazine, diflubenzuron, fluazuron, flucycloxuron, flufenoxuron, hexaflumoron, lufenuron, tebufenozide, teflubenzuron, triflumuron, novaluron, 1-(2,6-difluorobenzoyl)-3-(2-fluoro-4-(trifluoromethyl)phenylurea, 1-(2,6-difluorobenzoyl)-3-(2-fluoro-4-(1,1,2,2-tetrafluoroethoxy)-phenylurea and 1-(2,6-difluorobenzoyl)-3-(2-fluoro-4-trifluoromethyl)phenylurea.

In yet another embodiment of the invention, adulticide insecticides and acaricides can also be added to the long-acting compositions of the present invention. These include pyrethrins (which include cinerin I, cinerin II, jasmolin I, jasmolin II, pyrethrin I, pyrethrin II and mixtures thereof) and pyrethroids, and carbamates including, but are not limited to, benomyl, carbanolate, carbaryl, carbofuran, methiocarb, metolcarb, promacyl, propoxur, aldicarb, butocarboxim, oxamyl, thiocarboxime and thiofanox. In one embodiment, the compositions can include permethrin in combination with an isoxazoline active agent.

In some embodiments, the long-acting injectable compositions of the present invention may include one or more antinematodal agents including, but not limited to, active agents in the benzimidazoles, imidazothiazoles, tetrahydropyrimidines, and organophosphate class of compounds. In some embodiments, benzimidazoles including, but not limited to, thiabendazole, cambendazole, parbendazole, oxibendazole, mebendazole, flubendazole, fenbendazole, oxfendazole, albendazole, cyclobendazole, febantel, thiophanate and its o,o-dimethyl analogue may be included in the compositions.

In other embodiments, the long-acting injectable compositions of the present invention may include an imidazothiazole compounds including, but not limited to, tetramisole, levamisole and butamisole. In still other embodiments, the long-acting compositions of the present invention may include tetrahydropyrimidine active agents including, but not limited to, pyrantel, oxantel, and morantel. Suitable organophosphate active agents include, but are not limited to, coumaphos, trichlorfon, haloxon, naftalofos and dichlorvos, heptenophos, mevinphos, monocrotophos, TEPP, and tetrachlorvinphos.

In other embodiments, the long-acting injectable compositions of the present invention may include the antinematodal compounds phenothiazine and piperazine as the neutral compound or in various salt forms, diethylcarbamazine, phenols such as disophenol, arsenicals such as arsenamide, ethanolamines such as bephenium, thenium closylate, and methyluridine; cyanine dyes including pyrvinium chloride, pyrvinium pamoate and dithiazanine iodide; isothiocyanates including bitoscanate, suramin sodium, phthalofyne, and various natural products including, but not limited to, hygromycin B, α-santonin and kainic acid.

In other embodiments, the long-acting injectable compositions of the present invention of the invention may include anti-trematodal agents. Suitable anti-trematodal agents include, but are not limited to, the miracils such as miracil D and mirasan; praziquantel, clonazepam and its 3-methyl derivative, oltipraz, lucanthone, hycanthone, oxamniquine, amoscanate, niridazole, nitroxynil, various bisphenol compounds known in the art including hexachlorophene, bithionol, bithionol sulfoxide and menichlopholan; various salicylanilide compounds including tribromsalane, oxyclozanide, diloxanide, rafoxanide, brotianide, bromoxanide and closantel; triclabendazole, diamfenetide, clorsulon, hetolin and emetine.

Anticestodal compounds may also be advantageously used in the long-acting compositions of the present invention of the invention including, but not limited to, arecoline in various salt forms, bunamidine, niclosamide, nitroscanate, paromomycin and paromomycin II.

In yet other embodiments, the long-acting injectable compositions of the present invention may include other active agents that are effective against arthropod parasites. Suitable active agents include, but are not limited to, bromocyclen, chlordane, DDT, endosulfan, lindane, methoxychlor, toxaphene, bromophos, bromophos-ethyl, carbophenothion, chlorfenvinphos, chlorpyrifos, crotoxyphos, cythioate, diazinon, dichlorenthion, diemthoate, dioxathion, ethion, famphur, fenitrothion, fenthion, fospirate, iodofenphos, malathion, naled, phosalone, phosmet, phoxim, propetamphos, ronnel, stirofos, allethrin, cyhalothrin, cypermethrin, deltamethrin, fenvalerate, flucythrinate, permethrin, phenothrin, pyrethrins, resmethrin, benzyl benzoate, carbon disulfide, crotamiton, diflubenzuron, diphenylamine, disulfiram, isobornyl thiocyanato acetate, methoprene, monosulfiram, pirenonylbutoxide, rotenone, triphenyltin acetate, triphenyltin hydroxide, deet, dimethyl phthalate, and the compounds 1,5a,6,9,9a,9b-hexahydro-4a(4H)-dibenzofurancarboxaldehyde (MGK-11), 2-(2-ethylhexyl)-3a,4,7,7a-tetrahydro-4,7-methano-1H-isoindole-1,3(2H)dione (MGK-264), dipropyl-2,5-pyridinedicarboxylate (MGK-326) and 2-(octylthio)ethanol (MGK-874).

An antiparasitic agent that can be combined with an isoxazoline compounds in the long-acting compositions of the present invention can be a biologically active peptide or protein including, but not limited to, depsipeptides, which act at the neuromuscular junction by stimulating presynaptic receptors belonging to the secretin receptor family resulting in the paralysis and death of parasites. In one embodiment of the depsipeptide, the depsipeptide is emodepside (see Willson et al., Parasitology, Jan. 2003, 126(Pt 1):79-86). In another embodiment, the depsipeptide is PF1022A or a derivative thereof.

In another embodiment, the long-acting injectable compositions of the present invention may comprise an active agent from the neonicotinoid class of pesticides. The neonicotinoids bind and inhibit insect specific nicotinic acetylcholine receptors. In one embodiment, the neonicotinoid insecticidal agent that can be combined with an isoxazoline compound to form a long-acting injectable composition of the invention is imidacloprid. Imidacloprid is a well-known neonicotinoid active agent and is the key active ingredient in the topical parasiticide products Advantage^{®}, Advantage^{®} II, K9 Advantix^{®}, and K9 Advantix^{®} II sold by Bayer Animal Health and the oral soft-chewable composition Advantus^{™} from Piedmont Animal Health. Agents of this class are described, for example, in U.S. Patent No. 4,742,060 or in EP 0 892 060.

In another embodiment, the long-acting injectable compositions of the present invention may comprise nitenpyram, another active agent of the neonicotinoid class of pesticides. Nitenpyram has the following chemical structure and is the active ingredient in the oral product CAPSTAR^{™} Tablets sold by Novartis Animal Health.

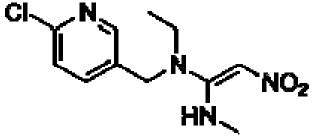

Nitenpyram is active against adult fleas when given daily as an oral tablet. Nitenpyram works by interfering with normal nerve transmission and leads to the death of the insect. Nitenpyram has a very fast onset of action against fleas. For example, CAPSTAR^{™} Tablets begin to act against fleas in as early as 30 minutes after administration and is indicated for use as often as once a day. However, nitenpyram is only known to be effective when administered orally as a systemic parasiticide, as with CAPSTAR^{™} Tablets.

In yet another embodiment, the invention provides the long-acting compositions of the present invention comprising 4-[5-[3-chloro-5-(trifluoromethyl)phenyl]-4,5-dihydro-5-(trifluoromethyl)-3-isoxazolyl]-N-[2-oxo-2-[(2,2,2-trifluoroethyl)amino]ethyl]-1-naphthalanecarboxamide (Compound of formula Ia) in combination with nitenpyram.

In yet another embodiment, the invention provides the long-acting compositions of the present invention comprising 4-[5-[3-chloro-5-(trifluoromethyl)phenyl]-4,5-dihydro-5-(trifluoromethyl)-3-isoxazolyl]-N-[2-oxo-2-[(2,2,2-trifluoroethyl)amino]ethyl]-1-naphthalanecarboxamide (Compound of formula Ia) in combination with imidacloprid.

In certain embodiments, an insecticidal agent that can be combined with the long-acting compositions of the present invention is a semicarbazone, such as metaflumizone.

In another embodiment, the long-acting injectable compositions of the present invention may advantageously include a combination of isoxazoline compounds known in the art. These active agents are described in WO 2007/079162, WO 2007/075459 and US 2009/0133319, WO 2007/070606 and US 2009/0143410, WO 2009/003075, WO 2009/002809, WO 2009/024541, WO 2005/085216 and US 2007/0066617 and WO 2008/122375.

In another embodiment of the invention, nodulisporic acid and its derivatives (a class of known acaricidal, anthelmintic, anti-parasitic and insecticidal agents) may be added to the long-acting compositions of the present invention. These compounds are used to treat or prevent infections in humans and animals and are described, for example, in U.S. Patent No. 5,399,582, 5,962,499, 6,221,894 and 6,399,786. The compositions may include one or more of the known nodulisporic acid derivatives in the art, including all stereoisomers, such as those described in the patents cited above.

In another embodiment, anthelmintic compounds of the amino acetonitrile class (AAD) of compounds such as monepantel (ZOLVIX), and the like, may be added to the. the long-acting compositions of the present invention These compounds are described, for example, in WO 2004/024704 and U.S. Patent No. 7,084,280; Sager et al., Veterinary Parasitology, 2009, 159, 49-54; Kaminsky et al., Nature vol. 452, 13 March 2008, 176-181.

The compositions of the invention may also include aryloazol-2-yl cyanoethylamino compounds such as those described in US Patent No. 8,088,801 to Soll et al., and thioamide derivatives of these compounds, as described in U.S. Patent No. 7,964,621.

The long-acting injectable compositions of the present invention may also be combined with paraherquamide compounds and derivatives of these compounds, including derquantel (see Ostlind et al., Research in Veterinary Science, 1990, 48, 260-61; and Ostlind et al., Medical and Veterinary Entomology, 1997, 11, 407-408). The paraherquamide family of compounds is a known class of compounds that include a spirodioxepino indole core with activity against certain parasites (see Tet. Lett. 1981, 22, 135; J. Antibiotics 1990, 43, 1380, and J. Antibiotics 1991, 44, 492). In addition, the structurally related marcfortine family of compounds, such as marcfortines A-C, are also known and may be combined with the compositions of the invention (see J. Chem. Soc. - Chem. Comm. 1980, 601 and Tet. Lett. 1981, 22, 1977). Further references to the paraherquamide derivatives can be found, for example, in WO 91/09961, WO 92/22555, WO 97/03988, WO 01/076370, WO 09/004432, U.S. Patent 5,703,078 and U.S. Patent 5,750,695.

In general, the additional active agent is included in the long-acting compositions of the present invention in an amount of between about 0.1 µg and about 1000 mg. More typically, the additional active agent may be included in an amount of about 10 µg to about 500 mg, about 1 mg to about 300 mg, about 10 mg to about 200 mg or about 10 mg to about 100 mg.

In other embodiments of the invention, the additional active agent may be included in the composition to deliver a dose of about 5 µg/kg to about 50 mg/kg per weight of the animal. In other embodiments, the additional active agent may be present in an amount sufficient to deliver a dose of about 0.01 mg/kg to about 30 mg/kg, about 0.1 mg/kg to about 20 mg/kg, or about 0.1 mg/kg to about 10 mg/kg of weight of animal. In other embodiments, the additional active agent may be present in a dose of about 5 µg/kg to about 200 µg/kg or about 0.1 mg/kg to about 1 mg/kg of weight of animal. In still another embodiment of the invention, the additional active agent is included in a dose between about 0.5 mg/kg to about 50 mg/kg.

The long-acting compositions of the present invention, as defined in the appended claims, have been surprisingly discovered to be stable and effective against a broad spectrum of ectoparasites, and possibly also endoparasites if another active is included, for an extended period of time; e.g., a period from three (3) to six (6) months while exhibiting favorable properties with respect to the site of injection.

Liquid PEGs as provided for herein are those polyethylene glycols that are liquid at room temperature (20 - 30 °C). Polyethylene glycols have the following structural formula:

H-(O-CH₂-CH₂)ₙ-OH.

Non-limiting examples for n in the above formula are those compounds when n is from 1 to about 10,000 (e.g., from about 4 to about 25). Liquid PEGs include combinations of different polyethylene glycols. Non-limiting examples of liquid PEGs include PEG 200, PEG 300, PEG 400, PEG 600, and PEG 1000 or combinations thereof.

Pharmaceutically acceptable polymers other than PEGs are specifically excluded from the inventive long-acting compositions. Examples of pharmaceutically acceptable polymers that are specifically excluded from the inventive long-acting compositions include polylactides, polyglycolides, polycaprolactones, polyanhydrides, polyamides, polyurethanes, polyester amides, polyorthoesters, polydioxanones, polyacetals, polyketals, polycarbonates, polyorthocarbonates, polyphosphazenes, polyhydroxybutyrates, polyhydroxyvalerates, polyalkylene oxalates, polyalkylene succinates, poly(malic acid), poly(amino acids), poly(methyl vinyl ether), poly(maleic anhydride), chitin, chitosan, and copolymers, terpolymers, or combinations or mixtures therein including copolymers of polylactides, polycaprolactones, polyglycolides (e.g., poly(lactide-co-glycolide).

The co-solvents used in the long-acting injectable compositions may be a single or a blend of co-solvents. Co-solvents may be used in the compositions of the invention to improve the solubility of the isoxazoline active agent and/or to lower the viscosity of the compositions. In one embodiment, the co-solvents used in the long-acting injectable compositions of the present invention include polar solvents that are miscible in water. Non-limiting examples of these co-solvents include ethanol, isopropanol, glycol ethers (e.g., including, but limited to, diethyleneglycol monoethyl ether (DGME, Transcutol^{®}), butyl diglycol, dipropylene glycol n-butyl ether, ethyleneglycol monoethyl ether, ethyleneglycol monomethyl ether, dipropylene glycol monomethyl ether, propylene glycol monomethyl ether, propylene glycol monoethyl ether, and the like), propylene glycol, glycerin, carbonates (e.g., propylene carbonate or ethylene carbonate), 2-pyrrolidone, substituted 2-pyrrolidones including N-methylpyrrolidone, 1-ethylpyrrolidone, l-octylpyrrolidone, 1-dodecylpyrrolidone, 1-isopropylpyrrolidone, l-(*sec-* or *t*or n-butyl)pyrrolidone, 1-hexylpyrrolidone, 1-vinyl-2-pyrrolidone, 1-cyclohexylpyrrolidone, 1-(2-hydroxyethyl)-pyrrolidone, 1-(3-hydroxypropyl)pyrrolidone, 1-(2-methoxyethyl)-pyrrolidone, 1-(3-methoxypropyl)pyrrolidone and 1-benzylpyrrolidone; dimethyl isosorbide (DMI), dimethylacetamide (DMA), dimethylsulfoxide (DMSO), glycerol formal or a mixture of at least two of these solvents.

In one embodiment, the long-acting compositions of the invention comprise a polar protic solvent including, but not limited to, an alcohol such a C₁-C₆alcohol including, but not limited to, ethanol, isopropanol. In another embodiment, the polar protic solvent is a glycol, such as glycerol or propylene glycol, or a glycol ether such as diethylene glycol monoethyl ether (Transcutol) or other commonly used glycol ethers such as those described above.

In another embodiment, the long-acting injectable compositions of the invention comprise a polar aprotic solvent including, but not limited to, N-methylpyrrolidone, dimethyl isosorbide, dimethylacetamide, dimethylsulfoxide or propylene carbonate.

In yet another embodiment of the invention, the compositions of the invention include non-water miscible co-solvents or solvents with only partial solubility in water. Non-water miscible solvents include esters of aliphatic carboxylic acids (including fatty acids) and glycerol (glycerides) or esters of aliphatic carboxylic acids (including fatty acids) and propylene glycol. Non-water miscible solvents also include oils that are acceptable for injectable compositions. Non-limiting examples of these co-solvents include benzyl alcohol, benzyl benzoate, ethyl acetate, triacetin, lipids, triglycerides including medium chain triglycerides such C₈-C₁₀ triglycerides such as capric/caprilic triglycerides, propylene glycol derivatives (e.g. propylene glycol monolaurate), caprylocaproyl polyoxyl-8 glycerides (Labrasol) (non-ionic water dispersible surfactant, isopropyl myristate, or a mixture of at least two of these co-solvents.

The composition of the invention includes a pharmaceutically acceptable neutral oil as a main component of the composition. When a neutral oil is used in combination with a liquid PEG, it may be necessary to use a bridging solvent or a surfactant to ensure miscibility. The neutral oil included in the composition of the invention is selected from triglycerides of fractionated plant fatty acids with chain lengths of C₈ to C₁₀, including caprylic/capric triglycerides. Two commercially available products are known as MIGLYOL^{®} 810 and MIGLYOL^{®}812. In another embodiment, the neutral oil is a triglyceride of fractionated plant fatty acids with chain lengths of C₈ and C₁₀ combined with linoleic acid (about 4-5 %). A commercially available product is known as MIGLYOL^{®} 818. In yet another embodiment, the neutral oil is a glycerin ester of fractionated plant fatty acids with chain lengths of C₈ and C₁₀ combined with succinic acid. A commercially available product is known as MIGLYOL^{®} 829. In another embodiment, the neutral oil may be a propylene glycol diester of saturated plant fatty acids with chain lengths of C₈ and C₁₀. A commercially available product is known as MIGLYOL^{®} 840 (propylene glycol dicaprylate/dicaprate). In yet another embodiment, the co-solvent may be a mixture of two or more neutral oils. Other oils that are acceptable to include in the long-acting injectable compositions of the invention include, but are not limited to, castor oil, cottonseed oil, sesame oil, soybean oil and safflower oil.

In one embodiment, the compositions of the invention may comprise about 0.5% to about 70% (w/v) of a co-solvent. In another embodiment, the compositions of the invention may comprise about 0.5% to about 60% (w/v) or about 1 to about 50% (w/v) of a co-solvent. In still another embodiment, the compositions may comprise about 1% to about 40% (w/v), about 5% to about 50% (w/v), about 5% to about 40% (w/v) or about 5% to about 35% (w/v) of a co-solvent. In other embodiments, the compositions of the invention may comprise about 1% to about 20% (w/v), about 2% to about 15% (w/v) or about 2% to about 10% (w/v) of the co-solvent.

The long-acting injectable compositions of the invention may include pharmaceutically acceptable additives or excipients. Pharmaceutically acceptable additives and excipients include, but are not limited to, surfactants, antioxidants, preservatives, pH stabilizing agents (e.g. buffers), and other non-active excipients. In another embodiment, the compositions of the invention may comprise about 0.01% to about 20% (w/v) of a pharmaceutically acceptable additive, excipient or mixtures thereof. In other embodiments, the compositions may comprise about 0.01% to about 5% (w/v), about 0.1% to about 10% (w/v) or about 0.1% to about 5% (w/v) of a pharmaceutically acceptable additive, excipient or mixtures thereof. In other embodiments the compositions may comprise about 5 to about 15% (w/v) or about 5 to about 10% (w/v) of a pharmaceutically acceptable additive, excipient or mixtures thereof. In yet another embodiment, the compositions may comprise about 7 to about 10% of a pharmaceutically acceptable additive, excipient or mixtures thereof.

Surfactants may be present in the inventive compositions at concentrations of about 0.1 % to about 10% (w/w), about 1 % to about 10% (w/w) or about 5% to about 10% (w/w). More typically, surfactants may be present at concentrations of about 0.1 % to about 5% (w/w) or about 1 to about 5% (w/w). Examples of surfactants that may be used in the compositions include, but are not limited to, glyceryl monooleate, polyoxyethylene sorbitan fatty acid esters, sorbitan esters including sorbitan monooleate (Span^{®} 20), polyvinyl alcohol, polysorbates including polysorbate 20 and polysorbate 80, *d*-α-tocopherol polyethylene glycol 1000 succinate (TPGS), sodium lauryl sulfate, co-polymers of ethylene oxide and propylene oxide (e.g. poloxamers such as LUTROL ^{®} F87 and the like), polyethylene glycol castor oil derivatives including polyoxyl 35 castor oil (Cremophor^{®} EL), polyoxyl 40 hydrogenated castor oil (Cremophor^{®} RH 40), polyoxyl 60 hydrogenated castor oil (Cremophor^{®} RH60); propylene glycol monolaurate (LAUROGLYCOL ^{®}); glyceride esters including glycerol caprylate/caprate (CAPMUL^{®} MCM) , polyglycolized glycerides (GELUCIRE^{®}), PEG 300 caprylic/capric glycerides (Softigen^{®} 767), PEG 400 caprylic/capric glycerides (Labrasol^{®}), PEG 300 oleic glycerides (Labrafil^{®} M-1944CS), PEG 300 linoleic glycerides (Labrafil^{®} M-2125CS); polyethylene glycol stearates and polyethylene glycol hydroxy stearates including polyoxyl 8 stearate (PEG 400 monostearate) , polyoxyl 40 stearate (PEG 1750 monostearate, and the like). Polyethylene glycol stearates (synonyms include macrogol stearates, polyoxylstearates, polyoxyethylene stearates, ethoxylated stearates; CAS No. 9004-99-3, 9005-08-7) are mixtures of mono- and distearate esters of mixed polyoxyethylene polymers. Polyethylene glycol hydroxystearate is a mixture of mono- and diesters of hydroxystearic acid with polyethylene glycols. One polyethylene glycol hydroxystearate that may be used in the compositions is polyethylene glycol 12-hydroxystearate. In another embodiment, the inventive compositions may include the surfactant polyethylene glycol 15 12-hydroxystearate (Kolliphor^{®} HS 15 from BASF), a mixture of mono- and diesters of 12-hydroxystearic acid with 15 moles of ethylene oxide. Again, these compounds, as well as their amounts are well known in the art. In another embodiment of the invention, the inventive compositions may include polyoxyl 35 castor oil (Kolliphor^{®} EL) as a surfactant. In other embodiments, the inventive compositions may include polyoxyl 40 hydrogenated castor oil (Kolliphor^{®} RH 40) or polyoxyl 60 hydrogenated castor oil as surfactants. The compositions of the invention may also include a combination of surfactants.

The inventive compositions may contain other inert ingredients such as antioxidants, preservatives, or pH stabilizers. These compounds are well known in the composition art. Antioxidants such as vitamin E, alpha tocopherol, ascorbic acid, ascorbyl palmitate, citric acid, fumaric acid, malic acid, sodium ascorbate, sodium metabisulfate, sodium metabisulfite, n-propyl gallate, BHA (butylated hydroxy anisole), BHT (butylated hydroxy toluene), BHA and citric acid, monothioglycerol, tert-butyl hydroquinone (TBHQ), and the like, may be added to the present composition. The antioxidants are generally included in the compositions of the invention in amounts of about 0.01% to about 3%, or from about 0.01 to about 2% (w/v), based upon total weight of the composition (w/w). In another embodiment, the compositions contain about 0.05 to about 1.0% (w/w) of one or a mixture of antioxidants.

Preservatives, such as the parabens (methylparaben and/or propylparaben), are suitably used in the composition in amounts ranging from about 0.01 to about 2.0%, with about 0.05 to about 1.0% being especially preferred. Other preservatives include benzalkonium chloride, benzethonium chloride, benzoic acid, benzyl alcohol, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, sodium benzoate, sodium propionate, sorbic acid, thimerosal, and the like. Preferred ranges for these compounds include from about 0.01 to about 5%.

Compounds which stabilize the pH of the composition are also contemplated. Again, such compounds are well known to a practitioner in the art as well as how to use these compounds. Buffering systems include, for example, systems selected from the group consisting of acetic acid/acetate, malic acid/malate, citric acid/citrate, tartaric acid/tartrate, lactic acid/lactate, phosphoric acid/phosphate, glycine/glycimate, tris, glutamic acid/glutamates and sodium carbonate.

Dosage forms may contain from about 0.5 mg to about 5 g of an active agent or a combination of active agents. More typically, the amount of active agent(s) in the compositions of the invention will be from about 1 mg to about 3 g. In another embodiment, the amount of active agent(s) in the compositions will be from about 20 mg to about 3 g. In another embodiment, the amount of active agent(s) present in the compositions will be from about 20 mg to about 2 g, about 20 mg to about 1.5 g or about 20 mg to about 1 g. In other embodiments, the amount of active agent(s) in the compositions will be from about 20 mg to about 500 mg, about 30 mg to about 200 mg or about 50 mg to about 200 mg. In still another embodiment, the amount of active agent(s) present in the compositions will be from about 50 mg to about 2 g, about 50 mg to about 1 g or about 50 mg to about 500 mg. In yet another embodiment of the invention, the amout of active agent(s) present will be from about 100 mg to about 2 g, about 100 mg to about 1 g or about 100 mg to about 500 mg.

In another embodiment, the amount of active agent(s) present in the compositions of the invention is from about 1 mg to about 500 mg of an active agent, about 1 mg to about 100 mg or about 1 mg to about 25 mg. In still other embodiments, the amount of the active agent present in the compositions is about 10 mg about 50 mg or about 10 mg to about 100 mg. In other embodiments, the amount of active agent present in the compositions is about 50 mg to about 200 mg, about 100 mg to about 300 mg, about 100 mg to about 400 mg, about 200 mg to about 500 mg, about 300 mg to about 600 mg, about 400 mg to about 800 mg, or about 500 mg to about 1000 mg.

The compositions of the invention are made by mixing the appropriate amount of the active agent(s), a neutral oil that is selected from triglycerides of fractionated plant fatty acids with chain lengths of C₈ to C₁₀, a co-solvent (if present) and, optionally, an antioxidant, pharmaceutically acceptable additive and/or excipient to form a composition of the invention. In some embodiments the compositions of the present invention can be obtained by following the method of making these forms described above by the description of making these forms found in general composition text known to those in the art, e.g. Remington - The Science and Practice of Pharmacy (21st Edition) (2005), Goodman & Gilman's The Pharmacological Basis of Therapeutics (11th Edition) (2005) and Ansel's Pharmaceutical Dosage Forms and Drug Delivery Systems (8th Edition), edited by Allen et al., Lippincott Williams & Wilkins, (2005).

### Methods of Treatment

References to methods of treatment by therapy or surgery in this description are to be interpreted as references to compounds, pharmaceutical compositions and medicaments of the present invention for use in those methods.

In another aspect of the invention, a method for preventing or treating a parasite infestation/infection in an animal is provided, comprising administering to the animal a long-acting injectable composition comprising an effective amount of at least one isoxazoline compound as defined herein, a neutral oil that is selected from triglycerides of fractionated plant fatty acids with chain lengths of C₈ to C₁₀, optionally a co-solvent, optionally, an antioxidant, and optionally a pharmaceutically acceptable additive and/or excipient, wherein the parasites are treated or prevented for about 3 to 6 months. The compositions of the invention have long-lasting efficacy against ectoparasites (e.g. fleas and ticks) and in certain embodiments may also be active against endoparasites that harm animals.

In one embodiment of the invention, methods for the treatment or prevention of a parasitic infestation or infection in a livestock animal are provided, which comprise administering a long-acting injectable composition as defined herein comprising an effective amount of at least one isoxazoline active agent to the animal. Ectoparasites against which the methods and compositions of the invention are effective include, but are not limited to, fleas, ticks, mites, mosquitoes, flies and lice. In certain embodiments wherein the inventive compositions include one or more additional active agents that are active against internal parasites the compositions and methods of the invention may also be effective against endoparasites including, but not limited to, cestodes, nematodes, hookworms and roundworms of the digestive tract of animals and humans.

In an alternative embodiment of the invention, methods for the treatment or prevention of a parasitic infestation or infection in a domestic animal are provided, which comprise administering a long-acting injectable composition as defined herein comprising an effective amount of at least one isoxazoline active agent to the animal. Ectoparasites against which the methods and compositions of the invention are effective include, but are not limited to, fleas, ticks, mites, mosquitoes, flies and lice. In certain embodiments wherein the inventive compositions include one or more additional active agents that are active against internal parasites the compositions and methods of the invention may also be effective against endoparasites including, but not limited to, cestodes, nematodes, hookworms and roundworms of the digestive tract of animals and humans.

In one embodiment for treatment against ectoparasites, the ectoparasite is one or more insect or arachnid including those of the genera *Ctenocephalides, Rhipicephalus, Dermacentor, Ixodes, Boophilus, Amblyomma, Haemaphysalis, Hyalomma, Sarcoptes, Psoroptes, Otodectes, Chorioptes, Hypoderma, Damalinia, Linognathus, Haematopinus, Solenoptes, Trichodectes,* and *Felicola.*

In another embodiment for the treatment against ectoparasites, the ectoparasite is from the genera *Ctenocephalides, Rhipicephalus, Dermacentor, Ixodes* and/or *Boophilus.* The ectoparasites treated include but are not limited to fleas, ticks, mites, mosquitoes, flies, lice, blowfly and combinations thereof. Specific examples include, but are not limited to, cat and dog fleas (*Ctenocephalides felis, Ctenocephalides* sp. and the like), ticks (*Rhipicephalus* sp., *Ixodes* sp., *Dermacentor* sp., *Amblyomma* sp. and the like), and mites (*Demodex* sp., *Sarcoptes* sp., *Otodectes* sp. and the like), lice (*Trichodectes* sp., *Cheyletiella* sp., *Linognathus* sp., and the like), mosquitoes (*Aedes* sp., *Culex* sp., *Anopheles* sp., and the like) and flies (*Haematobia* sp. *including Haematobia irritans, Musca* sp., *Stomoxys* sp. *including Stomoxys calcitrans, Dermatobia* sp., *Cochliomyia* sp., and the like).

Additional examples of ectoparasites include but are not limited to the tick genus *Boophilus,* especially those of the species *microplus* (cattle tick), *decoloratus* and *annulatus;* myiasis such as *Dermatobia hominis* (known as Berne in Brazil) and *Cochliomyia hominivorax* (greenbottle); sheep myiasis such as *Lucilia sericata, Lucilia cuprina* (known as blowfly strike in Australia, New Zealand and South Africa). Flies proper, namely those whose adult constitutes the parasite, such as *Haematobia irritans* (horn fly) and *Stomoxys calcitrans* (stable fly); lice such as *Linognathus vituli,* etc.; and mites such as *Sarcoptes scabiei* and *Psoroptes ovis.* The above list is not exhaustive and other ectoparasites are well known in the art to be harmful to animals and humans. These include, for example migrating dipterous larvae.

In some embodiments of the invention, the composition can also be used to treat against endoparasites such as those helminths selected from the group consisting of *Anaplocephala, Ancylostoma, Necator, Ascaris, Capillaria, Cooperia, Dipylidium, Dirofilaria, Echinococcus, Enterobius, Fasciola, Haemonchus, Oesophagostomum, Ostertagia, Toxocara, Strongyloides, Toxascaris, Trichinella, Trichuris,* and *Trichostrongylus,* among others.

In one embodiment, the invention provides methods for the treatment and prevention of parasitic infections and infestations in or on animals (either wild or domesticated), including livestock and companion animals such as cats, dogs, horses, birds including chickens, sheep, goats, pigs, deer, turkeys and cattle, with the aim of ridding these hosts of parasites commonly encountered by such animals.

In an embodiment, the invention provides methods and compositions for the treatment or prevention of parasitic infections and infestations in livestock animals (e.g., sheep and cattle) or companion animals including, but not limited to, cats and dogs. The methods and compositions are particularly effective for preventing or treating parasitic infestations of cattle and sheep with fleas and ticks.

In another embodiment, the methods and compositions of the invention are used for the treatment or prevention of parasitic infections and infestations in cattle or sheep. When treating livestock animals such as cattle or sheep, the methods and compositions are particularly effective against *Rhipicephalus (Boophilus) microplus, Haematobia irritans* (horn fly), *Stomoxys calcitrans* (stable fly), and sheep myiasis such as *Lucilia sericata, Lucilia cuprina* (known as blowfly strike in Australia, New Zealand and South Africa).

The terms "treating" or "treat" or "treatment" are intended to mean the administration of a long-acting composition of the present invention to an animal that has a parasitic infestation for the eradication of the parasite or the reduction of the number of the parasites infesting the animal undergoing treatment. It is noted that the compositions of the invention may be used to prevent such a parasitic infestation.

The terms "prevent", "prevention" and "prophylaxis" are intended to mean the administration of the long-acting compositions of the present invention to the animal before the parasitic infection or infestation has occurred in order to keep said infection or infestation from occurring. Administration of the long-acting compositions at recommended regular intervals effectively prevents new parasitic infestations or infections in animals by killing new parasites that attack an animal before they can multiply to establish an infestation or infection.

The compositions of the invention are administered in parasiticidally effective amounts which are which are suitable to control the parasite in question to the desired extent, as described below. In each aspect of the invention, the compounds and compositions of the invention can be applied against a single pest or combinations thereof.

By "antiparasitic effective amount" is intended a sufficient amount of a composition of the invention to eradicate or reduce the number of parasites infesting the animal. In some embodiments, an effective amount of the active agent achieves at least 70% efficacy against the target parasite. In other embodiments, an effective amount of the active agent achieves at least 80%, or at least 90% efficacy against the target pests. Preferably, an effective amount of the active agent will achieve at least 95%, at least 98% or 100% efficacy against the target parasites.

Generally, a dose of from about 0.001 to about 100 mg per kg of body weight given as a single dose or in divided doses for a period of from 1 to 5 days will be satisfactory but, of course, there can be instances where higher or lower dosage ranges are indicated, and such are within the scope of this invention. It is well within the routine skill of the practitioner to determine a particular dosing regimen for a specific host and parasite.

In some embodiments for companion animals, the dose of the isoxazoline active agent administered from the topical compositions of the invention is between about 0.1 to about 50 mg per kg of body weight. More typically the dose of the isoxazoline active agent administered is about 0.5 to about 30 mg/kg or about 0.5 to about 30 mg/kg body weight. In yet another embodiment, the dose of the isoxazoline active agent will be from about 0.5 to about 20 mg/kg, about 0.5 to about 10 mg/kg or about 0.5 to about 5 mg/kg body weight. In another embodiment, the dose will be from about 0.5 to about 2.5 mg/kg body weight. In another embodiment, the dose of the isoxazoline active agent administered is about 10 to about 30 mg/kg, about 15 to about 30 mg/kg or about 20 to about 30 mg/kg of body weight.

In other embodiments, the dose administered may be lower depending on the animal and the isoxazoline administered. For example, if the composition comprises the more active enantiomer of the isoxazoline compounds a lower dose may be administered. In some embodiments, the dose is from about 0.1 to about 30 mg/kg of body weight. In another embodiment, the dose may be from about 0.1 to about 20 mg/kg or about 0.1 to about 10 mg/kg of body weight. In another embodiment, a dose of from about 0.1 to about 5 mg/kg, from about 0.1 to about 2.5 mg/kg body weight will be used. In other embodiments, the dose may be from about 1 to about 20 mg/kg of body weight or about 1 to about 10 mg/kg. In yet another embodiment, the dose may be from about 5 to about 20 mg/kg or about 10 to about 20 mg/kg of body weight.

The volume of the dose of the long-acting injectable compositions is typically less than about 10 mL/50 kg body weight of the animal being treated. In other embodiments, the injection volume is about 7 mL/50 kg body weight. In yet other embodiments, the injection volume is less than about 5 mL/50 kg, less than about 3 mL/50 kg body weight or less than about 2 mL/50 kg of body weight. In yet other embodiments, the injection volume is from about 0.1 to about 2 mL/50 kg body weight of the animal. In other embodiments, the injection volume is about 0.05 to about 1 mL/50 kg body weight of the animal.

In other embodiments for the treatment of livestock animals such as cattle or sheep, doses of the isoxazoline active agent administered may be about 0.1 to about 40 mg/kg of body weight. More typically the doses administered will be about 1 to about 30 mg/kg, about 1 to about 20 mg/kg or about 1 to about 10 mg/kg of bodyweight. In yet another embodiment, the dose may be from about 10 to about 25 mg/kg, about 15 to about 30 mg/kg of body weight or about 20-30 mg/kg of body weight.

In one embodiment of the method of use in livestock animals (e.g., cattle or sheep), the long-acting compositions of the present invention as defined herein comprising an isoxazoline compound has an efficacy against ectoparasites including, but not limited to, fleas, ticks, mites, and parasitic flies, of at least about 90.0% or higher for about 3 months, or longer. In another embodiment, the long-acting compositions of the present invention provide an efficacy against ectoparasites of at least 95.0% or higher for about 3 months or longer.

In another embodiment, the long-acting compositions of the present invention provide an efficacy against ectoparasites in livestock animals (e.g., cattle or sheep) of at least about 80% for two months, or longer. In another embodiment, the long-acting compositions of the present invention efficacy against ectoparasites in livestock animals (e.g., cattle or sheep) of about 90% for at least about 2 months. In still another embodiment, the compositions provide an efficacy of about 95% for about 2 months or longer. In another embodiment, the long-acting compositions of the present invention efficacy against ectoparasites in livestock animals (e.g., cattle or sheep) of about 90% for about 3 months, or longer. In still another embodiment, the compositions provide an efficacy of about 95% for about 3 months or longer.

In another embodiment, the long-acting compositions of the present invention have an efficacy of at least about 80% against ectoparasites for about 3 months, or longer. In still another embodiment, the long-acting compositions of the invention provide an efficacy of at least about 90% against ectoparasites for 3 months or longer. In yet another embodiment, the long-acting compositions of the present invention of the invention provide an efficacy of at least about 95% against ectoparasites for 3 months or longer. In still another embodiment, the long-acting compositions of the present invention provide an efficacy against ectoparasites in livestock animals (e.g., cattle or sheep) of at least 80% or at least 90% for about 3 months to about 6 months or longer.

In other embodiments, the long-acting compositions of the invention have an efficacy of at least about 90% against ectoparasites including, but not limited to, fleas, ticks, mites and parasitic flies, of about 7 months or longer, about 8 months or longer or about 9 months or longer. In other embodiments, the long-acting compositions of the invention have an efficacy of at least about 90% against ectoparasites including, but not limited to, fleas, ticks, mites and parasitic flies, of about 10 months or longer, about 118 months or longer or even about 12 months or longer.

In another aspect of the invention, a kit for the treatment or prevention of a parasitic infestation in an animal is provided, which comprises a long-acting composition of the invention and a syringe.
In a preferred embodiment, the isoxazoline is a compound of formula (Ie) shown below, or a pharmaceutically or pharmaceutically acceptable salt thereof:

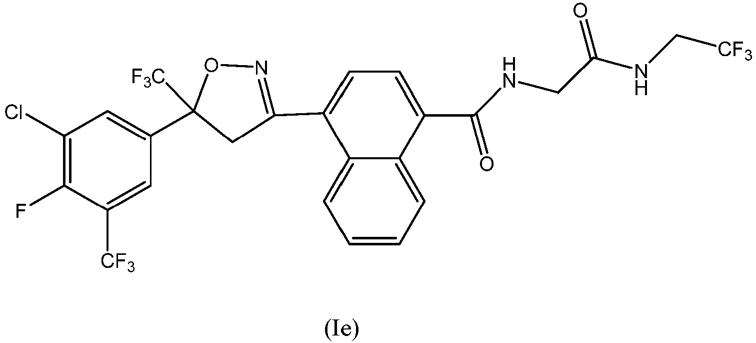

In an embodiment, the compound of formula (Ie), or compositions comprising the compound, are enriched in one enantiomer over the other enantiomer in a weight:weight ratio of at least 1.5:1. In another embodiment, the compounds of formula (Ie) and compositions comprising the compounds are enriched in one enantiomer in a weight:weight ratio of at least 2:1, at least 5:1, at least 10:1 or at least 20:1.

In one embodiment, the isooxazoline is a compound of formula (S)-Ie, or a pharmaceutically or pharmaceutically acceptable salt thereof, shown below:

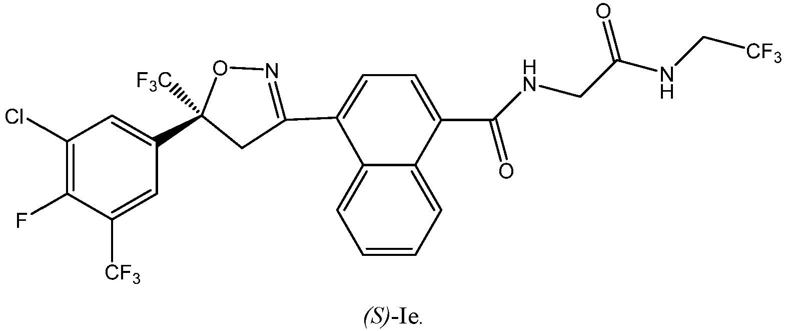

In another embodiment, the isoxazoline is a compound of formula (*R*)-Ie, or a pharmaceutically or pharmaceutically acceptable salt thereof, shown below:

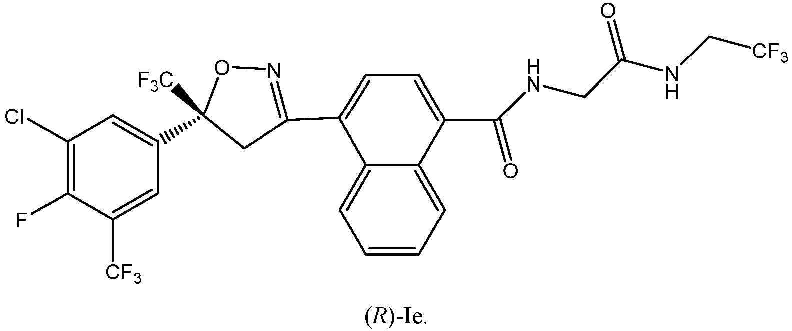

In another embodiment, the invention provides pesticidal and parasiticidal compositions comprising a compound of formula *(S)*-Ie. In yet another embodiment, the invention provides pesticidal and parasiticidal compositions comprising a compound of formula (*R*)-Ie. For the avoidance of doubt, the compositions of the invention comprising a compound of formula (*S*)-Ie or (R)-Ie may be enriched in the desired enantiomer at the ratios discussed above.
In another embodiment, the compounds of formula (Ie), in compositions comprising the compounds, are essentially pure enantiomers. In one embodiment, the composition of the invention comprise a compound of formula (Ie) that is substantially enriched in an enantiomer. The term "substantially enriched" is meant wherein the weight: weight ratio is at least about 1.5:1 or higher in favor of the desired enantiomer. In another embodiment, the compositions of the invention are substantially enriched in the (S)-enantiomer. In another embodiment, the compositions of the invention are substantially enriched in the (R)-enantiomer.

In another embodiment of the invention, the compounds of formula (Ie), in compositions comprising the compounds, are enriched in the *(S)*-enantiomer in a weight: weight ratio of at least about 2:1, *(S)* to (*R*)*,* or greater. In yet another embodiment, the compositions of the invention comprise a compound of formula (Ie) that is enriched in the *(S)*-enantiomer in a weight:weight ratio of at least about 5:1, *(S)* to (*R*)*,* or greater. In still another embodiment, the compositions of the invention comprise a compound of formula (Ie), that is enriched in the *(S)*-enantiomer in a weight: weight ratio of at least about 10:1, *(S)* to (*R*)*,* or greater. In another embodiment, the compositions of the invention comprise a compound of formula (Ie), that is enriched in the *(S)-*enantiomer in a weight: weight ratio of at least about 20:1, *(S)* to (*R*)*,* or greater. In still another embodiment, the compositions of the invention comprise a compound of formula (Ie), that is essentially the pure *(S)*-enantiomer.

### ANIMAL HEALTH APPLICATIONS

### I. Veterinary compositions:

The compounds of formula (Id) and compositions comprising the compounds are useful for the prevention and treatment of parasitic infestations/infections in animals. In a preferred embodiment, the compositions of the invention comprise an effective amount of an isoxazoline of formula (Ie) or (*S*)-Ie, or a pharmaceutically acceptable salt thereof. The compositions are intended to be administered to an animal including, but not limited to, mammals, birds and fish. Examples of mammals include but are not limited to humans, cattle, sheep, goats, llamas, alpacas, pigs, horses, donkeys, dogs, cats and other livestock or domestic mammals. Examples of birds include turkeys, chickens, ostriches and other livestock or domestic birds. The use of the compounds of formula (Id) to protect companion animals, such as dogs and cats, and livestock animals, such as cattle and sheep, from ectoparasites is particularly useful. Dosage forms may typically contain from about 0.1 mg to about 5 g. In other embodiments, the dosage form may contain about 0.5 mg to about 5 g of an active agent. In one embodiment of the dosage form, the dosage may contain from about 1 mg to about 500 mg of an active agent, typically about 25 mg, about 50 mg, about 100 mg, about 200 mg, about 300 mg, about 400 mg, about 500 mg, about 600 mg, about 800 mg, or about 1000 mg.

In one embodiment of the invention, the active agent may be present in the composition at a concentration of about 0.05 to about 10% weight/volume. In another embodiment of the invention, the active agent may be present in the composition as a concentration from about 0.1 to about 2% weight/volume. In yet another embodiment of the invention, the active agent may be present in the composition as a concentration from about 0.25 to about 1.5% weight/volume. In still another embodiment of the invention, the active agent may be present in the composition as a concentration about 1% weight/volume.

### II. Methods of Treatment:

As discussed above, the compounds of formula (Id) are effective against ectoparasites and may be used to treat and prevent parasitic infestations in or on animals. In one embodiment, the present invention provides a method of treating or preventing an ectoparasite infestation in or on an animal (*e.g.* a mammal or bird) comprising administering an ectoparasiticidally effective amount of a compound of formula (Id), or pharmaceutically acceptable salts thereof, or a composition comprising the compound, to the animal. In another embodiment, the methods of the invention comprise administering an effective amount of a compound of formula (Ie) or (*S*)-Ie, or a pharmaceutically acceptable salt thereof, to the animal.

In another embodiment when the compounds of formula (Id) are administered in combination with compounds that are active against endoparasites, the invention provides a method for treating or preventing an endoparasitic infection and an ectoparasitic infestation in and on an animal, comprising administering a composition comprising an effective amount of a compound of formula (Id) in combination with an effective amount of at least a second active agent, or pharmaceutically acceptable salts thereof, to the animal.

Mammals which can be treated include but are not limited to humans, cats, dogs, cattle, chickens, cows, bison, deer, goats, horses, llamas, camels, pigs, sheep and yaks. In one embodiment of the invention, the mammals treated are humans, cats or dogs.

In one embodiment of the invention, the compositions of the invention comprising a compound of formula (Id) in combination with an additional compound that is active against endoparasites are effective against endoparasites that are resistant to active agents of the macrocyclic lactone class. In one embodiment, the compounds and compositions of the invention are effective for controlling *Haemonchus contortus, Ostertagia circumcincta* and *Trichostrongylus colubriformis* in mammals or birds.

In another embodiment, the invention provides a method for treating an parasitic infestation and/or infection in an animal, comprising administering an effective amount of a compound of formula (Id) in combination with an effective amount of activators of invertebrate GABA receptors, including an avermectin or milbemycin, to the animal in need thereof. Avermectins that may be used in combination with the compounds of the invention include, but are not limited to abamectin, dimadectin, doramectin, emamectin, eprinomectin, ivermectin, latidectin, lepimectin, and selamectin. Milbemycins compounds that may be used in combination with the compounds of the invention include, but are not limited to, milbemectin, milbemycin D, milbemycin oxime, moxidectin and nemadectin. Also included are the 5-oxo and 5-oxime derivatives of said avermectins and milbemycins.

In one embodiment for the treatment against ectoparasites, the ectoparasite is from the genera *Ctenocephalides, Rhipicephalus, Dermacentor, Ixodes, Amblyomma, Haemaphysalis, Hyalomma, Sarcoptes, Psoroptes, Otodectes, Chorioptes, Hypoderma, Damalinia, Linognathus, Haematopinus, Solenoptes, Trichodectes,* and *Felicola.* The ectoparasites treated include but are not limited to fleas, ticks, mites, mosquitoes, flies, lice, blowfly and combinations thereof. Specific examples include but are not limited to cat and dog fleas (*Ctenocephalides felis, Ctenocephalides spp.* and the like), ticks (*Rhipicephalus spp., Ixodes* spp., *Dermacentor* spp., *Amblyomma spp.* and the like), and mites (*Demodex spp., Sarcoptes spp., Otodectes spp.* and the like), lice (*Trichodectes spp., Cheyletiella spp., Linognathus spp.,* and the like), mosquitoes (*Aedes spp., Culex spp., Anopheles spp.,* and the like) and flies (*Haematobia* spp., *Musca* spp., *Stomoxys* spp., *Dermatobia* spp., *Cochliomyia* spp., and the like). In yet another embodiment for the treatment against ectoparasites, the ectoparasite is a flea and/or tick.

Additional examples of ectoparasites that may be controlled with the compounds of formula (Ie) include, but are not limited, to the tick *Rhipicephalus microplus* (cattle tick), *Rhipicephalus decoloratus* and *Rhipicephalus annulatus;* myiasis such as *Dermatobia hominis* (known as Berne in Brazil) and *Cochliomyia hominivorax* (greenbottle); sheep myiasis such as *Lucilia sericata, Lucilia cuprina* (known as blowfly strike in Australia, New Zealand and South Africa). Flies proper, namely those whose adult constitutes the parasite, such as *Haematobia irritans* (horn fly); lice such as *Linognathus vitulorum,* etc.; and mites such as *Sarcoptes scabiei* and *Psoroptes ovis.* The above list is not exhaustive and other ectoparasites are well known in the art to be harmful to animals and humans. These include, for example migrating dipterous larvae.

In one embodiment, when administered with another compound that is active against endoparasites, the compounds and compositions of the invention may be used for treating or preventing an endoparasitic infection of the following parasite: *Anaplocephala (Anoplocephala), Ancylostoma, Necator, Ascaris, Brugia, Bunostomum, Capillaria, Chabertia, Cooperia, Cyathostomum, Cylicocyclus, Cylicodontophorus, Cylicostephanus, Craterostomum, Dictyocaulus, Dipetalonema, Dipylidium, Dirofilaria, Dracunculus, Echinococcus, Enterobius, Fasciola, Filaroides, Habronema, Haemonchus, Metastrongylus, Moniezia, Necator, Nematodirus, Nippostrongylus, Oesophagostomum, Onchocerca, Ostertagia, Oxyuris, Parascaris, Schistosoma, Strongylus, Taenia, Toxocara, Strongyloides, Toxascaris, Trichinella, Trichuris, Trichostrongylus, Triodontophorus, Uncinaria, Wuchereria,* and combinations thereof.

In another embodiment of the invention, the parasite is *Haemonchus contortus, Ostertagia circumcincta, Trichostrongylus axei, Trichostrongylus colubriformis, Cooperia curticei, Nematodirus battus, Dirofilaria immitis, and combinations thereof.*

In another embodiment of the invention, the compounds and compositions of the invention are suitable for controlling pests such as insects selected from the group consisting of *Blatella germanica, Heliothis virescens, Leptinotarsa decemlineata, Tetramorium caespitum* and combinations thereof.

The phytoparasitic nematodes include, for example, *Anguina* spp., *Aphelenchoides* spp., *Belonolaimus* spp., *Bursaphelenchus* spp., *Ditylenchus dipsaci, Globodera* spp., *Helicotylenchus* spp., *Heterodera* spp., *Longidorus* spp., *Meloidogyne* spp., *Pratylenchus* spp., *Radopholus similis, Rotylenchus* spp., *Trichodorus* spp., *Tylenchorhynchus* spp., *Tylenchulus* spp., *Tylenchulus semipenetrans, Xiphinema* spp.

In addition, with or without the other pesticidal agents added to the composition, the invention can also be used to treat other pests which include but are not limited to pests:
(1) from the order of *Isopoda,* for example *Oniscus asellus, Armadillidium vulgare* and *Porcellio scaber;*
(2) from the order of *Diplopoda,* for example *Blaniulus guttulatus;*
(3) from the order of *Chilopoda,* for example *Geophilus carpophagus* and *Scutigera* spp.;
(4) from the order of *Symphyla,* for example *Scutigerella immaculata;*
(5) from the order of *Thysanura,* for example *Lepisma saccharina;*
(6) from the order of *Collembola,* for example *Onychiurus armatus;*
(7) from the order of *Blattaria,* for example *Blatta orientalis, Periplaneta americana, Leucophaea maderae* and *Blattella germanica;*
(8) from the order of *Hymenoptera,* for example *Diprion* spp., *Hoplocampa* spp., *Lasius* spp., *Monomorium pharaonis* and *Vespa* spp.;
(9) from the order of *Siphonaptera,* for example *Xenopsylla cheopis* and *Ceratophyllus* spp.;
(10) from the order of *Anoplura* (*Phthiraptera*)*,* for example, *Damalinia* spp., *Haematopinus* spp., *Linognathus* spp., *Pediculus* spp., *Trichodectes* spp.;
(11) from the class of Arachnida, for example, *Acarus* siro, *Aceria sheldoni, Aculops* spp., *Aculus* spp., *Amblyomma* spp., *Argas* spp., *Boophilus* spp., *Brevipalpus* spp., *Bryobia praetiosa, Chorioptes* spp., *Dermanyssus gallinae, Eotetranychus* spp., *Epitrimerus pyri, Eutetranychus* spp., *Eriophyes* spp., *Hemitarsonemus* spp., *Hyalomma* spp., *Ixodes* spp., *Latrodectus mactans, Metatetranychus* spp., *Oligonychus* spp., *Ornithodoros* spp., *Panonychus* spp., *Phyllocoptruta oleivora, Polyphagotarsonemus latus, Psoroptes* spp., *Rhipicephalus* spp., *Rhizoglyphus* spp., *Sarcoptes* spp., *Scorpio maurus, Steneotarsonemus* spp., *Tarsonemus* spp., *Tetranychus* spp., *Vasates lycopersici.;*
(12) from the class of Bivalva, for example, *Dreissena* spp.;
(13) from the order of Coleoptera, for example, *Acanthoscelides obtectus, Adoretus* spp., *Agelastica alni, Agriotes* spp., *Amphimallon solstitialis, Anobium punctatum, Anoplophora* spp., *Anthonomus* spp., *Anthrenus* spp., *Apogonia* spp., *Atomaria* spp., *Attagenus* spp., *Bruchidius obtectus, Bruchus* spp., *Ceuthorhynchus* spp., *Cleonus mendicus, Conoderus* spp., *Cosmopolites* spp., *Costelytra zealandica, Curculio* spp., *Cryptorhynchus lapathi, Dermestes* spp., *Diabrotica* spp., *Epilachna* spp., *Faustinus cubae, Gibbium psylloides, Heteronychus arator, Hylamorpha elegans, Hylotrupes bajulus, Hypera postica, Hypothenemus* spp., *Lachnosterna consanguinea, Leptinotarsa decemlineata, Lissorhoptrus oryzophilus, Lixus* spp., *Lyctus* spp., *Meligethes aeneus, Melolontha melolontha, Migdolus* spp., *Monochamus* spp., *Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga* spp., *Popillia japonica, Premnotrypes* spp., *Psyl*/*iodes chrysocephala, Ptinus* spp., *Rhizobius ventralis, Rhizopertha dominica, Sitophilus* spp., *Sphenophorus* spp., *Sternechus* spp., *Symphyletes* spp., *Tenebrio molitor, Tribolium* spp., *Trogoderma* spp., *Tychius* spp., *Xylotrechus* spp., *Zabrus* spp.;
(14) from the order of Diptera, for example, *Aedes* spp., *Anopheles* spp., *Bibio hortulanus, Calliphora erythrocephala, Ceratitis capitata, Chrysomyia* spp., *Cochliomyia* spp., *Cordylobia anthropophaga, Culex* spp., *Cuterebra* spp., *Dacus oleae, Dermatobia hominis, Drosophila* spp., *Fannia* spp., *Gastrophilus* spp., *Hylemyia* spp., *Hyppobosca* spp., *Hypoderma* spp., *Liriomyza* spp., *Lucilia* spp., *Musca* spp., *Nezara* spp., *Oestrus* spp., *Oscinella frit, Pegomyia hyoscyami, Phorbia* spp., *Stomoxys* spp., *Tabanus* spp., *Tannia* spp., *Tipula paludosa, Wohlfahrtia* spp.;
(15) from the class of Gastropoda, for example, *Arion* spp., *Biomphalaria* spp., *Bulinus* spp., *Deroceras* spp., *Galba* spp., *Lymnaea* spp., *Oncomelania* spp., *Succinea* spp.;
(16) from the class of helminths, for example, *Ancylostoma duodenale, Ancylostoma ceylanicum, Ancylostoma braziliensis, Ancylostoma* spp., *Ascaris lubricoides, Ascaris* spp., *Brugia malayi, Brugia timori, Bunostomum* spp., *Chabertia* spp., *Clonorchis* spp., *Cooperia* spp., *Dicrocoelium* spp, *Dictyocaulus filaria, Diphyllobothrium latum, Dracunculus medinensis, Echinococcus granulosus, Echinococcus multilocularis, Enterobius vermicularis, Faciola* spp., *Haemonchus* spp., *Heterakis* spp., *Hymenolepis nana, Hyostrongulus* spp., *Loa Loa, Nematodirus* spp., *Oesophagostomum* spp., *Opisthorchis* spp., *Onchocerca volvulus, Ostertagia* spp., *Paragonimus* spp., *Schistosomen* spp., *Strongyloides fuelleborni, Strongyloides stercoralis, Strongyloides* spp., *Taenia saginata, Taenia solium, Trichinella spiralis, Trichinella nativa, Trichinella britovi, Trichinella nelsoni, Trichinella pseudopsiralis, Trichostrongulus* spp., *Trichuris trichuria, Wuchereria bancrofti.;*
(17) from the order of Heteroptera, for example, *Anasa tristis, Antestiopsis* spp., *Blissus* spp., *Calocoris* spp., *Campylomma livida, Cavelerius* spp., *Cimex* spp., *Creontiades dilutus, Dasynus piperis, Dichelops furcatus, Diconocoris hewetti, Dysdercus* spp., *Euschistus* spp., *Eurygaster* spp., *Heliopeltis* spp., *Horcias nobilellus, Leptocorisa* spp., *Leptoglossus phyllopus, Lygus* spp., *Macropes excavatus, Miridae, Nezara* spp., *Oebalus* spp., *Pentomidae, Piesma quadrata, Piezodorus* spp., *Psallus seriatus, Pseudacysta persea, Rhodnius* spp., *Sahlbergella singularis, Scotinophora* spp., *Stephanitis nashi, Tibraca* spp., *Triatoma* spp.;
(18) from the order of Homoptera, for example, *Acyrthosipon* spp., *Aeneolamia* spp., *Agonoscena* spp., *Aleurodes* spp., *Aleurolobus barodensis, Aleurothrixus* spp., *Amrasca* spp., *Anuraphis cardui, Aonidiella* spp., *Aphanostigma piri, Aphis* spp., *Arboridia apicalis, Aspidiella* spp., *Aspidiotus* spp., *Atanus* spp., *Aulacorthum solani, Bemisia* spp., *Brachycaudus helichrysii, Brachycolus* spp., *Brevicoryne brassicae, Calligypona marginata, Carneocephala fulgida, Ceratovacuna lanigera, Cercopidae, Ceroplastes* spp., *Chaetosiphon fragaefolii, Chionaspis tegalensis, Chlorita onukii, Chromaphis juglandicola, Chrysomphalus ficus, Cicadulina mbila, Coccomytilus halli, Coccus* spp., *Cryptomyzus ribis, Dalbulus* spp., *Dialeurodes* spp., *Diaphorina* spp., *Diaspis* spp., *Doralis* spp., *Drosicha* spp., *Dysaphis* spp., *Dysmicoccus* spp., *Empoasca* spp., *Eriosoma* spp., *Erythroneura* spp., *Euscelis bilobatus, Geococcus coffeae, Homalodisca coagulata, Hyalopterus arundinis, Icerya* spp., *Idiocerus* spp., *Idioscopus* spp., *Laodelphax striatellus, Lecanium* spp., *Lepidosaphes* spp., *Lipaphis erysimi, Macrosiphum* spp., *Mahanarva fimbriolata, Melanaphis sacchari, Metcalfiella* spp., *Metopolophium dirhodum, Monellia costalis, Monelliopsis pecanis, Myzus* spp., *Nasonovia ribisnigri, Nephotettix* spp., *Nilaparvata lugens, Oncometopia* spp., *Orthezia praelonga, Parabemisia myricae, Paratrioza* spp., *Parlatoria* spp., *Pemphigus* spp., *Peregrinus maidis, Phenacoccus* spp., *Phloeomyzus passerinii, Phorodon humuli, Phylloxera* spp., *Pinnaspis aspidistrae, Planococcus* spp., *Protopulvinaria pyriformis, Pseudaulacaspis pentagona, Pseudococcus* spp., *Psylla* spp., *Pteromalus* spp., *Pyrilla* spp., *Quadraspidiotus* spp., *Quesada gigas, Rastrococcus* spp., *Rhopalosiphum* spp., *Saissetia* spp., *Scaphoides titanus, Schizaphis graminum, Selenaspidus articulatus, Sogata* spp., *Sogatella furcifera, Sogatodes* spp., *Stictocephala festina, Tenalaphara malayensis, Tinocallis caryaefoliae, Tomaspis* spp., *Toxoptera* spp., *Trialeurodes vaporariorum, Trioza* spp., *Typhlocyba* spp., *Unaspis* spp., *Viteus vitifolii.;*
(19) from the order of Isoptera, for example, *Reticulitermes* spp., *Odontotermes* spp.;
(20) from the order of Lepidoptera, for example, *Acronicta major, Aedia leucomelas, Agrotis* spp., *Alabama argillacea, Anticarsia* spp., *Barathra brassicae, Bucculatrix thurberiella, Bupalus piniarius, Cacoecia podana, Capua reticulana, Carpocapsa pomonella, Cheimatobia brumata, Chilo* spp., *Choristoneura fumiferana, Clysia ambiguella, Cnaphalocerus* spp., *Earias insulana, Ephestia kuehniella, Euproctis chrysorrhoea, Euxoa* spp., *Feltia* spp., *Galleria mellonella, Helicoverpa* spp., *Heliothis* spp., *Hofmannophila pseudospretella, Homona magnanima, Hyponomeuta padella, Laphygma* spp., *Lithocolletis blancardella, Lithophane antennata, Loxagrotis albicosta, Lymantria* spp., *Malacosoma neustria, Mamestra brassicae, Mocis repanda, Mythimna separata, Oria* spp., *Oulema oryzae, Panolis flammea, Pectinophora gossypiella, Phyllocnistis citrella, Pieris* spp., *Plutella xylostella, Prodenia* spp., *Pseudaletia* spp., *Pseudoplusia includens, Pyrausta nubilalis, Spodoptera* spp., *Thermesia gemmatalis, Tinea pellionella, Tineola bisselliella, Tortrix viridana, Trichoplusia* spp.;
(21) from the order of Orthoptera, for example, *Acheta domesticus, Blatta orientalis, Blattella germanica, Gryllotalpa* spp., *Leucophaea maderae, Locusta* spp., *Melanoplus* spp., *Periplaneta americana, Schistocerca gregaria.;*
(22) from the order of Thysanoptera, for example, *Baliothrips biformis, Enneothrips flavens, Frankliniella* spp., *Heliothrips* spp., *Hercinothrips femoralis, Kakothrips* spp., *Rhipiphorothrips cruentatus, Scirtothrips* spp., *Taeniothrips cardamoni, Thrips* spp.;
(23) from the class of Protozoa, for example, *Eimeria* spp.

In each aspect of the invention, the compounds and compositions of the invention can be applied against a single pest or combinations thereof.

The invention will now be further described by way of the following non-limiting examples.

### EXAMPLES

The invention is further described by the following non-limiting examples which further illustrate the invention, and are not intended, nor should they be interpreted to, limit the scope of the invention.

### Synthesis Examples

### Synthesis Example 1: Synthesis of (S)-Ie

The compound of formula (*S*)-Ie was prepared according to Scheme 2 below. Compound **2-1** is described in US 7,951,828 B1. Preparation of the compound **2-4** is described in US 8,217,180 B2 and US 8,546,618 B2. Cinchona alkaloid-based chiral phase transfer catalyst similar to **2-6** are prepared according to the procedures described in, for example, US 9,126,995 B2, WO 2011/104089 and US 2014/0206633. Further, Matoba et al., Angew. Chem. 2010, 122, 5898-5902 describes the use of these catalysts to prepare enantiomerically pure isoxazoline compounds.

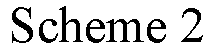

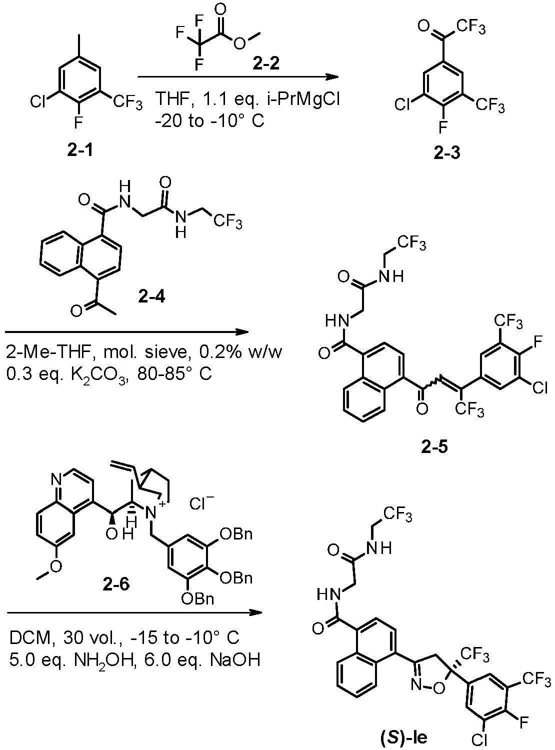

### Step 1

1-Chloro-2-fluoro-5-iodo-3-(trifluoromethyl)benzene (2-1, 100 g, 0.31 mol) and tetrahydrofuran (THF, 200 ml, 2 volumes) were charged into a 500 ml reactor under an atmosphere of nitrogen. The mixure was cooled to -15 to -20° C and a solution of i-PrMgCl in THF (2M, 170 ml, 0.34 mol, 1.1 eq.) was added to the reactor slowly (over 30 min.) at -20 to -5° C. The resulting mixture was stirred for an additional 0.5-1 hour and checked for reaction completion by GeMS, which showed that the starting material was consumed. Methyl 2,2,2-trifluoroacetate was added to the reaction mixture over 0.5-1 hour at -20 to -5° C. The resulting mixture was stirred overnight at -20 to -10° C and checked for reaction conversion. When the reaction was complete, aqueous HCl (1 M, 500 ml) was added and the mixture was stirred for 1-2 hours at -5 to 5° C. The quenched reaction mixture was extracted with cyclohexane twice (500 ml, 200 ml) and the combined organic layers were concentrated under vacuum to provide intermediate 2-3 as a crude product (62.0 g, purity 98.4%, 77.7%).

### Step 2

2-methyltetrahydrofuran (2-Me-THF, 25 ml, 5 vol.), intermediate 2-4 (5.0 g, 17 mmol, 1 eq.), molecular sieves (1.0 g, 20% w/w) and potassium carbonate (0.7 g, 5.1 mmol, 0.3 eq.) were charged into a 100 ml, 3-neck flask. The resulting mixture was warmed to 75-85° C and compound 2-3 was added drop-wise to the mixture at 75-85° C over 0.5-1 hour. The mixture was then stirred for an additional 4 hours at the same temperature and tested by HPLC for reaction progression. Additional K₂CO₃ (0.2 g, 1.7 mmol) and 2-3 (0.1 g, 0.34 mmol, 0.2 eq.) were added and the mixture was stirred for a further 16 hours. The mixture was cooled to 30-40° C and filtered. The filtrate was concentrated to a brown solid. The product was purified by chromatography (silica gel, petroleum ether/ethyl acetate) to yield the product as a yellow solid (6.6 grams, 79.7% purity). The product was then recrystallized from acetonitrile to yield compound 2-5 in 44.9 % yield (4.0 grams) and 91.6% purity.

### Step 3

Dichloromethane (DCM, 150 ml, 30 vol.) and 2-5 (5.0 g, 7.95 mmol, 1.0 eq.) were charged into a 500 ml 3-neck flask. The mixture was stirred for 10-30 min. until the compound was dissolved and then cooled to -10 to 15° C. The chiral phase transfer catalyst 2-6 was added (0.18 g, 0.024 mmol, 0.03 eq.) to the solution and then a solution of NH₂OH (50% w/w)/NaOH/H₂0 (20 ml, 5 vol.) was added dropwise at -10 to 15° C over 0.5-1 hour. The resulting mixture was stirred for 16 hours at -10 to 15° C and sampled for analysis by HPLC to check the reaction completion, at which time there was less than 3.0% of the starting material. The organic layer was separated and washed with a saturated solution of KH₂PO₄ (20 ml, 4 vol.) twice. The resulting organic layer was further washed with brine (20 ml) twice and then concentrated under vacuum at 30-40° C with the concomitant addition of toluene (20 ml, 4 vol.) and then concentrated to dryness.

To 4.9 grams of the isolated crude product was charged 15 ml of toluene in a 3-neck flask and the mixture was heated to 60-70° C to dissolve the solid. The resulting solution was cooled slowly to 45-50° over 1 hour and seed (0.025 g, 0.05% w/w) was added. The seeded mixture was stirred for 1 hour at 45-50° C and then cooled further to 37-42° C over 1 hour and then stirred for a further 6 hours. During this time the product was observed to crystallize from solution. The mixture was cooled to 30-35° C over 1 hour and stirred for 3 hours. The solid was filtered and the cake washed with toluene (10 ml, 2 vol.). The cake was then dried in an oven at 40-45° under vacuum for 6 hours to yield 2.3 grams (45.1% yield) of (S)-Ie in 99.4% purity and 99.3% chiral purity.

Using the same approach but an alternative chiral phase transfer catalyst (e.g. isomer of 2-6), the compound (R)-Ie may be made. Alternatively, a racemic compound of formula (Ie) may be prepared without the use of a chiral phase transfer catalyst. The final step in these processes are described in the examples below.

### Synthesis Example 2: racemic compound (Ie)

### Step 3

Into a 5 liter reactor was charged 2 liters (10 volumes) of DCM and 200.0 grams (0.32 mol, 1.0 eq.) and the mixture was stirred for 10-30 minutes to dissolve the solid. The solution was cooled to 0-5° C and NH₂OH (50% w/w)/NaOH/H₂O (104.9 g/76.3 g/1.0 L) was added dropwise at 0-25° C over 30-60 min. The resulting mixture was stirred at 10-25° C for 3 hours and then sampled to check the reaction conversion by HPLC, which showed that the starting material was present at less than 3.5%. The mixture was allowed to settle and the DCM layer was washed with a saturated solution of KH₂PO₄ (0.8 L, 4 vol.) twice. The resulting organic layer was further washed with brine (0.8 L, 4 vol.) twice. The combined organic layers were concentrated under vacuum at 30-40° C to dryness and to provide the crude product as a yellow solid (196.0 g, purity: 94%, chiral purity: 49.7%). The crude product was purified by chromatography over silica gel using DCM:ethanol (100:1 to 20:1) to get 138.0 grams of the pure product (purity: 99.5%, chiral purity: 49.7%). The product was further dried to remove toluene to yield 125.0 grams as a light yellow solid (yield, 61.2%, purity: 99.5%, chiral purity: 49.7%).

### Synthesis Example 3: Preparation of compound (S)-(If)

The compound of formula (S)-(If) was prepared using a process very similar to that shown above for (S)-(Ie) with the key difference that 1-iodo-3,5-dichlorobenzene was used as starting material instead of 1-Chloro-2-fluoro-5-iodo-3-(trifluoromethyl)benzene. The process for the preparation of (S)-(If) is shown in Scheme 3 below and the detailed procedure for Step 3 is provided.

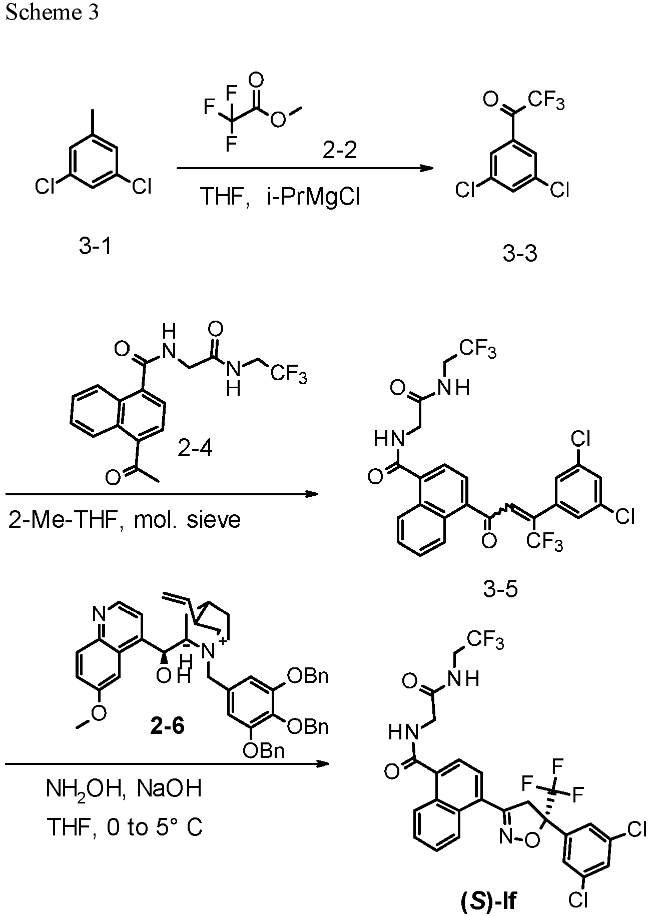

### Step 3

Dichloromethane (150 ml, 30 vol.) and intermediate 3-5 (5.0 g, 8.66 mmol, 1.0 eq.) were charged into a 3-neck 500 ml flask and stirred for 10-30 minutes to dissolve the solid. The solution was then cooled to 0-5° C and chiral phase transfer catalyst 2-6 was added (0.20 g, 0.026 mmol, 0.03 eq.). To the resulting solution was added a solution of NH₂OH (50% w/w)/NaOH/H₂O (20 ml, 5 vol.) drop-wise at 0 to 5° C over 0.5-1 hour. The resulting reaction mixture was stirred for 2 hours at 0-5° C and then sampled to check the reaction completion by HPLC, which showed less than 1% starting material. The layers were allowed to settle and the DCM layer was washed with saturated KH₂PO₄ (20 ml, 4 vol.) twice. The organic layer was then washed with brine (20 ml, 4 vol.) twice. The combined organic layers were concentrated under vacuum at 30-40° C with concomitant addition of toluene (20 ml, 4 vol., twice) and then concentrated to dryness. Toluene (15 ml, 3 vol.) and the crude product (5.0 g) were charged into a 50 ml, 3-neck flask and the mixture was heated to 60-70° C to dissolve the solid. The resulting mixture was cooled slowly to 45-50° C over 2 hours and filtered. The filter cake was washed with toluene (10 ml, 2 vol.) and the filtrate was dried in an oven under vacuum at 40-50° C for 6 hours to yield the product (2.5 g, yield: 49.0%, purity 99.4%, chiral purity: 99.3%).

### Synthesis Example 4: Preparation of compound (Ic) where X¹ and X² = Cl and X³=F

The compound of formula (Ic), where X¹ and X² = Cl and X³=F, was prepared according to a process very similar to that shown in Schemes 2 and 3 above with the key difference that 1,3-dichloro-2-fluoro-5-iodobenzene was used as starting material instead of 1-chloro-2-fluoro-5-iodo-3-(trifluoromethyl)benzene. Step 3 of the process is described in detail below.

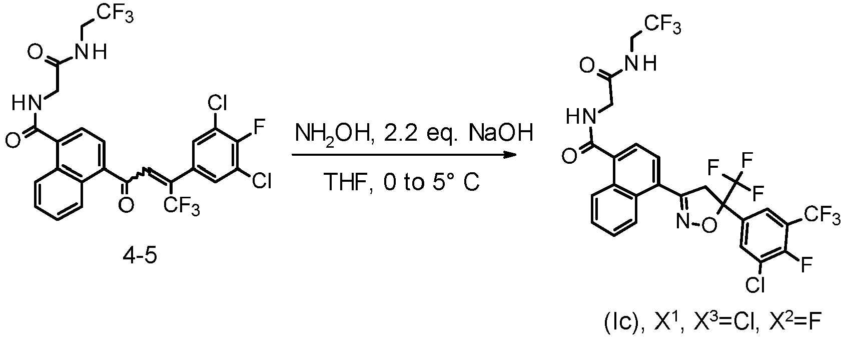

Compound 4-5 (5.0 g, 8.4 mmol, 1.0 eq.) and THF (30 ml, 6 vol.) were charged into a 100 ml 3-neck flask. The solid was dissolved and NaOH (1.5 g, 50%, 18.5 mmol, 2.2 eq.) and hydroxylamine sulfate (0.6 g, 4.62 mmol, 0.55 eq.) were added to the mixture at 0-5° C. The reaction mixture was stirred for 90 minutes and sampled for analysis by HPLC, which indicated that less than 1.0% of the starting material was left. To the resulting mixture was added KH₂PO₄ (1.3 g, 10.9 mmol, 1.3 eq.) and the mixture was warmed to 20-25° C. The layers were allowed to separate and the organic layer isolated. The THF was removed by distillation with concomitant addition of acetonitrile (20 ml, 4 vol.) twice and then concentrated to dryness. Acetonitrile (20 ml, 4 vol.) was added to dissolve the residue and the solution was cooled to 25-30° C slowly and stirred at this temperature for 180 min. The solid was filtered and the cake washed with acetonitrile (10 ml, 2 vol.). The solid was dried under vacuum at 30-35° C for 6 hours to obtain the product (2.1 g, 98.9% purity, yield, 41.2%).

### Veterinary Long-acting Injectable Formulation Examples

The following long-acting injectable compositions are prepared by mixing the following ingredients:
Examples denoted by "*" are reference examples that are not within the scope of the claims.
Formulation Example 5*:

| | |
|---|---|
| Compound of formula (Ia) | 30 % (w/v) |
| Ethanol | 9 % (w/v) |
| PEG 400 | QS. |

Formulation Example 6*

| | |
|---|---|
| Compound of formula (Ia) | 15 % (w/w) |
| PEG 400 | 85% (w/w) |

Formulation Example 7*

| | |
|---|---|
| Compound of formula (Ia) | 26% (w/w) |
| PEG 400 | 74% (w/w) |

Formulation Example 8*

| | |
|---|---|
| Compound of formula (Ia) | 26 % (w/w) |
| PEG 400 | 66 % (w/w) |
| Ethanol | 8 % (w/w) |

Formulation Example 9*

| | |
|---|---|
| Compound of formula (Ia) | 26 % (w/w) |
| PEG 400 | 66 % (w/w) |
| Isopropanol | 8 % (w/w) |

Formulation Example 10*

| | |
|---|---|
| Compound of formula (Ia) | 26% (w/w) |
| PEG 400 | 64% (w/w) |
| Capryol 90 | 10% (w/w) |

Formulation Example 11*

| | |
|---|---|
| Compound of formula (Ia) | 26% (w/w) |
| PEG | 66% (w/w) |
| Benzyl alcohol | 8% (w/w) |

Formulation Example 12*

| | |
|---|---|
| Compound of formula (S)-Ia | 13% (w/w) |
| PEG 400 | 79% (w/w) |
| Ethanol | 8 % (w/w) |

Formulation Example 13*:

| | |
|---|---|
| Compound of formula Ic where X¹, X³= Cl, X² is F | 30 % (w/v) |
| Ethanol | 9 % (w/v) |
| PEG 400 | QS. |

Formulation Example 14*:

| | |
|---|---|
| Compound of formula (Ic), where X¹=Cl, X²=F, X³=CF₃ | 30 % (w/v) |
| Ethanol | 9 % (w/v) |
| PEG 400 | QS. |

Formulation Example 15*

| | |
|---|---|
| Compound of formula(*S*)-Ic, where X¹, X³= Cl, X² is F | 13% (w/w) |
| PEG 400 | 79% (w/w) |
| Ethanol | 8 % (w/w). |

Formulation Example 16*

| | |
|---|---|
| Compound of formula(*S*)-Ic, where X¹=Cl, X²=F, X³=CF₃ | 13% (w/w) |
| PEG 400 | 79% (w/w) |
| Ethanol | 8 % (w/w). |

Formulation Example 17*

| | | |
|---|---|---|
| Compound of formula | (*S*)-Ic, where X¹, X³= Cl, X² is H | 13% (w/w) |
| PEG 400 | 79% (w/w) | |
| Ethanol | 8 % (w/w). | |

Formulation Example 18*

| | |
|---|---|
| Compound of formula (*S*)-Ic, where X¹, X², X³= chloro | 13% (w/w) |
| PEG 400 | 79% (w/w) |
| Ethanol | 8 % (w/w). |

Formulation Example 19*

| | |
|---|---|
| Compound of formula (*S*)-Ic, X¹=Cl, X²=F, X³=CF₃ | 10% (w/v) |
| Ethanol | 8 % (w/w) |
| PEG 400 | QS. |

Formulation Example 20*

| | |
|---|---|
| Compound of formula (*S*)-Ic, X¹=Cl, X²=F, X³=CF₃ | 10% (w/v) |
| PEG 400 | QS. |

Formulation Example 21*

| | |
|---|---|
| Compound of formula (*S*)-Ic, X¹=Cl, X²=F, X³=CF₃ | 5% (w/v) |
| PEG 400 | QS. |

Formulation Example 22*

| | |
|---|---|
| Compound of formula (*S*)-Ic, X¹=Cl, X²=F, X³=CF₃ | 10% (w/v) |
| Ethanol | 3 % (w/v) |
| PEG 400 | QS. |

Formulation Example 23*

| | |
|---|---|
| Compound of formula (*S*)-Ic, X¹=Cl, X²=F, X³=CF₃ | 5% (w/v) |
| Ethanol | 8 % (w/v) |
| PEG 400 | QS. |

Formulation Example 24*

| | |
|---|---|
| Compound of formula (*S*)-Ic, X¹=Cl, X²=F, X³=CF₃ | 2.5% (w/v) |
| PEG 400 | QS. |

Formulation Example 25*

| | |
|---|---|
| Compound of formula (*S*)-Ic, X¹=Cl, X²=F, X³=CF₃ | 2.5% (w/v) |
| Ethanol | 8 % (w/v) |
| PEG 400 | QS. |

Formulation Example 26

| | |
|---|---|
| Compound of formula (S)-Ie | 20% (w/v) |
| Medium chain triglycerides (e.g. Miglyol^{®} 812) | QS |
| Sorbitan monooleate | 0.5% (w/v) |
| Benzyl alcohol | 8% (w/v). |

Formulation Example 27

| | |
|---|---|
| Compound of formula (S)-Ie | 10% (w/v) |
| Medium chain triglycerides (e.g. Miglyol^{®} 812) | QS |
| Sorbitan monooleate | 0.5% (w/v) |
| Benzyl alcohol | 8% (w/v). |

Formulation Example 28

| | |
|---|---|
| Compound of formula (S)-Ie | 5% (w/v) |
| Medium chain triglycerides (e.g. Miglyol^{®} 812) | QS |
| Sorbitan monooleate | 0.5% (w/v) |
| Benzyl alcohol | 8% (w/v). |

Formulation Example 29

| | |
|---|---|
| Compound of formula (S)-Ie | 2.5% (w/v) |
| Medium chain triglycerides (e.g. Miglyol^{®} 812) | QS |
| Sorbitan monooleate | 0.5% (w/v) |
| Benzyl alcohol | 8% (w/v). |

Formulation Example 30

| | |
|---|---|
| Compound of formula (S)-Ie | 20% (w/v) |
| PEG 400 | QS |
| Medium chain triglycerides (e.g. Miglyol^{®} 812) | 30% (w/v) |
| Sorbitan monooleate | 0.5% (w/v) |
| Benzyl alcohol | 8% (w/v). |

Formulation Example 31

| | |
|---|---|
| Compound of formula (S)-Ie | 10% (w/v) |
| PEG 400 | QS |
| Medium chain triglycerides (e.g. Miglyol^{®} 812) | 30% (w/v) |
| Sorbitan monooleate | 0.5% (w/v) |
| Benzyl alcohol | 8% (w/v). |

Formulation Example 32

| | |
|---|---|
| Compound of formula (S)-Ie | 5% (w/v) |
| PEG 400 | QS |
| Medium chain triglycerides (e.g. Miglyol^{®} 812) | 30% (w/v) |
| Sorbitan monooleate | 0.5% (w/v) |
| Benzyl alcohol | 8% (w/v). |

Formulation Example 33

| | |
|---|---|
| Compound of formula (S)-Ie | 2.5% (w/v) |
| PEG 400 | QS |
| Medium chain triglycerides (e.g. Miglyol^{®} 812) | 30% (w/v) |
| Sorbitan monooleate | 0.5% (w/v) |
| Benzyl alcohol | 8% (w/v). |

Formulation Example 34

| | |
|---|---|
| Compound of formula (S)-Ie | 20% (w/v) |
| Propylene glycol dicaprylate/dicaprate (e.g. Miglyol^{®} 840) | QS |
| Sorbitan monooleate | 0.5% (w/v) |
| Benzyl alcohol | 8% (w/v). |

Formulation Example 35

| | |
|---|---|
| Compound of formula (S)-Ie | 10% (w/v) |
| Propylene glycol dicaprylate/dicaprate (e.g. Miglyol^{®} 840) | QS |
| Sorbitan monooleate | 0.5% (w/v) |
| Benzyl alcohol | 8% (w/v). |

Formulation Example 36

| | |
|---|---|
| Compound of formula (S)-Ie | 5% (w/v) |
| Propylene glycol dicaprylate/dicaprate (e.g. Miglyol^{®} 840) | QS |
| Sorbitan monooleate | 0.5% (w/v) |
| Benzyl alcohol | 8% (w/v). |

Formulation Example 37

| | |
|---|---|
| Compound of formula (S)-Ie | 2.5% (w/v) |
| Propylene glycol dicaprylate/dicaprate (e.g. Miglyol^{®} 840) | QS |
| Sorbitan monooleate | 0.5% (w/v) |
| Benzyl alcohol | 8% (w/v). |

Formulation Example 38

| | |
|---|---|
| Compound of formula (S)-Ie | 20% (w/v) |
| PEG 400 | QS |
| Propylene glycol dicaprylate/dicaprate (e.g. Miglyol^{®} 840) | 30% (w/v) |
| Sorbitan monooleate | 0.5% (w/v) |
| Benzyl alcohol | 8% (w/v). |

Formulation Example 39

| | |
|---|---|
| Compound of formula (S)-Ie | 10% (w/v) |
| PEG 400 | QS |
| Propylene glycol dicaprylate/dicaprate (e.g. Miglyol^{®} 840) | 30% (w/v) |
| Sorbitan monooleate | 0.5% (w/v) |
| Benzyl alcohol | 8% (w/v). |

Formulation Example 40

| | |
|---|---|
| Compound of formula (S)-Ie | 5% (w/v) |
| PEG 400 | QS |
| Propylene glycol dicaprylate/dicaprate (e.g. Miglyol^{®} 840) | 30% (w/v) |
| Sorbitan monooleate | 0.5% (w/v) |
| Benzyl alcohol | 8% (w/v). |

Formulation Example 41

| | |
|---|---|
| Compound of formula (S)-Ie | 2.5% (w/v) |
| PEG 400 | QS |
| Propylene glycol dicaprylate/dicaprate (e.g. Miglyol^{®} 840) | 30% (w/v) |
| Sorbitan monooleate | 0.5% (w/v) |
| Benzyl alcohol | 8% (w/v). |

Formulation Example 42

| | |
|---|---|
| Compound of formula (S)-Ie | 20% (w/v) |
| PEG 400 | QS |
| Propylene glycol dicaprylate/dicaprate (e.g. Miglyol^{®} 840) | 30% (w/v) |
| Sorbitan monooleate | 0.5% (w/v). |

Formulation Example 43

| | |
|---|---|
| Compound of formula (S)-Ie | 10% (w/v) |
| PEG 400 | QS |
| Propylene glycol dicaprylate/dicaprate (e.g. Miglyol^{®} 840) | 30% (w/v) |
| Sorbitan monooleate 0.5% | (w/v). |

Formulation Example 44

| | |
|---|---|
| Compound of formula (S)-Ie | 5% (w/v) |
| PEG 400 | QS |
| Propylene glycol dicaprylate/dicaprate (e.g. Miglyol^{®} 840) | 30% (w/v) |
| Sorbitan monooleate | 0.5% (w/v). |

Formulation Example 45

| | |
|---|---|
| Compound of formula (S)-Ie | 2.5% (w/v) |
| PEG 400 | QS |
| Propylene glycol dicaprylate/dicaprate (e.g. Miglyol^{®} 840) | 30% (w/v) |
| Sorbitan monooleate | 0.5% (w/v). |

Formulation Example 46

| | |
|---|---|
| Compound of formula (S)-Ie | 20% (w/v) |
| PEG 400 | QS |
| Medium chain triglycerides (e.g. Miglyol^{®} 812) | 30% (w/v) |
| Sorbitan monooleate | 0.5% (w/v). |

Formulation Example 47

| | |
|---|---|
| Compound of formula (S)-Ie | 10% (w/v) |
| PEG 400 | QS |
| Medium chain triglycerides (e.g. Miglyol^{®} 812) | 30% (w/v) |
| Sorbitan monooleate | 0.5% (w/v). |

Formulation Example 48

| | |
|---|---|
| Compound of formula (S)-Ie | 5% (w/v) |
| PEG 400 | QS |
| Medium chain triglycerides (e.g. Miglyol^{®} 812) | 30% (w/v) |
| Sorbitan monooleate | 0.5% (w/v). |

Formulation Example 49

| | |
|---|---|
| Compound of formula (S)-Ie | 2.5% (w/v) |
| PEG 400 | QS |
| Medium chain triglycerides (e.g. Miglyol^{®} 812) | 30% (w/v) |
| Sorbitan monooleate | 0.5% (w/v). |

### Example 50: Viscosity of Long-Acting Formulations

The viscosity of the formulations in Examples 19-25 was measured to determine their suitability for injection. Polyethylene glycol 400 (PEG 400) was used as a reference. The conditions below were used for the measurement:
Instrument: LVDV-E Brookfield viscometer
Spindle: S31
Speed: 60 revolutions per minute (rpm)
Temperature: 25° C
Sample volume: 9.0 mL
Measurement time: 2 to 3 minutes

| **Formulation** | **Measured viscosity in cPs** | **Calculated Total Allowable Error** |
|---|---|---|
| Example 19* | 75.5 | ±5.75 |
| Example 20* | 134.5 | ±6.344 |
| Example 21* | 109.5 | ±6.093 |
| Example 22* | 102.5 | ±6.023 |
| Example 23* | 60.0 | ±5.598 |
| Example 24* | 95.5 | ±5.953 |
| Example 25* | 55.5 | ±5.553 |
| PEG 400 | 93.0 | ±5.929 |

| | | |
|---|---|---|
| *Reference examples not within the scope of the claims. | | |

The viscosity of each of the compositions from Examples 19 to 25 was found to be suitable for administration by injection.

### Efficacy examples:

### Example 51: Efficacy of Injectable Formulation Against Rhipicephalus (Boophilus) microplus ticks.

The efficacy of long-acting injectable compositions of the invention comprising the compounds of formula (Ie) and (S)-Ie, against *Rhipicephalus microplus* ticks on cattle was determined against an untreated control group. The efficacy of compositions comprising the compounds (Ie) and (S)-e were also compared with injectable compositions comprising afoxolaner (formula Ia, 4-[5-[3-chloro-5-(trifluoromethyl)phenyl]-4,5-dihydro-5-(trifluoromethyl)-3-isoxazolyl]-N-[2-oxo-2-[(2,2,2-trifluoroethyl)amino]ethyl]-1-naphthalanecarboxamide), and comparative compounds of formulae (If) and (S)-If shown below. 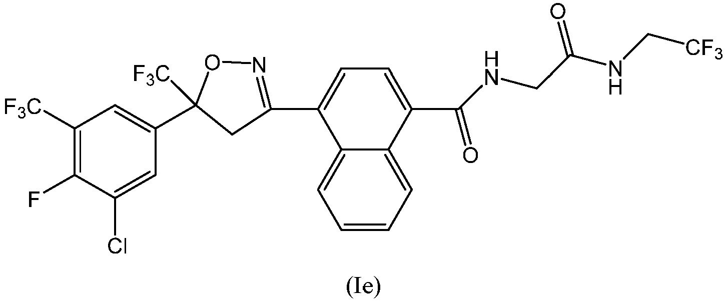 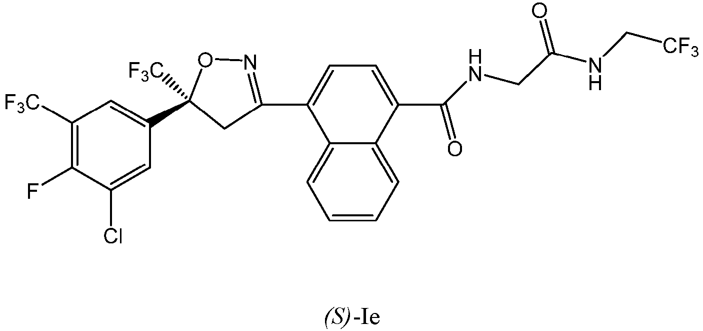 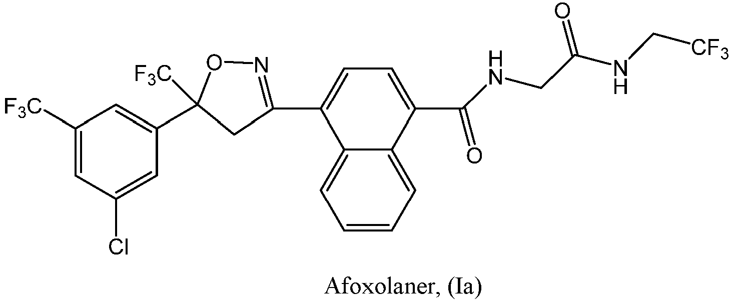 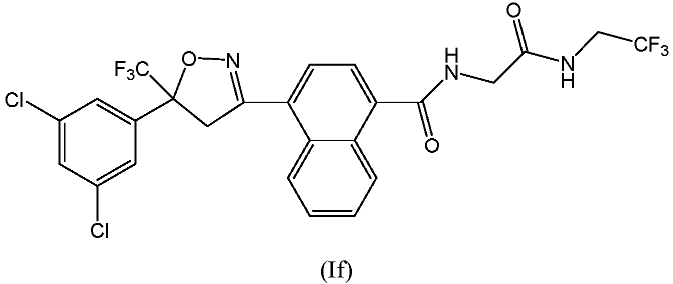 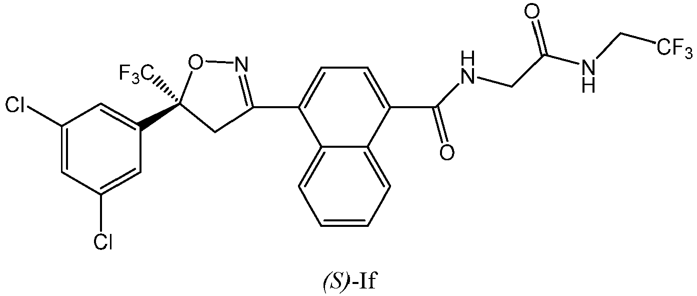 Each compound was formulated in an injectable composition at a concentration of 10% (w/v) in a carrier comprising 8% (w/v) ethanol and Q.S. with polyethylene glycol 400. The racemic compounds afoxolaner and (If) were dosed at 1.0 mg/kg body weight and the *(S)*-enantiomers (*(S)*-Ie and *(S)*-If) were dosed at 0.5 mg/kg body weight. Six healthy head of cattle were used in each study group. Cattle in Group 1 were untreated (control). Cattle in Groups 2, 3, 4, 5 and 6 were treated on Day 0 subcutaneously with injectable compositions comprising the compounds of formula Ie, (S)-If, *(S)*-Ie, afoxolaner (Ia) and (If), respectively. Several weeks before treatment, cattle were infested three times a week with approximately 2500 *Rhipicephalus microplus* larvae to establish ongoing infestations. Each animal was challenged by infestation with approximately 5000 *R. microplus* larvae on Days 7 and 21 and every 14 days thereafter. Ticks dropping from each animal in the previous 24 hours were collected daily and counted from Day 1 until the end of the study. The cattle in study Groups 3, 5 and 6 were not infested further when the efficacy of the treatment dropped significantly.

Tables 1A, 1B and 1C below show the total tick count % efficacy of injectable compositions comprising compounds of the invention ((Ie) and *(S)*-Ie) against *R. microplus* for selected days through Day 110 post treatment compared with the untreated control group and the comparison isoxazoline compounds. As evidenced from the data in Tables 1A, 1B and 1C below, the compositions comprising the compounds (Ie) and *(S)*-Ie of the invention dosed at 1.0 mg/kg and 0.5 mg/kg, respectively, provide surprising and unexpected efficacy against *Rhipicephalus microplus* ticks for an extended period of time compared with compositions comprising afoxolaner, (If) or *(S)*-If. Further, the efficacy of compounds (Ie) and *(S)*-Ie is also faster-acting than compositions comprising the other isoxazoline compounds. The superior efficacy of compounds (Ie) (racemic) and *(S)*-Ie against *R*. *microplus* ticks is unexpected and unpredictable.

**Table 1A: Tick Count Efficacy Against Rhipicephalus microplus**

| **Treatment Group** | **Average % Efficacy (Tick Count)** | | | | | | |
|---|---|---|---|---|---|---|---|
| | **Day 5** | **Day 10** | **Day 20** | **Day 30** | **Day 40** | **Day 49** | **Day 55** |
| **Group 2 (Ie)** | 69 | 100 | 100 | 100 | 100 | 100 | 100 |
| **Group 3 ((*S*)-If)** | 39 | 27 | 33 | 86 | 100 | 36 | 0 |
| **Group 4 ((*S*)-Ie)** | 66 | 93 | 100 | 100 | 100 | 100 | 100 |
| **Group 5 afoxolaner** | 38 | 48 | 77 | 99 | 100 | 94 | 73 |
| **Group 6 (If)** | 70 | 62 | 57 | 84 | 100 | 70 | 14 |

**Table 1B: Tick Count Efficacy Against Rhipicephalus microplus**

| **Treatment Group** | **Average % Efficacy (Tick Count)** | | | | | | |
|---|---|---|---|---|---|---|---|
| | **Day 60** | **Day 65** | **Day 70** | **Day 75** | **Day 80** | **Day 85** | **Day 88** |
| **Group 2 (Ie)** | 100 | 100 | 100 | 100 | 100 | 100 | 100 |
| **Group 3 ((*S*)-If)** | - | - | - | - | - | - | - |
| **Group 4 ((*S*)-Ie)** | 100 | 100 | 100 | 100 | 100 | 90 | 88 |
| **Group 5 afoxolaner** | 71 | 48 | 3 | 0 | - | - | - |
| **Group 6 (If)** | - | - | - | - | - | - | - |

**Table 1C: Tick Count Efficacy against Rhipicephalus microplus**

| **Treatment Group** | **Average % Efficacy (Tick Count)** | | | |
|---|---|---|---|---|
| | **Day 95** | **Day 100** | **Day 105** | **Day 110** |
| **Group 2 (Ie)** | 100 | 100 | 93 | 82 |
| **Group 3 ((*S*)-If)** | - | - | - | - |
| **Group 4 ((*S*)-Ie)** | 92 | 88 | 76 | 65 |
| **Group 5 afoxolaner** | - | - | - | - |
| **Group 6 (If)** | - | - | - | - |

### Example 52: Efficacy of Long-Acting Injectable Compositions Against Haematobia irritans (horn fly) on Cattle

The efficacy of a long-acting injectable composition comprising the compound of formula (*S*)-Ie against horn fly on cattle was evaluated. Two groups of 15 cattle were selected for the study and randomly assigned to one of two groups. The cattle in Group 1 were untreated and the cattle in Group 2 were treated with a long-acting composition of the invention containing 10% (w/v) of the compound of formula (S)-Ie in a carrier comprising 8% (w/v) ethanol in PEG 400 (QS). The composition was administered to the cattle in Group 2 at a dose of 0.25 mL/50 kg body weight. Each animal was naturally infested with horn flies, and horn fly counts were performed on Days -2, 3, 7, 10, 13, 16, 20, 23, 27, 30, 34, 37, 43, 45 and 48. Tables 2A and 2B below provide the horn fly counts and the % reduction of the treated group relative to the control.

**Table 2A**

| **Treatment Group** | **Arithmetic Mean of Horn Fly Counts & % Reduction** | | | | | | | |
|---|---|---|---|---|---|---|---|---|
| | **Day 3** | **Day 7** | **Day 10** | **Day 13** | **Day 16** | **Day 20** | **Day 23** | **Day 27** |
| **Group 1 (untreated)** | 254.3 | 136.0 | 78.3 | 60.0 | 104.7 | 83.0 | 53.3 | 59.3 |
| **Group 2 ((*S*)-Ie)** | 26.7 | 35.0 | 16.0 | 22.0 | 44.7 | 36.0 | 30.7 | 22.7 |
| **% Reduction** | 89.5 | 74.3 | 79.6 | 63.3 | 57.3 | 56.6 | 42.5 | 61.8 |

**Table 2B**

| **Treatment Group** | **Arithmetic Mean of Horn Fly Counts & % Reduction** | | | | | |
|---|---|---|---|---|---|---|
| | **Day 30** | **Day 34** | **Day 37** | **Day 43** | **Day 45** | **Day 48** |
| **Group 1 (untreated)** | 71.0 | 47.7 | 73.3 | 43.0 | 66.7 | 44.0 |
| **Group 2 ((*S*)-Ie)** | 23.0 | 26.3 | 28.0 | 26.0 | 43.0 | 30.3 |
| **% Reduction** | 67.6 | 44.8 | 61.8 | 39.5 | 35.5 | 31.1 |

## Claims

1. A long-acting injectable composition for the treatment or prevention of a parasitic infection or infestation in an animal comprising:
a) an effective amount of at least one parasiticidal isoxazoline active agent, which is a compound of formula (Ic): 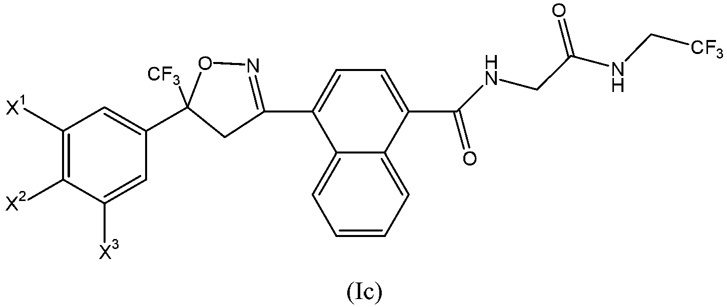
or a pharmaceutically acceptable salt thereof;
wherein
X¹, X² and X³ are each independently H, halogen, C₁-C₃alkyl or C₁-C₃haloalkyl;
b) a neutral oil that is selected from triglycerides of fractionated plant fatty acids with chain lengths of C₈ to C₁₀;
c) optionally, at least one co-solvent;
d) optionally, an antioxidant; and
e) optionally at least one pharmaceutically acceptable additive, excipient or mixtures thereof wherein no other pharmaceutically acceptable polymers are present.

2. The long-acting injectable composition according to claim 1, wherein the isoxazoline active agent is enriched in an enantiomer.

3. The long-acting injectable composition according to claim 2, wherein the isoxazoline active agent is a compound of formula (S)-Ic: 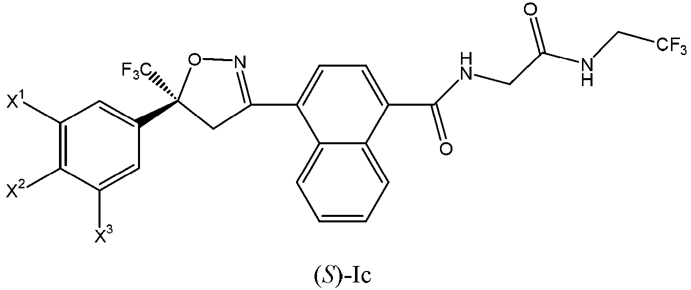
wherein X¹, X² and X³ are independently chloro, fluoro or CF₃;
or a pharmaceutically acceptable salt thereof.

4. The long-acting injectable composition according to any one of claims 1 to 3, wherein the composition comprises a co-solvent, and wherein said co-solvent is a C₁-C₆alcohol, a glycol ether, glycerol, propylene glycol, a cyclic carbonate, 2-pyrrolidone, N-methylpyrrolidone, dimethyl isosorbide, dimethylacetamide, dimethylsulfoxide or glycerol formal, or a combination thereof.

5. The long-acting injectable composition according to any one of claims 1 to 3, wherein the composition comprises a co-solvent, and wherein said co-solvent is benzyl alcohol, benzyl benzoate, ethyl acetate, triacetin, a lipid, a C₈-C₁₀ triglyceride, a C₈-C₁₀ triglyceride combined with linoleic acid, a C₈-C₁₀ triglyceride combined with succinic acid, a propylene glycol diester of C₈-C₁₀ fatty acids, castor oil, cottonseed oil, sesame oil, soybean oil or safflower oil, or a combination thereof.

6. The long-acting injectable composition according to any one of claims 1 to 3, wherein the composition comprises a co-solvent, and wherein the co-solvent is ethanol, isopropanol or benzyl alcohol, or a mixture thereof.

7. The long-acting injectable composition according to any one of claims 1 to 6, wherein the composition comprises a pharmaceutically acceptable excipient, and wherein the excipient is a surfactant.

8. The long-acting injectable composition according to claim 7, wherein the surfactant is selected from the group consisting of glyceryl monooleate, polyoxyethylene sorbitan fatty acid esters, sorbitan monooleate, polyvinyl alcohol, polysorbate 20, polysorbate 80, d*-*α-tocopherol polyethylene glycol 1000 succinate, sodium lauryl sulfate, co-polymers of ethylene oxide and propylene oxide, polyethylene glycol castor oil derivatives, propylene glycol monolaurate, glycerol caprylate/caprate, polyglycolized glycerides, PEG 300 caprylic/capric glycerides, PEG 400 caprylic/capric glycerides, PEG 300 oleic glycerides, PEG 300 linoleic glycerides, polyethylene glycol stearates and polyethylene glycol hydroxy stearates, or a combination thereof.

9. The long-acting injectable composition according to claim 1 comprising:
a) about 0.5 to about 30% (w/v) of an isoxazoline compound of formula (Ic): 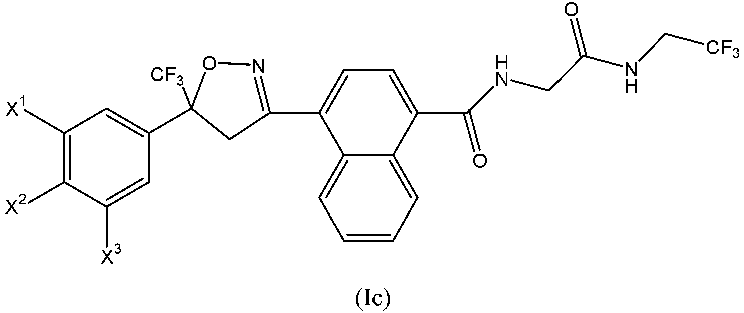 or a pharmaceutically acceptable salt thereof;
wherein
X¹, X² and X³ are each independently H, halogen, C₁-C₃alkyl or C₁-C₃haloalkyl;
b) a neutral oil that is selected from triglycerides of fractionated plant fatty acids with chain lengths of C₈ to C₁₀;
c) optionally, about 5% to 40% (w/v) of co-solvent, wherein said co-solvent is selected from the group consisting of ethanol and isopropanol;
d) optionally, about 0.01% to about 2% (w/v) of an antioxidant; and
e) optionally about 0.01% to about 5% (w/v) of a pharmaceutically acceptable additive, excipient or mixtures thereof
wherein the neutral oil is present in the overall composition in a proportion representing the complement to 100% of the composition.

10. The long-acting injectable composition according to claim 9, wherein;
X¹ is Cl; X² is F; and X³ is -CF₃.

11. The long-acting composition according to any one of claims 1 to 10, which further comprises an effective amount of at least one additional active agent.

12. The long-acting composition according to claim 11, wherein the additional pharmaceutically active agent is a macrocyclic lactone, a neonicotinoid active agent, a 1-N-arylpyrazole active agent, a cyclic depsipeptide, a benzimidazole, an imidazothiazole, a tetrahydropyrimidine active agent, an organophosphate active agent, levamisole, a paraherquamide active agent, a marcfortine active agent, praziquantel, closantel, pyrantel, morantel, clorsulon, a spinosyn active agent, a spinosoid active agent, an amino acetonitrile active agent, an aryloazol-2-yl cyanoethyl active agent or an insect growth regulator.

13. The long-acting composition according to claim 12, wherein the macrocyclic lactone is abamectin, dimadectin, doramectin, emamectin, eprinomectin, ivermectin, latidectin, lepimectin, selamectin, ML-1,694,554, milbemectin, milbemycin D, moxidectin or nemadectin.

14. A long-acting injectable composition according to claim 1 for use in treating or preventing parasites in an animal in need thereof, wherein the parasites are treated or prevented for about 3 to 6 months.

15. A long-acting injectable composition for use according to claim 14 wherein the animal is a dog, a cat, an ovine or a bovine.

16. A long-acting injectable composition for use according to claim 14, wherein the parasites are treated or prevented for about 5 to 6 months.

17. A long-acting injectable composition for use according to claim 14 wherein the parasites are fleas and/or ticks.

## Patentansprüche

1. Lang wirkende injizierbare Zusammensetzung zur Behandlung oder Vorbeugung einer parasitären Infektion bzw. eines parasitären Befalls bei einem Tier, umfassend:
a) eine wirksame Menge mindestens eines Isoxazolin-Parasitizidwirkstoffs, bei dem es sich um eine Verbindung der Formel (Ic): 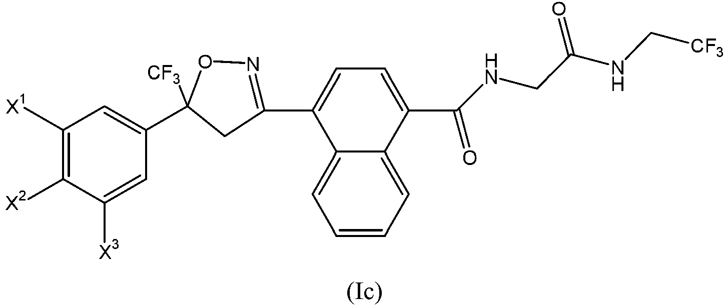
oder ein pharmazeutisch unbedenkliches Salz davon handelt;
wobei
X¹, X² und X³ jeweils unabhängig für H, Halogen, C₁-C₃Alkyl oder C₁-C₃Halogenalkyl stehen;
b) ein Neutralöl, das aus Triglyceriden fraktionierter Pflanzenfettsäuren mit Kettenlängen von C₈ bis C₁₀ ausgewählt ist;
c) gegebenenfalls mindestens ein Cosolvens;
d) gegebenenfalls ein Antioxidans; und
e) gegebenenfalls mindestens einen pharmazeutisch unbedenklichen Zusatzstoff, Hilfsstoff oder Gemische davon,
wobei keine anderen pharmazeutisch unbedenklichen Polymere vorhanden sind.

2. Lang wirkende injizierbare Zusammensetzung nach Anspruch 1, wobei der Isoxazolin-Wirkstoff in einem Enantiomer angereichert ist.

3. Lang wirkende injizierbare Zusammensetzung nach Anspruch 2, wobei es sich bei dem Isoxazolin-Wirkstoff um eine Verbindung der Formel (S)-Ic: 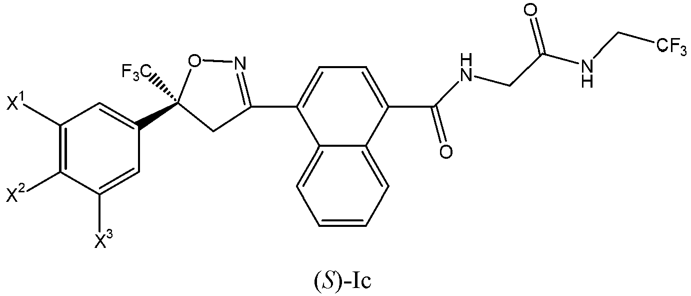
wobei X¹, X² und X³ unabhängig für Chlor, Fluor oder CF₃ stehen,
oder ein pharmazeutisch unbedenkliches Salz davon handelt.

4. Lang wirkende injizierbare Zusammensetzung nach einem der Ansprüche 1 bis 3, wobei die Zusammensetzung ein Cosolvens umfasst und wobei es sich bei dem Cosolvens um einen C₁-C₆Alkohol, einen Glykolether, Glycerin, Propylenglykol, ein cyclisches Carbonat, 2-Pyrrolidon, N-Methylpyrrolidon, Dimethylisosorbid, Dimethylacetamid, Dimethylsulfoxid oder Glycerinformal oder eine Kombination davon handelt.

5. Lang wirkende injizierbare Zusammensetzung nach einem der Ansprüche 1 bis 3, wobei die Zusammensetzung ein Cosolvens umfasst und wobei es sich bei dem Cosolvens um Benzylalkohol, Benzoesäurebenzylester, Essigsäureethylester, Triacetin, ein Lipid, ein C₈-C₁₀-Triglycerid, ein C₈-C₁₀-Triglycerid in Kombination mit Linolsäure, ein C₈-C₁₀-Triglycerid in Kombination mit Bernsteinsäure, einen Propylenglykoldiester von C₈-C₁₀-Fettsäuren, Ricinusöl, Baumwollsamenöl, Sesamöl, Sojaöl oder Safloröl oder eine Kombination davon handelt.

6. Lang wirkende injizierbare Zusammensetzung nach einem der Ansprüche 1 bis 3, wobei die Zusammensetzung ein Cosolvens umfasst und wobei es sich bei dem Cosolvens um Ethanol, Isopropanol oder Benzylalkohol oder ein Gemisch davon handelt.

7. Lang wirkende injizierbare Zusammensetzung nach einem der Ansprüche 1 bis 6, wobei die Zusammensetzung einen pharmazeutisch unbedenklichen Hilfsstoff umfasst und wobei es sich bei dem Hilfsstoff um ein Tensid handelt.

8. Lang wirkende injizierbare Zusammensetzung nach Anspruch 7, wobei das Tensid aus der Gruppe bestehend aus Glycerylmonooleat, Polyoxyethylensorbitanfettsäureestern, Sorbitanmonooleat, Polyvinylalkohol, Polysorbat 20, Polysorbat 80, Polyethylenglykol 1000-Bernsteinsäure-d-α-tocopherylester, Natriumlaurylsulfat, Copolymeren von Ethylenoxid und Propylenoxid, Polyethylenglykol-Ricinusöl-Derivaten, Propylenglykolmonolaurat, Glycerincaprylat/-caprat, polyglykolisierten Glyceriden, PEG 300-Capryl-/Caprinsäureglyceriden, PEG 400-Capryl-/Caprinsäureglyceriden, PEG 300-Ölsäureglyceriden, PEG 300-Linolsäureglyceriden, Polyethylenglykolstearaten und Polyethylenglykolhydroxystearaten oder einer Kombination davon ausgewählt ist.

9. Lang wirkende injizierbare Zusammensetzung nach Anspruch 1, umfassend:
a) etwa 0,5 bis etwa 30 % (Gew./Vol.) einer Isoxazolinverbindung der Formel (Ic): 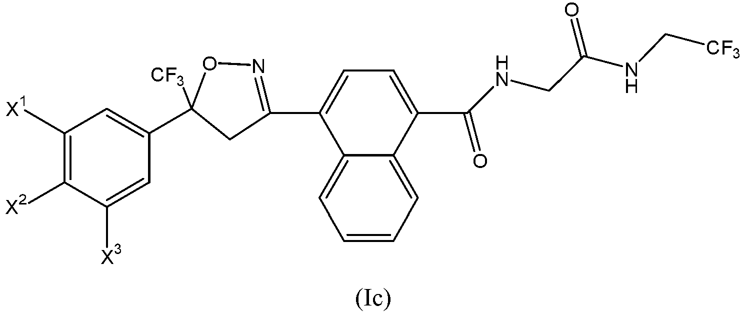
oder ein pharmazeutisch unbedenkliches Salz davon; wobei
X¹, X² und X³ jeweils unabhängig für H, Halogen, C₁-C₃Alkyl oder C₁-C₃Halogenalkyl stehen;
b) ein Neutralöl, das aus Triglyceriden fraktionierter Pflanzenfettsäuren mit Kettenlängen von C₈ bis C₁₀ ausgewählt ist;
c) gegebenenfalls etwa 5 % bis 40 % (Gew./Vol.) Cosolvens, wobei das Cosolvens aus der Gruppe bestehend aus Ethanol und Isopropanol ausgewählt ist;
d) gegebenenfalls etwa 0,01 % bis etwa 2 % (Gew./Vol.) eines Antioxidans; und
e) gegebenenfalls etwa 0,01 % bis etwa 5 % (Gew./Vol.) eines pharmazeutisch unbedenklichen Zusatzstoffs, Hilfsstoffs oder Gemischen davon,
wobei das Neutralöl in der Gesamtzusammensetzung in einem Anteil vorliegt, der die Zusammensetzung auf 100 % komplementiert.

10. Lang wirkende injizierbare Zusammensetzung nach Anspruch 9, wobei;
X¹ für Cl, X² für F und X³ für -CF₃ steht.

11. Lang wirkende Zusammensetzung nach einem der Ansprüche 1 bis 10, die ferner eine wirksame Menge mindestens eines zusätzlichen Wirkstoffs umfasst.

12. Lang wirkende Zusammensetzung nach Anspruch 11, wobei es sich bei dem zusätzlichen pharmazeutischen Wirkstoff um ein makrocyclisches Lacton, einen Neonicotinoid-Wirkstoff, einen 1-N-Arylpyrazol-Wirkstoff, ein cyclisches Depsipeptid, ein Benzimidazol, ein Imidazothiazol, einen Tetrahydropyrimidin-Wirkstoff, einen Organophosphat-Wirkstoff, Levamisol, einen Paraherquamid-Wirkstoff, einen Marcfortin-Wirkstoff, Praziquantel, Closantel, Pyrantel, Morantel, Clorsulon, einen Spinosyn-Wirkstoff, einen Spinosoid-Wirkstoff, einen Aminoacetonitril-Wirkstoff, einen Aryloazol-2-ylcyanoethyl-Wirkstoff oder einen Insektenwachstumsregulator handelt.

13. Lang wirkende Zusammensetzung nach Anspruch 12, wobei es sich bei dem makrocyclischen Lacton um Abamectin, Dimadectin, Doramectin, Emamectin, Eprinomectin, Ivermectin, Latidectin, Lepimectin, Selamectin, ML-1.694.554, Milbemectin, Milbemycin D, Moxidectin oder Nemadectin handelt.

14. Lang wirkende injizierbare Zusammensetzung nach Anspruch 1 zur Verwendung bei der Behandlung oder Vorbeugung gegen Parasiten bei einem behandlungsbedürftigen Tier, wobei etwa 3 bis 6 Monate gegen die Parasiten behandelt oder vorgebeugt wird.

15. Lang wirkende injizierbare Zusammensetzung zur Verwendung nach Anspruch 14, wobei es sich bei dem Tier um einen Hund, eine Katze, ein Schaf oder ein Rind handelt.

16. Lang wirkende injizierbare Zusammensetzung zur Verwendung nach Anspruch 14, wobei etwa 5 bis 6 Monate gegen die Parasiten behandelt oder vorgebeugt wird.

17. Lang wirkende injizierbare Zusammensetzung zur Verwendung nach Anspruch 14, wobei es sich bei den Parasiten um Flöhe und/oder Zecken handelt.

## Revendications

1. Composition injectable à action prolongée pour le traitement ou la prévention d'une infection ou infestation parasitaire chez un animal, comprenant :
a) une quantité efficace d'au moins un agent actif de type isoxazoline parasiticide qui est un composé de formule (Ic) : 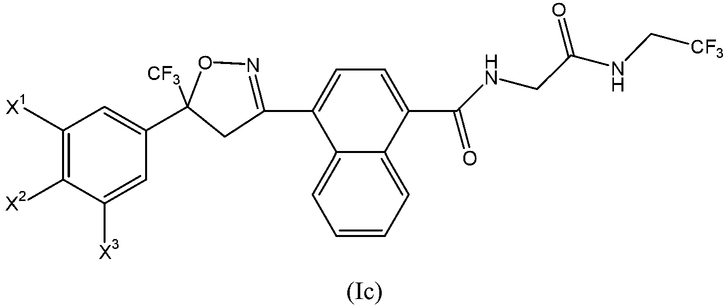
ou un sel pharmaceutiquement acceptable de celui-ci ; dans laquelle
X¹, X² et X³ sont chacun indépendamment H, halogène, C₁-C₃alkyle ou C₁-C₃halogénoalkyle ;
b) une huile neutre sélectionnée parmi les triglycérides d'acides gras végétaux fractionnés ayant des longueurs de chaînes de C₈ à C₁₀ ;
c) éventuellement, au moins un co-solvant ;
d) éventuellement, un antioxydant ; et
e) éventuellement au moins un additif, excipient pharmaceutiquement acceptable ou des mélanges correspondants,
dans laquelle aucun autre polymère pharmaceutiquement acceptable n'est présent.

2. Composition injectable à action prolongée selon la revendication 1, dans laquelle l'agent actif de type isoxazoline est enrichi en un énantiomère.

3. Composition injectable à action prolongée selon la revendication 2, dans laquelle l'agent actif de type isoxazoline est un composé de formule (S)-Ic : 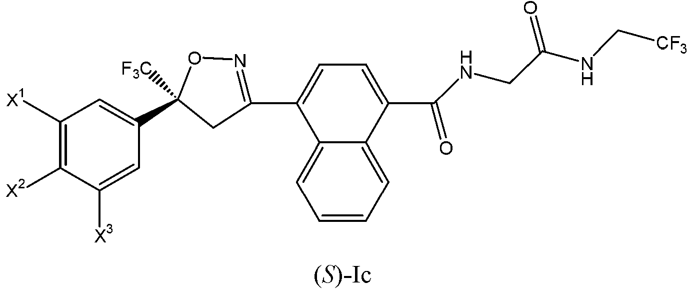
dans laquelle X¹, X² et X³ sont indépendamment chloro, fluoro ou CF₃ ;
ou un sel pharmaceutiquement acceptable de celui-ci.

4. Composition injectable à action prolongée selon l'une quelconque des revendications 1 à 3, dans laquelle la composition comprend un co-solvant, et dans laquelle ledit co-solvant est un alcool en C₁-C₆, un éther de glycol, le glycérol, le propylèneglycol, un carbonate cyclique, la 2-pyrrolidone, la N-méthylpyrrolidone, le diméthylisosorbide, le diméthylacétamide, le diméthylsulfoxyde ou le glycérol formal, ou une combinaison de ceux-ci.

5. Composition injectable à action prolongée selon l'une quelconque des revendications 1 à 3, dans laquelle la composition comprend un co-solvant, et dans laquelle ledit co-solvant est l'alcool benzylique, le benzoate de benzyle, l'acétate d'éthyle, la triacétine, un lipide, un triglycéride en C₈-C₁₀, un triglycéride en C₈-C₁₀ combiné avec de l'acide linoléique, un triglycéride en C₈-C₁₀ combiné avec de l'acide succinique, un diester de propylèneglycol d'acides gras en C₈-C₁₀, l'huile de ricin, l'huile de coton, l'huile de sésame, l'huile de soja ou l'huile de carthame, ou une combinaison de ceux-ci.

6. Composition injectable à action prolongée selon l'une quelconque des revendications 1 à 3, dans laquelle la composition comprend un co-solvant, et dans laquelle le co-solvant est l'éthanol, l'isopropanol ou l'alcool benzylique, ou un mélange de ceux-ci.

7. Composition injectable à action prolongée selon l'une quelconque des revendications 1 à 6, dans laquelle la composition comprend un excipient pharmaceutiquement acceptable, et dans laquelle l'excipient est un tensioactif.

8. Composition injectable à action prolongée selon la revendication 7, dans laquelle le tensioactif est choisi dans le groupe constitué par monooléate de glycéryle, esters d'acides gras de sorbitane polyoxyéthyléné, monooléate de sorbitane, alcool polyvinylique, polysorbate 20, polysorbate 80, succinate de d-α-tocophérol polyéthylène glycol 1000, laurylsulfate de sodium, copolymères d'oxyde d'éthylène et d'oxyde de propylène, dérivés d'huile de ricin de polyéthylène glycol, monolaurate de propylène glycol, caprylate/caprate de glycérol, glycérides polyglycolisés, glycérides capryliques/capriques PEG 300, glycérides capryliques/capriques PEG 400, glycérides oléiques PEG 300, glycérides linoléiques PEG 300, stéarates de polyéthylène glycol et hydroxystéarates de polyéthylène glycol, ou une combinaison de ceux-ci.

9. Composition injectable à action prolongée selon la revendication 1, comprenant :
a) environ 0,5 à environ 30 % (m/v) d'un composé d'isoxazoline de formule (Ic) : 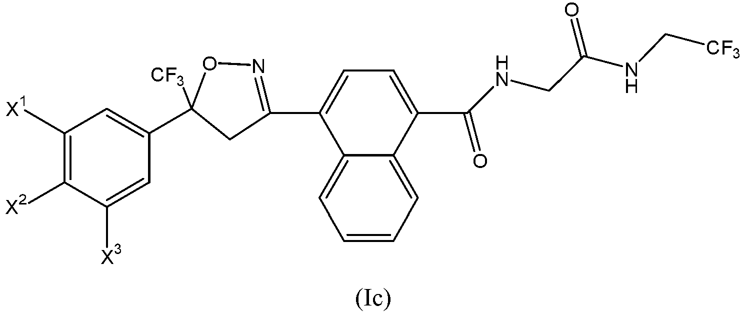
ou un sel pharmaceutiquement acceptable de celui-ci ; dans laquelle
X¹, X² et X³ sont chacun indépendamment H, halogène, C₁-C₃alkyle ou C₁-C₃halogénoalkyle ;
b) une huile neutre sélectionnée parmi les triglycérides d'acides gras végétaux fractionnés ayant des longueurs de chaînes de C₈ à C₁₀ ;
c) éventuellement, environ 5 % à 40 % (p/v) de co-solvant, ledit co-solvant étant choisi dans le groupe constitué de l'éthanol et de l'isopropanol ;
d) éventuellement environ 0,01 % à environ 2 % (p/v) d'un antioxydant ; et
e) éventuellement environ 0,01 % à environ 5 % (p/v) d'un additif ou excipient pharmaceutiquement acceptable ou de mélanges correspondants,
dans laquelle l'huile neutre est présente dans la composition globale en une proportion représentant le complément nécessaire pour atteindre 100 % de la composition.

10. Composition injectable à action prolongée selon la revendication 9, dans laquelle ;
X¹ est Cl ; X² est F ; et X³ est -CF₃.

11. Composition à action prolongée selon l'une quelconque des revendications 1 à 10, qui comprend en outre une quantité efficace d'au moins un agent actif supplémentaire.

12. Composition à action prolongée selon la revendication 11, dans laquelle l'agent pharmaceutiquement actif supplémentaire est une lactone macrocyclique, un agent actif néonicotinoïde, un agent actif 1-N-arylpyrazole, un depsipeptide cyclique, un benzimidazole, un imidazothiazole, un agent actif tétrahydropyrimidine, un agent actif organophosphoré, le lévamisole, un agent actif paraherquamide, un agent actif marcfortine, le praziquantel, le closantel, le pyrantel, le morantel, le clorsulon, un agent actif spinosyne, un agent actif spinosoïde, un agent actif aminoacétonitrile, un agent actif aryloazol-2-yl cyanoéthyl ou un régulateur de croissance des insectes.

13. Composition à action prolongée selon la revendication 12, dans laquelle la lactone macrocyclique est abamectine, dimadectine, doramectine, emamectine, eprinomectine, ivermectine, latidectine, lépimectine, sélamectine, ML-1,694,554, milbémectine, milbémycine D, moxidectine ou némadectine.

14. Composition injectable à action prolongée selon la revendication 1, destinée à être utilisée dans le traitement ou la prévention de parasites chez un animal qui en a besoin, dans laquelle les parasites sont traités ou prévenus pendant environ 3 à 6 mois.

15. Composition injectable à action prolongée destinée à être utilisée selon la revendication 14, dans laquelle l'animal est un chien, un chat, un ovin ou un bovin.

16. Composition injectable à action prolongée destinée à être utilisée selon la revendication 14, dans laquelle les parasites sont traités ou prévenus pendant environ 5 à 6 mois.

17. Composition injectable à action prolongée destinée à être utilisée selon la revendication 14, dans laquelle les parasites sont des puces et/ou des tiques.
